# EUROPÄISCHE PATENTSCHRIFT

(11) **EP 2 935 514 B1**
(45) Veröffentlichungstag und Bekanntmachung des Hinweises auf die Patenterteilung: **01.02.2017**
(21) Anmeldenummer: 13802885.7
(22) Anmeldetag: 09.12.2013
(51) Int. Cl.: C09K 19/34, C09K 19/04, C09K 19/02, C07D 401/04

(54) **FLÜSSIGKRISTALLINES MEDIUM**
LIQUID CRYSTAL MEDIUM
MILIEU CRISTALLIN LIQUIDE

(30) Priorität: 20.12.2012 DE 102012024900
(43) Veröffentlichungstag der Anmeldung: 28.10.2015
(73) Patentinhaber: Merck Patent GmbH, 64293 Darmstadt (DE)
(72) Erfinder: HIRSCHMANN, Harald, 64291 Darmstadt (DE); POHLE, Andreas, 64319 Pfungstadt (DE); CZANTA, Markus, 64287 Darmstadt (DE); SCHOENEFELD, Christian, 64832 Babenhausen (DE); MONTENEGRO, Elvira, 69469 Weinheim (DE); REIFFENRATH, Volker, 64380 Rossdorf (DE)
(86) Internationale Anmeldenummer: PCT/EP2013/003713
(87) Internationale Veröffentlichungsnummer: WO 2014/094999

(56) Entgegenhaltungen:
- DE-A1- 4 030 603
- US-A- 4 668 425
- US-A- 5 919 930
- US-A1- 2010 327 226
- US-A1- 2011 019 119
- US-B2- 6 861 107

## Beschreibung

Die vorliegende Erfindung betrifft ein flüssigkristallines Medium (FK-Medium), dessen Verwendung für elektrooptische Zwecke und dieses Medium enthaltende FK-Anzeigen.

Flüssigkristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nicht-linearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFT's aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei low-volt-Mischungen war es bisher nicht möglich, sehr hohe spezifische Widerstände zu realisieren. Weiterhin ist es wichtig, dass der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, dass auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Neben Flüssigkristallanzeigen, die eine Hintergrundbeleuchtung verwenden, also transmissiv und gegebenenfalls transflektiv betrieben werden, sind besonders auch reflektive Flüssigkristallanzeigen interessant. Diese reflektiven Flüssigkristallanzeigen benutzen das Umgebungslicht zur Informationsdarstellung. Somit verbrauchen sie wesentlich weniger Energie als hintergrundbeleuchtete Flüssigkristallanzeigen mit entsprechender Größe und Auflösung. Da der TN-Effekt durch einen sehr guten Kontrast gekennzeichnet ist, sind derartige reflektive Anzeigen auch bei hellen Umgebungsverhältnissen noch gut abzulesen. Dies ist bereits von einfachen reflektiven TN-Anzeigen, wie sie in z. B. Armbanduhren und Taschenrechnern verwendet werden, bekannt. Jedoch ist das Prinzip auch auf hochwertige, höher auflösende Aktiv-Matrix angesteuerte Anzeigen wie z. B. TFT-Displays anwendbar. Hier ist wie bereits bei den allgemeinen üblichen transmissiven TFT-TN-Anzeigen die Verwendung von Flüssigkristallen mit niedriger Doppelbrechung (Δn) nötig, um eine geringe optische Verzögerung (d · Δn) zu erreichen. Diese geringe optische Verzögerung führt zu einer meist akzeptablen geringen Blickwinkelabhängigkeit des Kontrastes (vgl. DE 30 22 818). Bei reflektiven Anzeigen ist die Verwendung von Flüssigkristallen mit kleiner Doppelbrechung noch wichtiger als bei transmissiven Anzeigen, da bei reflektiven Anzeigen die effektive Schichtdicke, die das Licht durchquert, ungefähr doppelt so groß ist wie bei transmissiven Anzeigen mit derselben Schichtdicke.

Zur Realisierung von 3D-Effekten mittels Shutterbrillen werden insbesondere schnell schaltende Mischungen mit niedrigen Rotationsviskositäten und einer entsprechend hohen optischen Anisotropie (Δn) eingesetzt. Elektrooptische Linsensyteme, mit denen eine 2 dimensionale Darstellung eines Displays in eine 3 dimensionale autostereoskopische Darstellung geschaltet werden kann, können unter Verwendung von Mischungen mit hoher optischer Anisotropie (Δn) realisiert werden.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringem Maße zeigen.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- Schaltbarkeit bei extrem tiefen Temperaturen (out-door-use, Automobil, Avionik)
- erhöhte Beständigkeit gegenüber UV-Strahlung (längere Lebensdauer)
- kleine Schwellenspannung.

Mit den aus dem Stand der Technik zur Verfügung stehenden Medien und Verbindungen, beispielsweise bekannt aus US 2010/327226 A1, US 2011/019119 A1, US 5,919,930 A, DE 40 30 603 A1 und US 4,668,425 A, ist es nicht möglich, diese Vorteile unter gleichzeitigem Erhalt der übrigen Parameter zu realisieren.

Bei höher verdrillten Zellen (STN) sind Medien erwünscht, die eine höhere Multiplexierbarkeit und/oder kleinere Schwellenspannungen und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht.

Insbesondere bei FK-Anzeigen für TV- und Video-Anwendungen (z. B. LCD-TV, Monitore, PDAs, Notebooks, Spielkonsolen) ist eine deutliche Verringerung der Schaltzeiten gewünscht. Dies erfordert FK-Mischungen mit niedrigen Rotationsviskositäten und hohen Werten für die Doppelbrechung Δn.

Der Erfindung liegt die Aufgabe zugrunde, Medien insbesondere für derartige MFK-, FFS-, IPS-, TN-, positive VA- oder STN-Anzeigen bereitzustellen, die die oben angegebenen Nachteile nicht oder nur in geringem Maße zeigen, und vorzugsweise schnelle Schaltzeiten und niedrige Rotationsviskositäten bei gleichzeitig hohem Klärpunkt, sowie eine hohe dielektrische Anisotropie und eine niedrige Schwellenspannung aufweisen.

Es wurde nun gefunden, dass diese Aufgabe gelöst werden kann, wenn man FK-Medien enthaltend eine oder mehrere Verbindungen der Formel I verwendet. Die Verbindungen der Formel I führen zu LC-Mischungen mit den oben angegebenen gewünschten Eigenschaften.

Gegenstand der Erfindung ist ein flüssigkristallines Medium, dadurch gekennzeichnet, dass es eine oder mehrere Verbindungen der Formel I 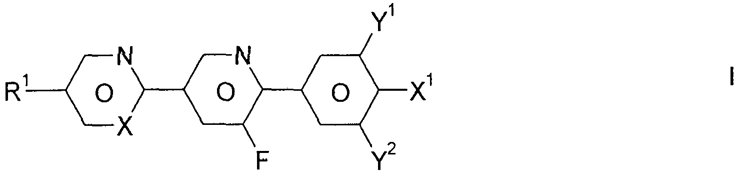 worin
- R¹: einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 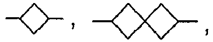 -O-, -CO-O- oder -O-CO- so ersetzt sein können, dass O-Atome nicht direkt miteinander verknüpft sind,
- X¹: F, Cl, CN, SF₅, SCN, NCS, halogenierter Alkylrest, halogenierter Alkenylrest, halogenierter Alkoxyrest oder halogenierter Alkenyloxyrest mit jeweils bis zu 6 C-Atomen,
- X: C-H oder N, und
- Y¹ und Y²: jeweils unabhängig voneinander H oder F
bedeuten,
enthält.

Überraschenderweise wurde gefunden, dass Mischungen enthaltend eine oder mehrere Verbindungen der Formel I eine hohe dielektrische Anisotropie Δε und gleichzeitig ein vorteilhaftes Verhältnis Rotationsviskosität γ₁/Klärpunkt aufweisen. Sie sind daher zur Realisierung von Flüssigkristallmischungen mit niedrigem γ₁ und hohen Δn-Werten besonders geeignet. Darüber hinaus zeigen die Verbindungen der Formel I eine gute Löslichkeit in FK-Medien. Erfindungsgemäße FK-Medien enthaltend eine oder mehrere Verbindungen der Formel I besitzen eine niedrige Rotationsviskosität, schnelle Schaltzeiten, eine sehr hohe positive dielektrische Anisotropie, eine hohe Doppelbrechung und einen breiten nematischen Phasenbereich. Sie sind deshalb besonders gut für Linsen, 2D/3D-Anwendungen, mobile Telefone, TV- und Video-Anwendungen geeignet.

Die Verbindungen der Formel I besitzen einen breiten Anwendungsbereich. In Abhängigkeit von der Auswahl der Substituenten können sie als Basismaterialien dienen, aus denen flüssigkristalline Medien zum überwiegenden Teil zusammengesetzt sind; es können aber auch den Verbindungen der Formel I flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Falls in den oben- und untenstehenden Formeln R¹ einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradedoxy.

Oxaalkyl bedeutet vorzugsweise geradkettiges 2-Oxapropyl (= Methoxymethyl), 2-(= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6-, oder 7-Oxaoctyl, 2-, 3-, 4-, 5-, 6-, 7- oder 8-Oxanonyl, 2-, 3-, 4-, 5-, 6-, 7-, 8- oder 9-Oxadexyl.

Falls R¹ einen Alkylrest bedeutet, in dem eine CH₂-Gruppe durch -CH=CH-ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Vorzugsweise bedeutet R¹ geradkettiges Alkyl mit 1 bis 6 C-Atomen, insbesondere Ethyl, Propyl und Pentyl, ferner Alkenyl mit 2 bis 6 C-Atomen.

In den oben- und untenstehenden Formeln ist X¹ vorzugsweise F, Cl oder ein- oder mehrfach fluorierter Alkyl- oder Alkoxyrest mit 1, 2 oder 3 C-Atomen oder ein- oder mehrfach fluorierter Alkenylrest mit 2 oder 3 C-Atomen bedeutet. X¹ ist besonders bevorzugt F, Cl, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₂CHF₂, OCF₂CF₂CHF₂, OCFHCF₂CF₃, OCFHCF₂CHF₂, OCF₂CF₂CF₃, OCF₂CF₂CClF₂, OCClFCF₂CF₃, OCH=CF₂ oder CH=CF₂, ganz besonders bevorzugt F oder OCF₃, ferner CF₃, OCF=CF₂, OCHF₂ und OCH=CF₂.

Besonders bevorzugt sind Verbindungen der Formeln I, worin X¹ F oder OCF₃, vorzugsweise F, bedeutet. Bevorzugte Verbindungen der Formel I sind solche, worin Y¹ und/oder Y² jeweils H bedeuten.

Ganz besonders bevorzugte Verbindungen der Formel I werden nachfolgend genannt: 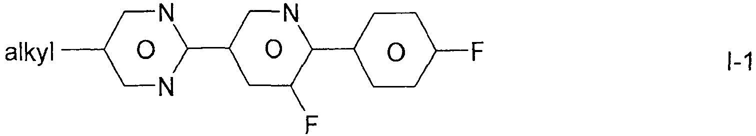 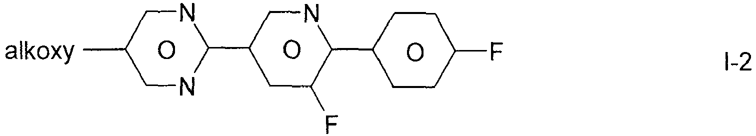 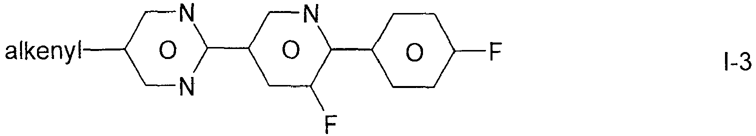 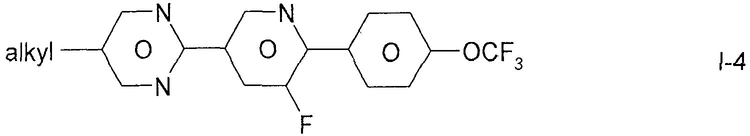 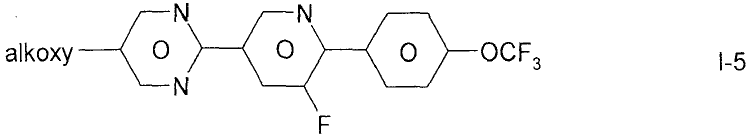 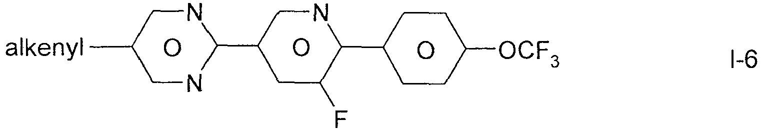 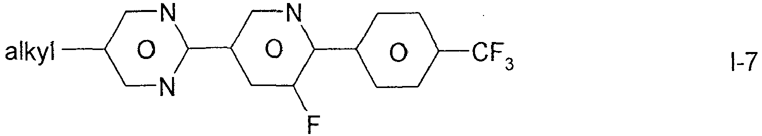 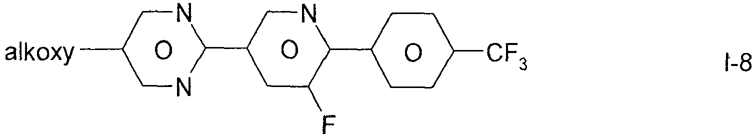 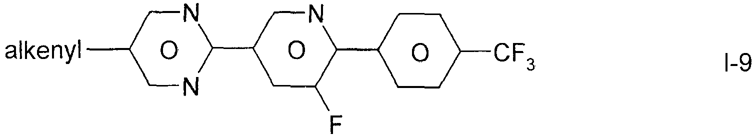 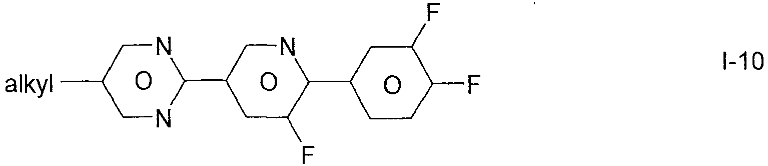 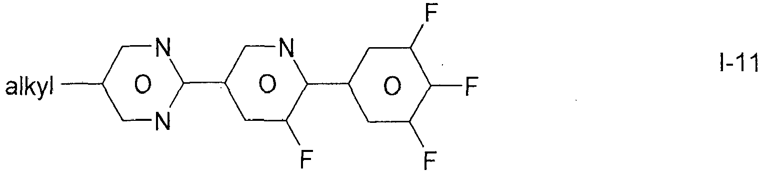 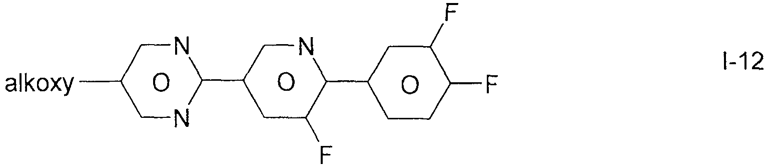 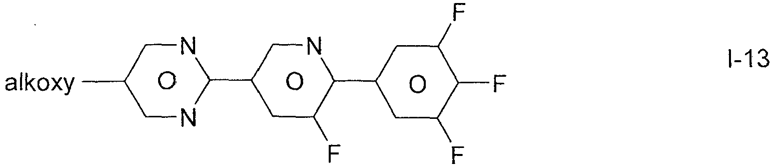 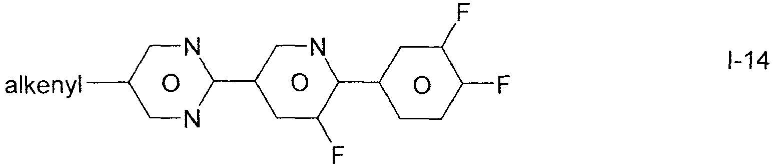 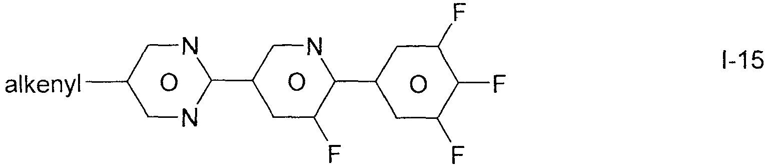 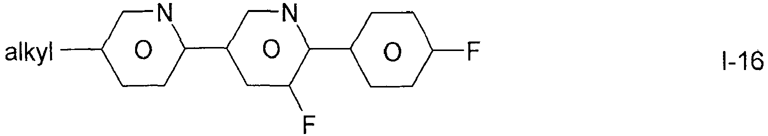 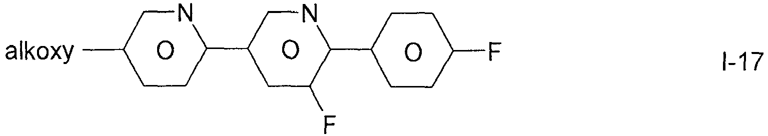 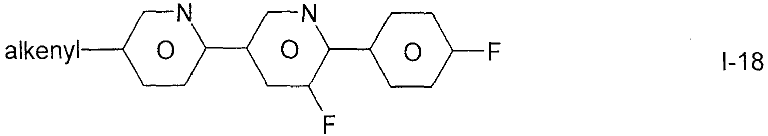 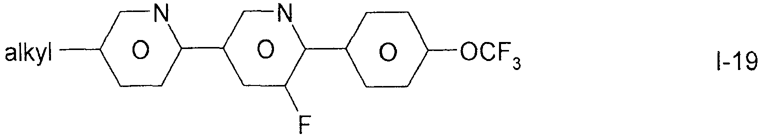 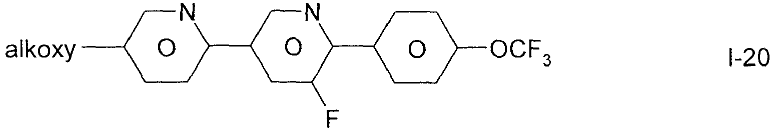 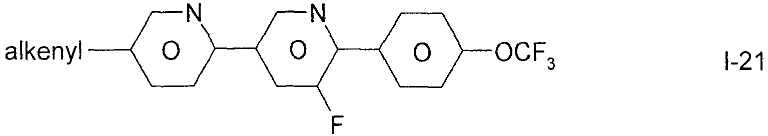 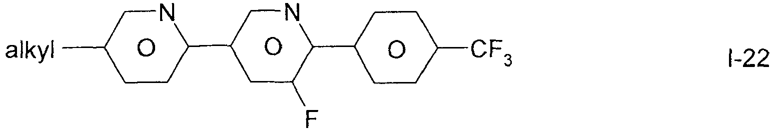 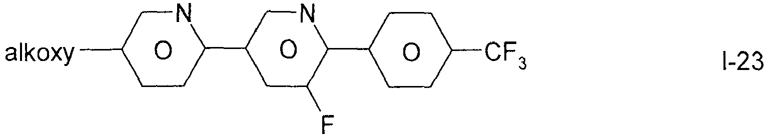 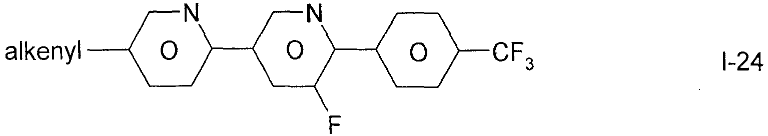 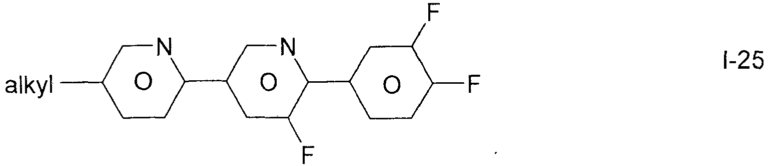 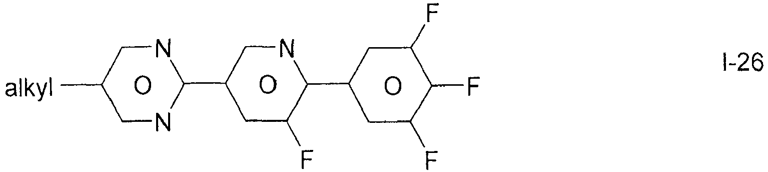 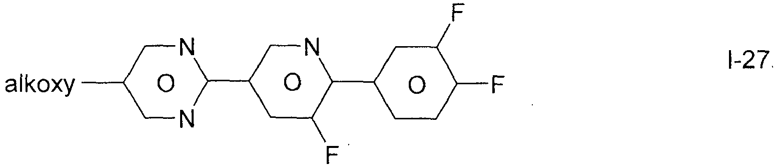 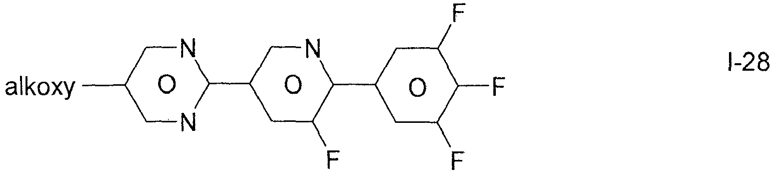 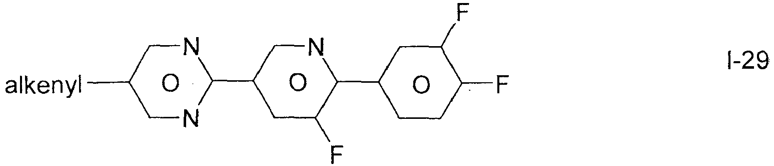 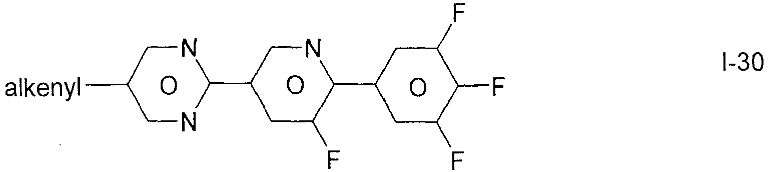 worin
- alkyl: ein geradkettiger Alkylrest mit 1-6 C-Atomen,
- alkenyl: ein geradkettiger Alkenylrest mit 2-6 C-Atomen,
- alkoxy: ein geradkettiger Alkoxylrest mit 1-6 C-Atomen,
bedeuten.

Besonders bevorzugt ist die Verbindung der Formel I-4.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formel I können beispielsweise wie folgt hergestellt werden:

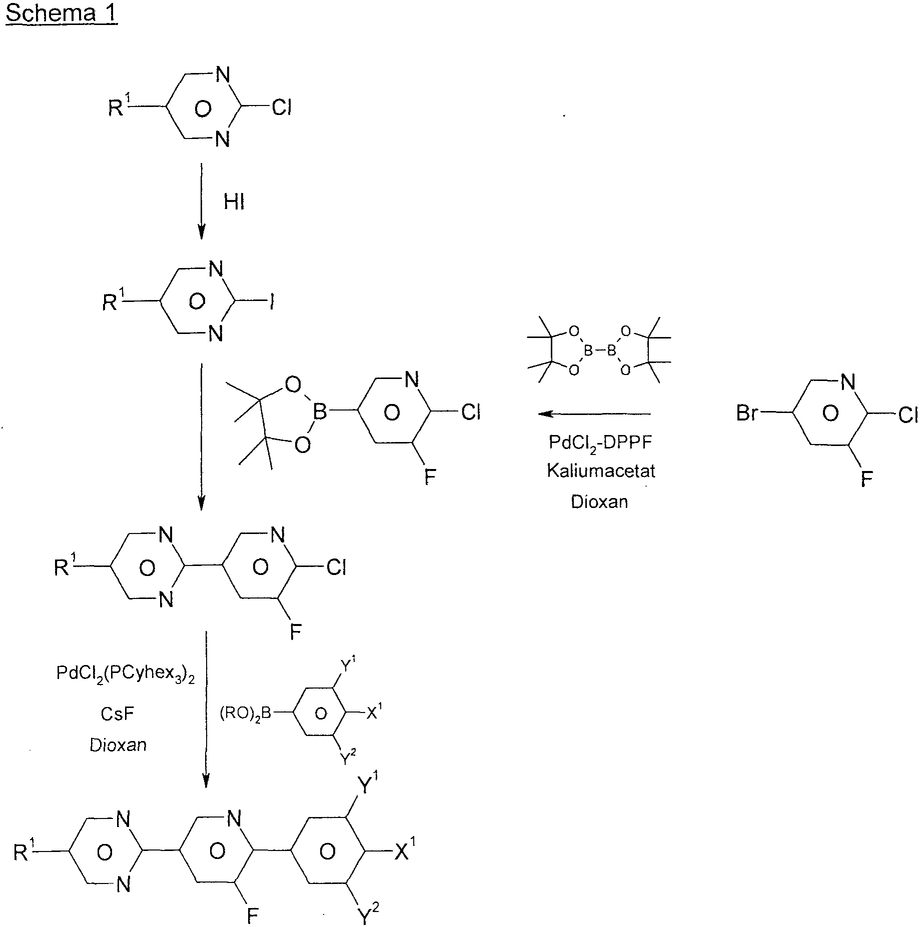

Besonders bevorzugte Verbindungen der Formel I werden wie folgt hergestellt:

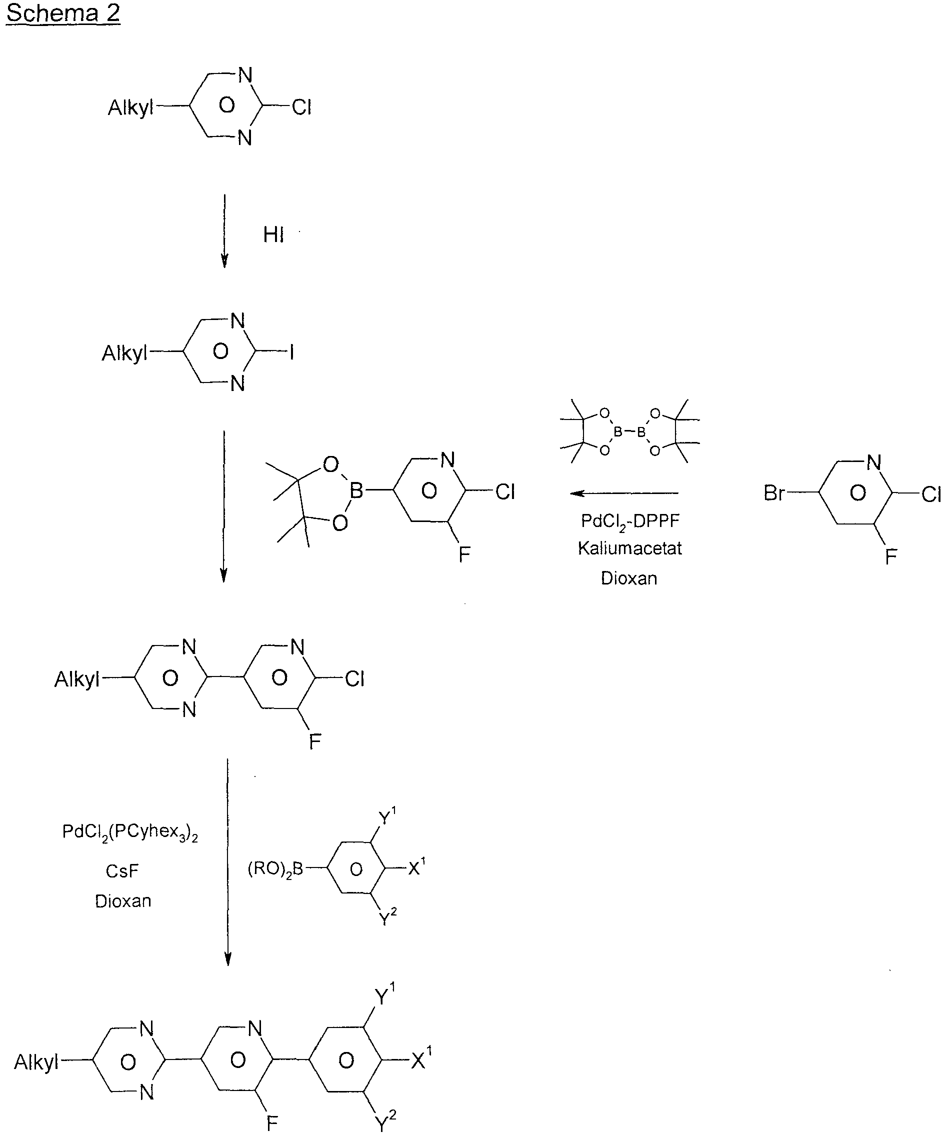

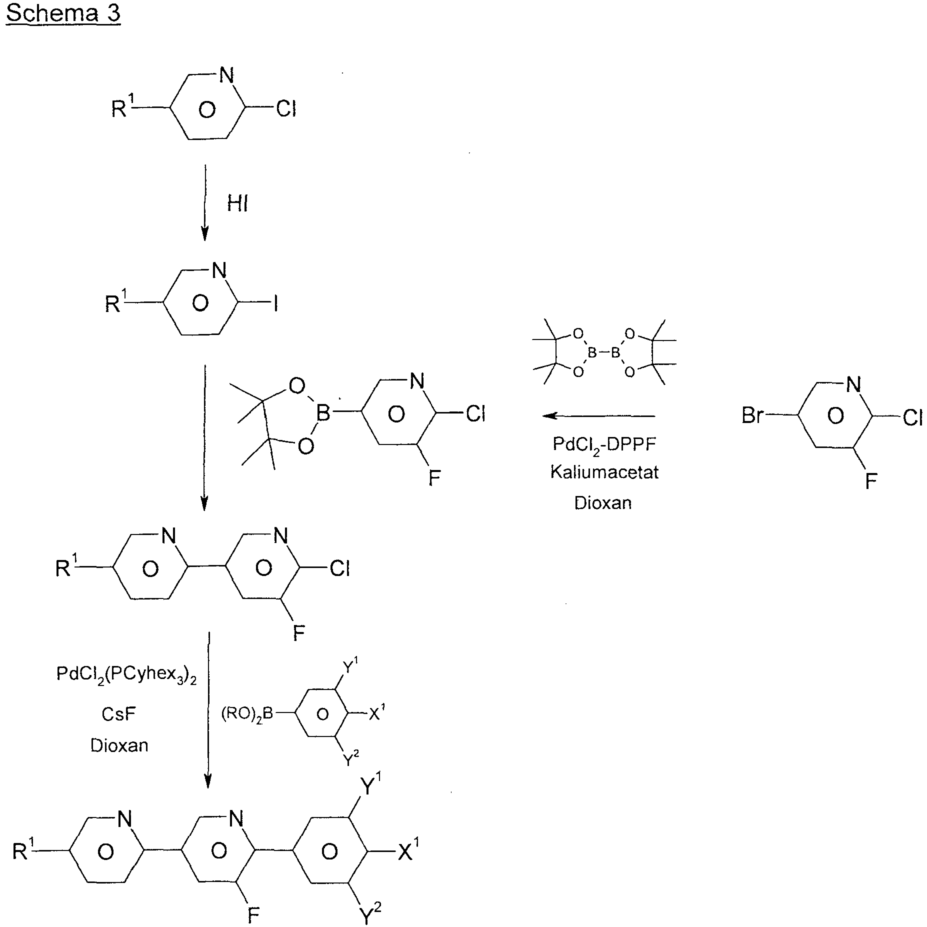

Weitere bevorzugte Ausführungsformen sind im Folgenden angegeben:
- Das Medium enthält zusätzlich eine oder mehrere Verbindungen der Formel II und/oder III 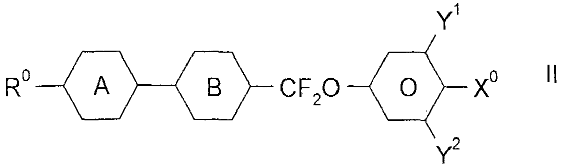 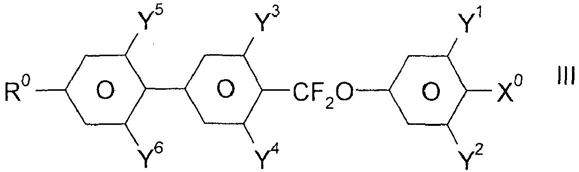 worin
   - R⁰: einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH, 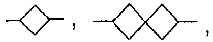 -O-, -CO-O- oder -O-CO- so ersetzt sein können, dass O-Atome nicht direkt miteinander verknüpft sind,
   - X⁰: F, Cl, CN, SF₅, SCN, NCS, halogenierter Alkylrest, halogenierter Alkenylrest, halogenierter Alkoxyrest oder halogenierter Alkenyloxyrest mit bis zu 6 C-Atomen, und
   - Y¹⁻⁶: jeweils unabhängig voneinander H oder F 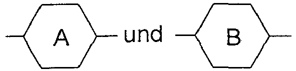 jeweils unabhängig voneinander 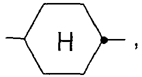 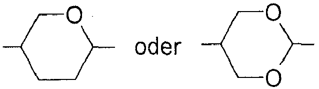
   bedeuten.
- Die Verbindungen der Formel II sind vorzugsweise ausgewählt aus den folgenden Formeln, 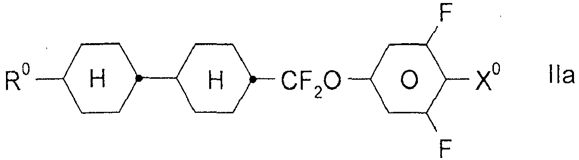 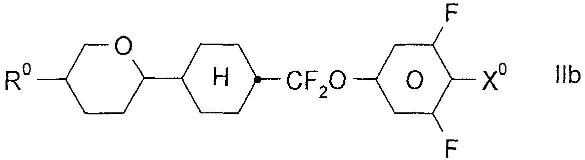 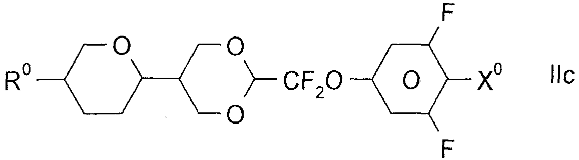 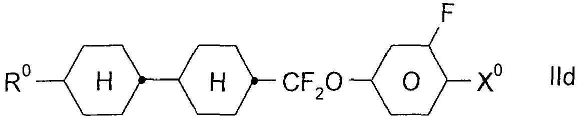 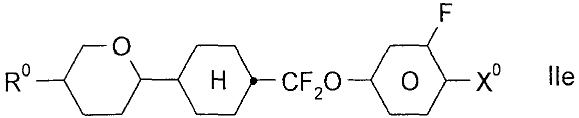 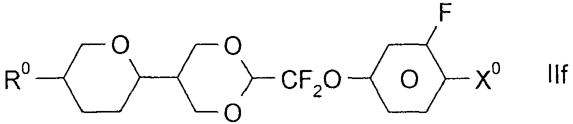
worin R⁰ und X⁰ die oben angegebenen Bedeutungen haben.

Vorzugsweise bedeutet R⁰ Alkyl mit 1 bis 6 C-Atomen. X⁰ bedeutet vorzugsweise F, ferner OCF₃ und CF₃. Besonders bevorzugt sind Verbindungen der Formel IIa und IIb, insbesondere Verbindungen der Formeln IIa und IIb, worin X⁰ F bedeutet.
- Die Verbindungen der Formel III sind vorzugsweise ausgewählt aus den folgenden Formeln, 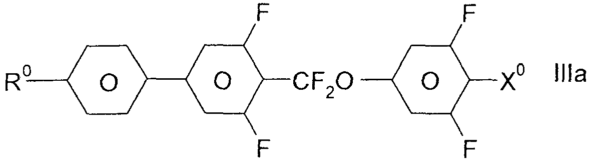 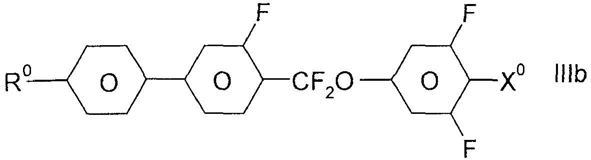 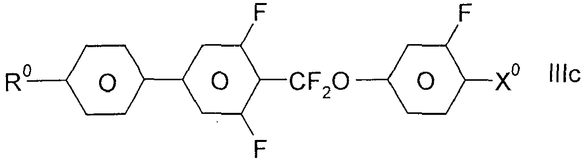 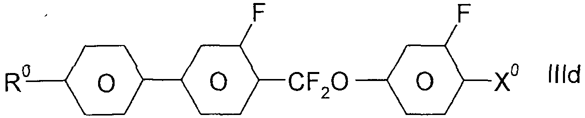 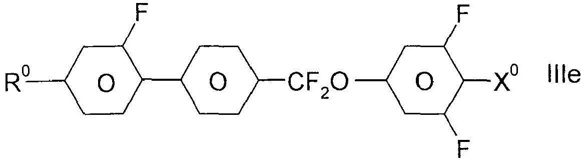 worin R⁰ und X⁰ die oben angegebenen Bedeutungen haben.
   Vorzugsweise bedeutet R⁰ Alkyl mit 1 bis 6 C-Atomen. X⁰ bedeutet vorzugsweise F, ferner OCF₃ und CF₃. Besonders bevorzugt sind Verbindungen der Formeln IIIa und IIIe, insbesondere Verbindungen der Formel IIIa;
- Das Medium enthält zusätzlich eine oder mehrere Verbindungen ausgewählt aus den folgenden Formeln, 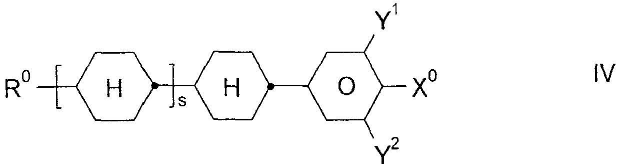 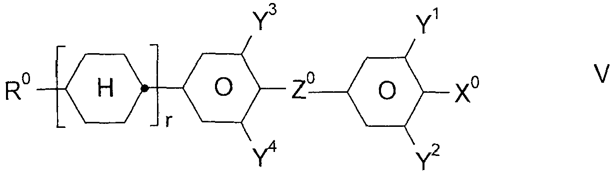 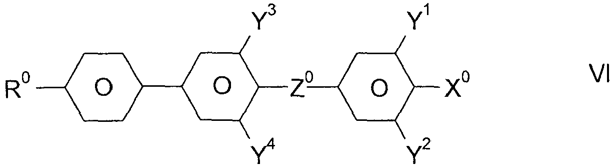 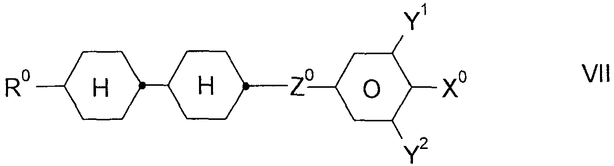 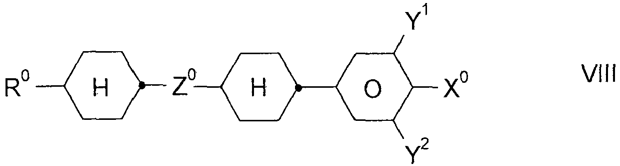 worin
   - R⁰, X⁰ und Y¹⁻⁴: die oben angegebenen Bedeutungen besitzen, und
   - Z⁰: -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- oder -OCF₂-, in Formel V und VI auch eine Einfachbindung,
   - r: 0 oder 1, und
   - s: 0 oder 1
   bedeuten;
- Die Verbindungen der Formel IV sind vorzugsweise ausgewählt aus folgenden Formeln, 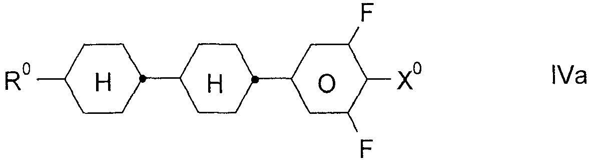 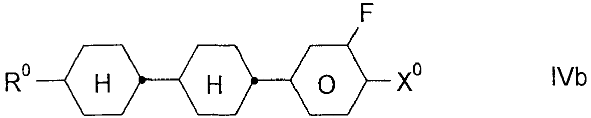 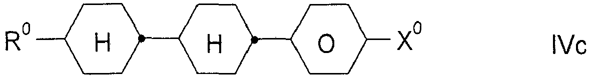 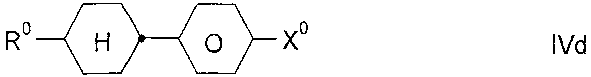 worin R⁰ und X⁰ die oben angegebenen Bedeutungen haben.
   Vorzugsweise bedeutet R⁰ Alkyl mit 1 bis 6 C-Atomen. X⁰ bedeutet vorzugsweise F oder OCF₃, ferner CF₃, OCF=CF₂ und Cl;
- Die Verbindungen der Formel V sind vorzugsweise ausgewählt aus den folgenden Formeln, 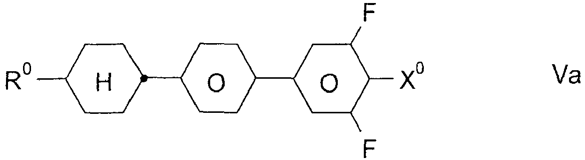 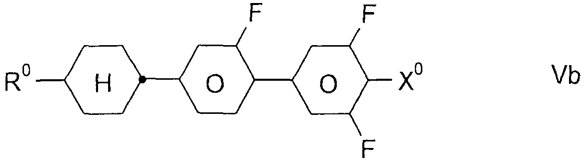 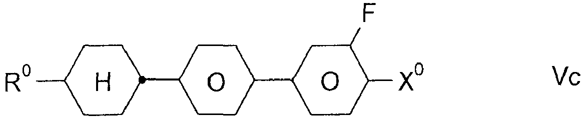 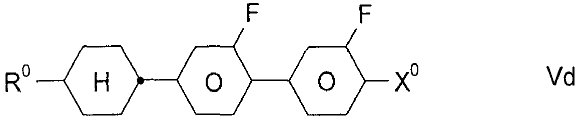 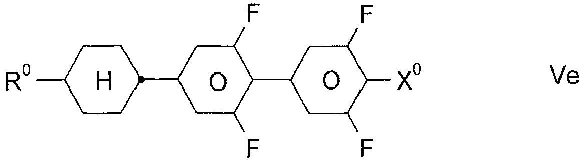 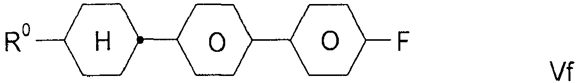 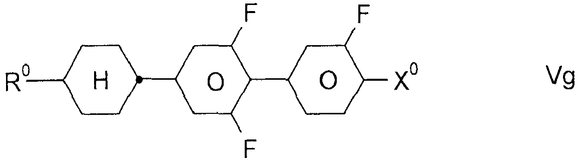 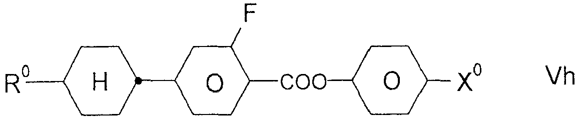 worin R⁰ und X⁰ die oben angegebenen Bedeutungen haben.
   Vorzugsweise bedeutet R⁰ Alkyl mit 1 bis 6 C-Atomen. X⁰ bedeutet vorzugsweise F und OCF₃, ferner OCHF₂, CF₃, OCF=CF₂ und OCH=CF₂;
- Die Verbindungen der Formel VI sind vorzugsweise ausgewählt aus den folgenden Formeln, 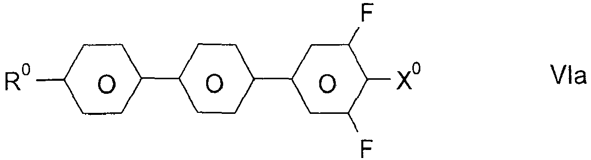 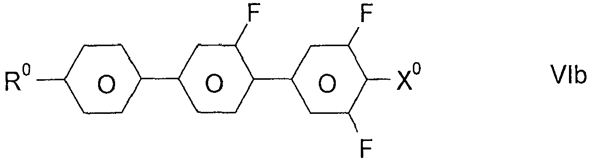 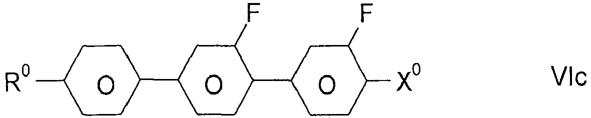 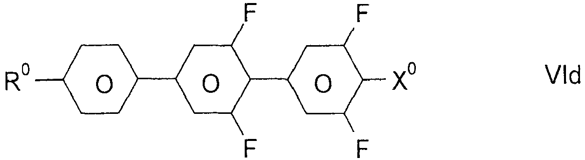 worin R⁰ und X⁰ die oben angegebenen Bedeutungen haben.
   Vorzugsweise bedeutet R⁰ Alkyl mit 1 bis 6 C-Atomen. X⁰ bedeutet vorzugsweise F, ferner OCF₃, CF₃, CF=CF₂, OCHF₂ und OCH=CF₂;
- Die Verbindungen der Formel VII sind vorzugsweise ausgewählt aus den folgenden Formeln, 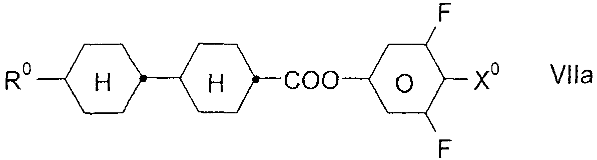 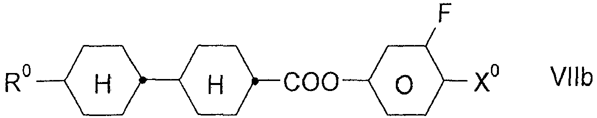 worin R⁰ und X⁰ die oben angegebenen Bedeutungen haben.
   Vorzugsweise bedeutet R⁰ Alkyl mit 1 bis 6 C-Atomen. X⁰ bedeutet vorzugsweise F, ferner OCF₃, OCHF₂ und OCH=CF₂.
- Das Medium enthält zusätzlich eine oder mehrere Verbindungen ausgewählt aus den folgenden Formeln, 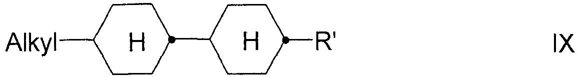 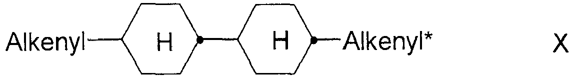 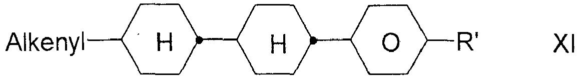 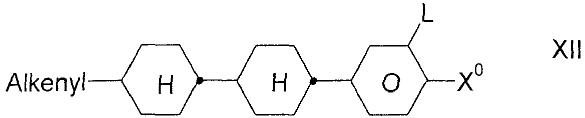 worin X⁰ die oben angegebenen Bedeutungen besitzen, und
   L H oder F,
   "Alkyl" C₁₋₆-Alkyl,
   R' C₁₋₆-Alkyl, C₁₋₆-Alkoxy oder C₂₋₆-Alkenyl, und
   "Alkenyl" und "Alkenyl*" jeweils unabhängig voneinander C₂₋₆-Alkenyl bedeuten.
- Die Verbindungen der Formeln IX-XII sind vorzugsweise ausgewählt aus folgenden Formeln, 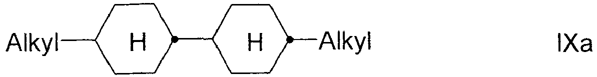 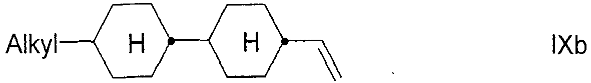 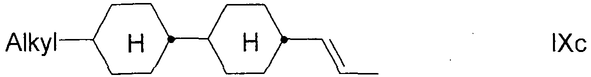 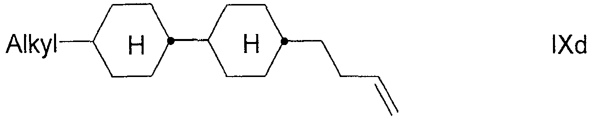 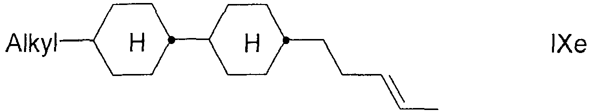 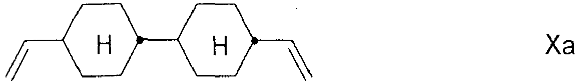 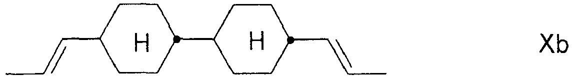 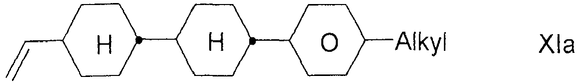 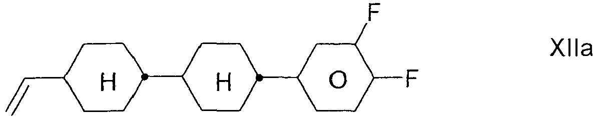 worin "Alkyl" die oben angegebene Bedeutung hat;
   Insbesondere bevorzugt sind die Verbindungen der Formeln XIa, IXb, IXc, Xa, Xb, XIa und Xlla. In den Formeln IXb und IX bedeutet "Alkyl" unabhängig voneinander vorzugsweise n-C₃H₇, n-C₄H₉ oder n-C₅H₁₁, insbesondere n-C₃H₇.
- Das Medium enthält zusätzlich eine oder mehrere Verbindungen ausgewählt aus den folgenden Formeln, 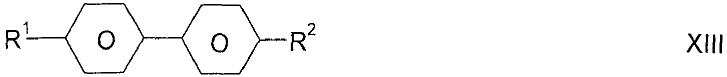 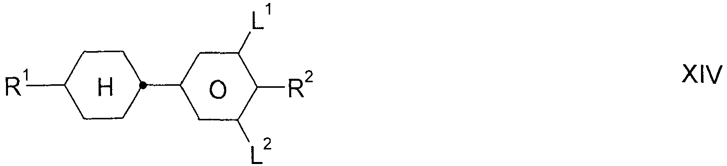 worin L¹ und L² die oben angegebenen Bedeutungen haben, und R¹ und R² jeweils unabhängig voneinander n-Alkyl, Alkoxy, Oxaalkyl, Fluoralkyl oder Alkenyl mit jeweils bis zu 6 C-Atomen bedeuten, und vorzugsweise jeweils unabhängig voneinander Alkyl mit 1 bis 6 C-Atomen bedeuten; In der Verbindung der Formel XIII bedeutet vorzugsweise mindestens einer der Reste R¹ und R² Alkenyl mit 2 bis 6 C-Atomen.
- Das Medium enthält eine oder mehrere Verbindungen der Formel XIII, worin mindestens einer der Reste R¹ und R² Alkenyl mit 2 bis 6 C-Atomen bedeutet, vorzugsweise solche ausgewählt aus folgenden Formeln, 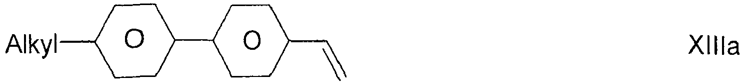 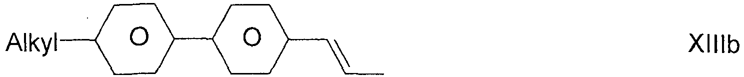 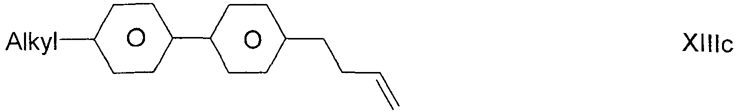 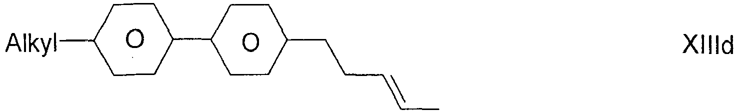
- Das Medium enthält eine oder mehrere Verbindungen der Formel XIIIe, 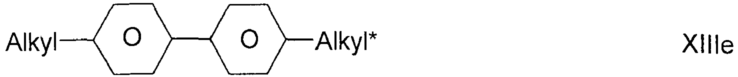 worin "Alkyl" und Alkyl*" die oben angegebenen Bedeutungen haben;
- Das Medium enthält eine oder mehrere Verbindungen der folgenden Formeln, 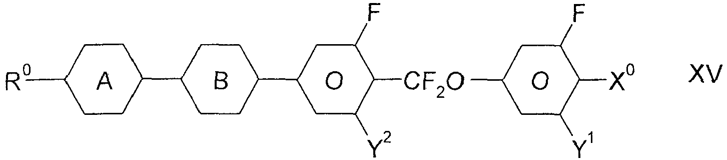 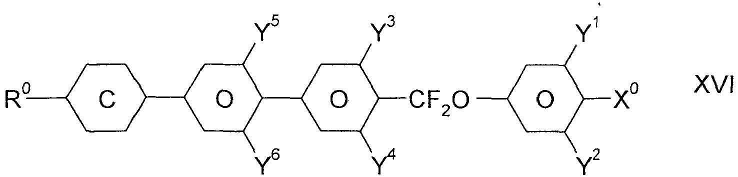 worin R⁰, X⁰ und Y¹⁻⁴ die in Formel I angegebenen Bedeutungen besitzen, und 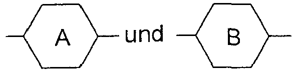 jeweils unabhängig voneinander 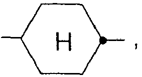 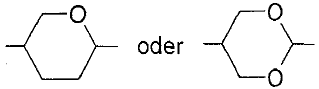 bedeuten,
   und 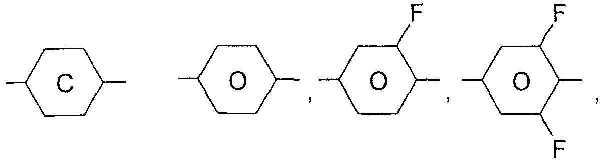 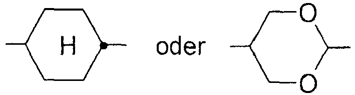 bedeutet;
- Die Verbindungen der Formeln XV und XVI sind vorzugsweise ausgewählt aus den folgenden Formeln, 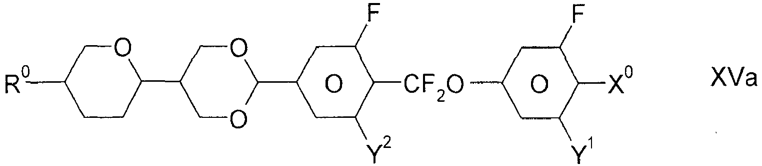 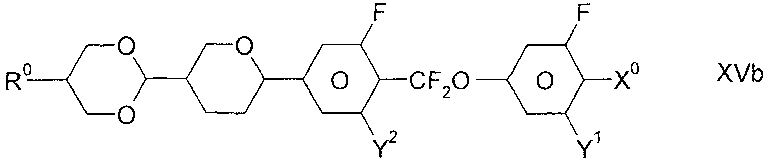 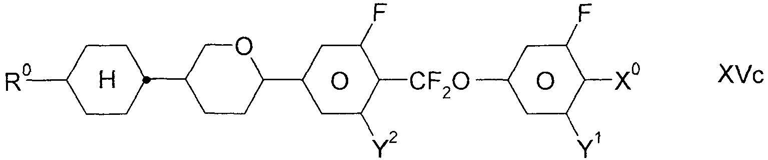 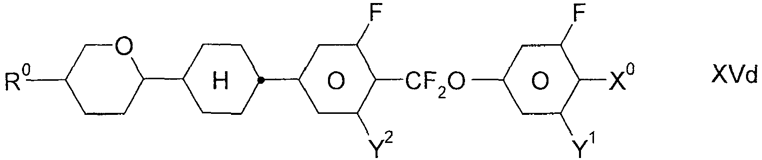 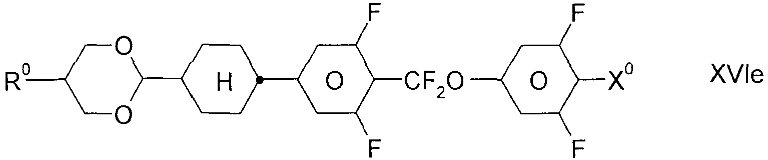 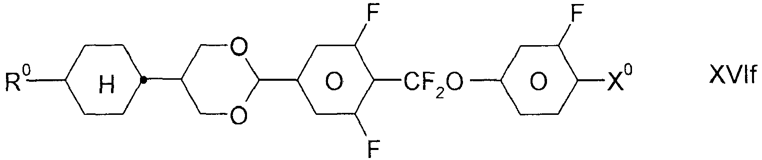 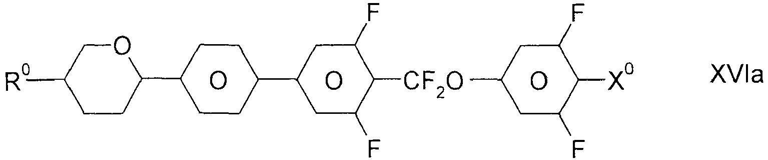 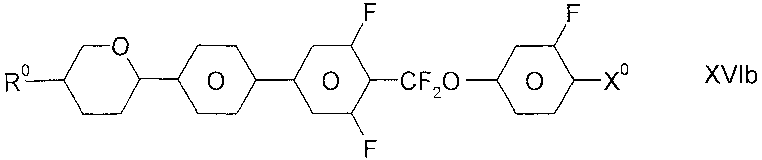 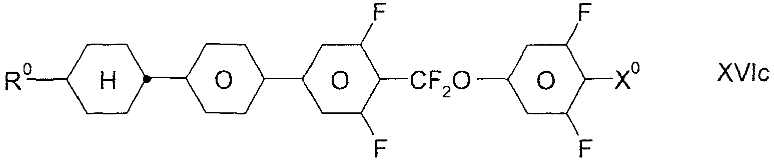 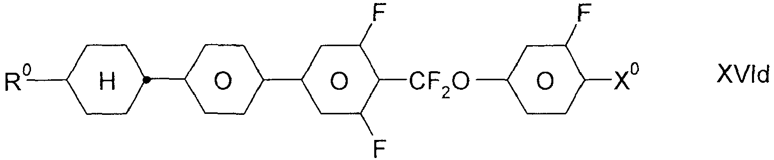 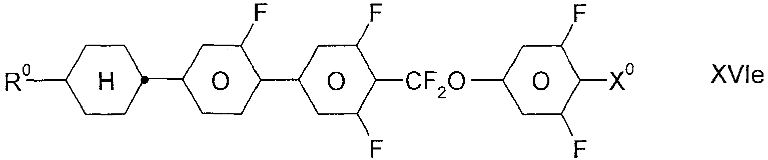 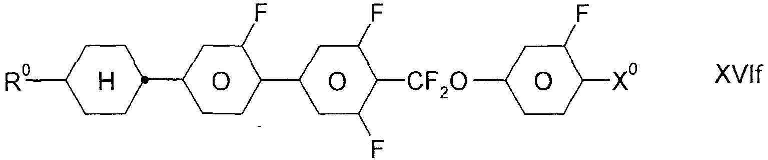 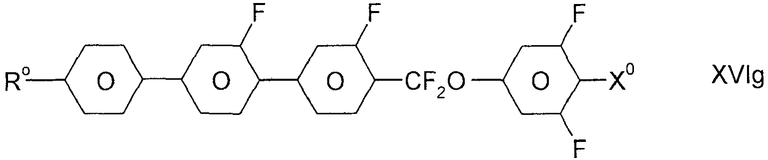 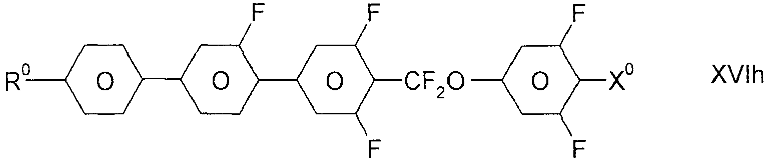 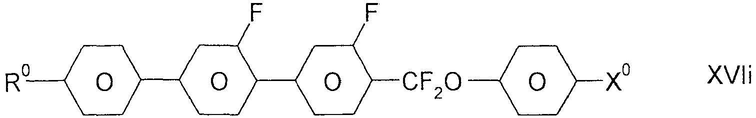 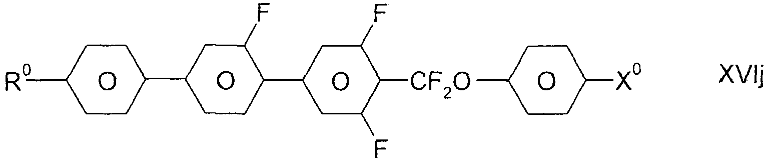 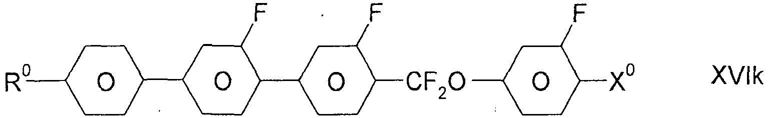 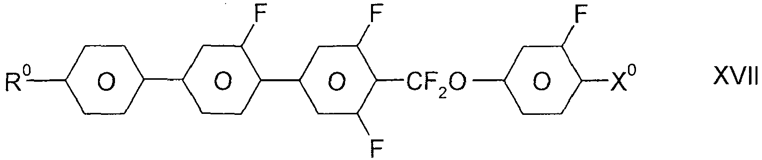 worin R⁰ und X⁰ die oben angegebenen Bedeutungen haben. Vorzugsweise bedeutet R⁰ Alkyl mit 1 bis 6 C-Atomen. X⁰ bedeutet vorzugsweise F, ferner OCF₃. Besonders bevorzugte Verbindungen der Formeln XV und XVa-XVf sind solche, worin Y¹ F und Y² H oder F, vorzugsweise F, bedeutet. Besonders bevorzugt enthält die erfindungsgemäße Mischung mindestens eine Verbindung der Formel XVf.
- Das Medium enthält eine oder mehrere Verbindungen der Formel XVII, 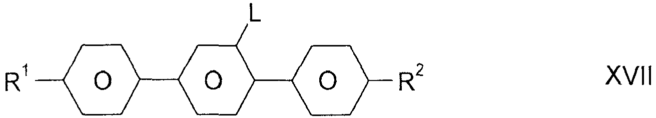 worin R¹ und R² die oben angegebenen Bedeutungen besitzen, und vorzugsweise jeweils unabhängig voneinander Alkyl mit 1 bis 6 C-Atomen bedeuten. L bedeutet H oder F.

Besonders bevorzugte Verbindungen der Formel XVII sind solche der Unterformeln, 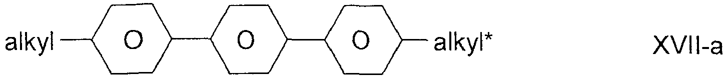 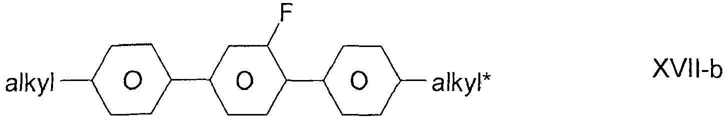 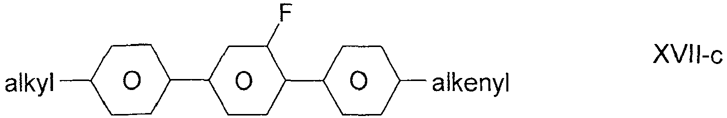 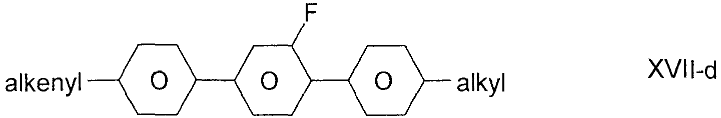 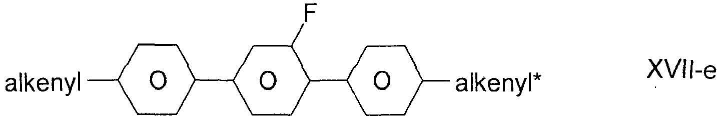 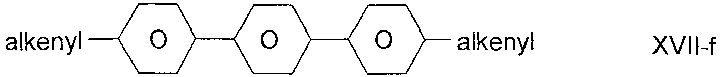 worin
- alkyl und alkyl*: jeweils unabhängig voneinander geradkettiger Alkylrest mit 1-6 C-Atomen, insbesondere Ethyl, Propyl und Pentyl,
- alkenyl und alkenyl*: jeweils unabhängig voneinander geradkettiger Alkenylrest mit 2-6 C-Atomen, insbesondere CH₂=CHC₂H₄, CH₃CH=CHC₂H₄, CH₂=CH und CH₃CH=CH,
bedeuten.

Besonders bevorzugt sind die Verbindungen der Formeln XVII-b und XVII-c. Ganz besonders bevorzugt sind die Verbindungen der Formeln 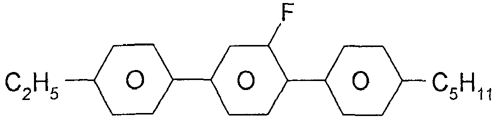 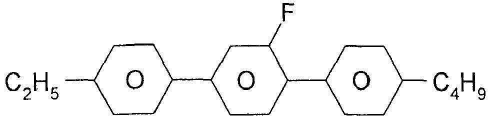 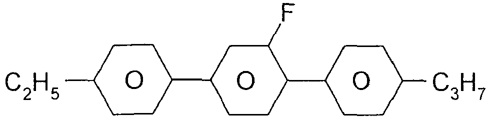 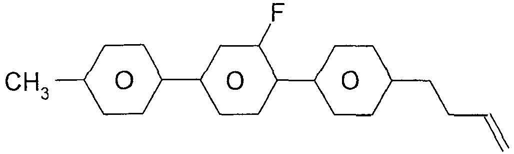 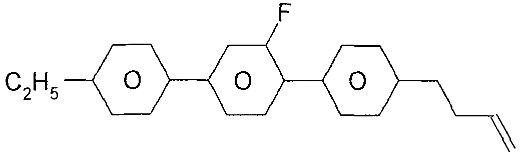 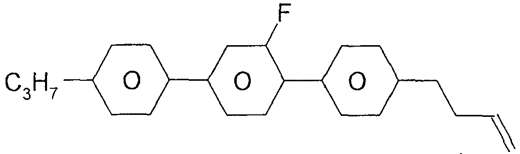
- Das Medium enthält zusätzlich eine oder mehrere Verbindungen der folgenden Formeln, 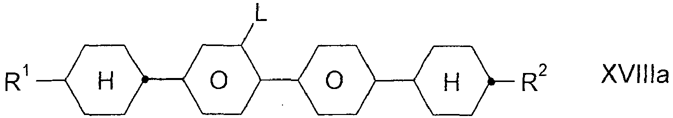 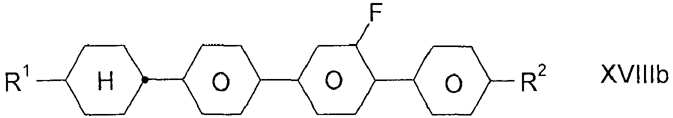 worin R¹ und R² die oben angegebenen Bedeutungen besitzen, und vorzugsweise jeweils unabhängig voneinander Alkyl mit 1 bis 6 C-Atomen bedeuten. L bedeutet H oder F;
- Das Medium enthält zusätzlich eine oder mehrere Verbindungen ausgewählt aus den folgenden Formeln, 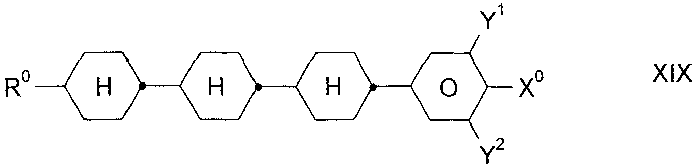 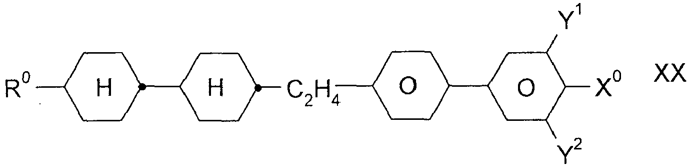 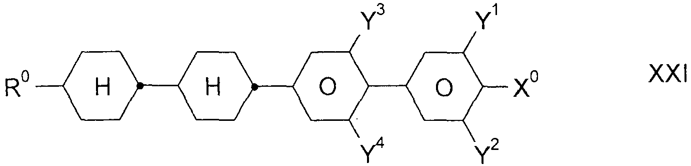 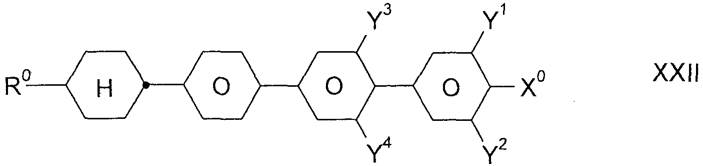 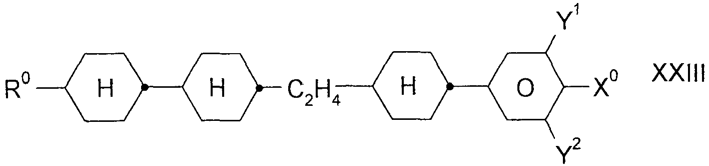 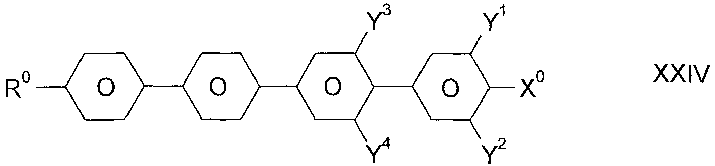 worin R⁰ und X⁰ jeweils unabhängig voneinander eine der oben angegebenen Bedeutungen haben und Y¹⁻⁴ jeweils unabhängig voneinander H oder F bedeuten. X⁰ ist vorzugsweise F, Cl, CF₃, OCF₃ oder OCHF₂. R⁰ bedeutet vorzugsweise Alkyl, Alkoxy, Oxaalkyl, Fluoralkyl oder Alkenyl mit jeweils bis zu 6 C-Atomen.

Besonders bevorzugt erhält die erfindungsgemäße Mischung eine oder mehrere Verbindungen der Formel XXIV-a, 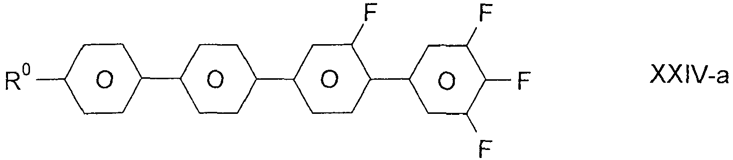 worin R⁰ die oben angegebene Bedeutungen hat. Vorzugsweise bedeutet R⁰ geradkettiges Alkyl, insbesondere Ethyl, n-Propyl, n-Butyl und n-Pentyl, und ganz besonders bevorzugt n-Propyl. Die Verbindung(en) der Formel XXIV, insbesondere der Formel XXIV-a werden vorzugsweise in Mengen von 0,5-20 Gew.%, besonders bevorzugt 1-15 Gew.% in den erfindungsgemäßen Mischungen eingesetzt.
- Das Medium enthält zusätzlich eine oder mehrere Verbindungen aus der Formel XXIV, 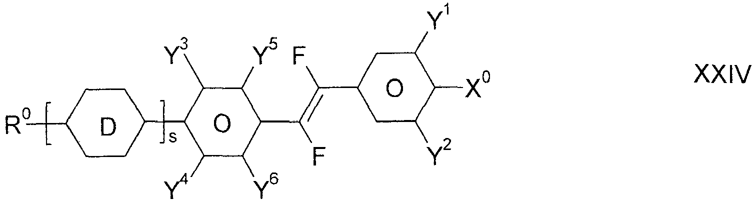 worin R⁰, X⁰ und Y¹⁻⁶ die in Formel I angegebene Bedeutung besitzen, s 0 oder 1, und 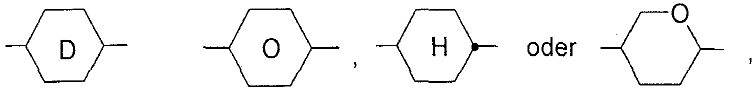
bedeuten.

In der Formel XXIV kann X⁰ auch ein Alkylrest mit 1-6 C-Atomen oder ein Alkoxyrest mit 1-6 C-Atomen bedeuten. Vorzugsweise ist der Alkyl- oder Alkoxyrest geradkettig.

Vorzugsweise bedeutet R⁰ Alkyl mit 1 bis 6 C-Atomen. X⁰ bedeutet vorzugsweise F;
- Die Verbindungen der Formel XXIV sind vorzugsweise ausgewählt aus den folgenden Formeln, 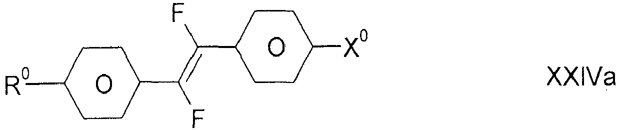 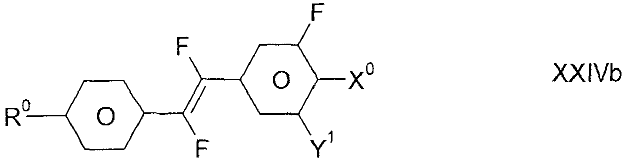 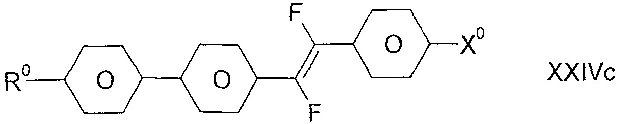 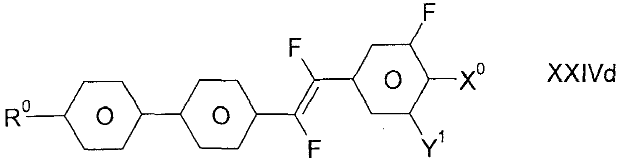 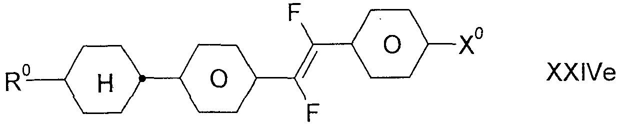 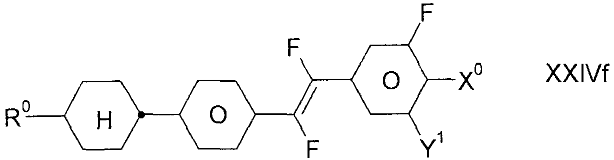 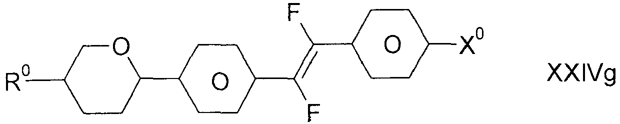 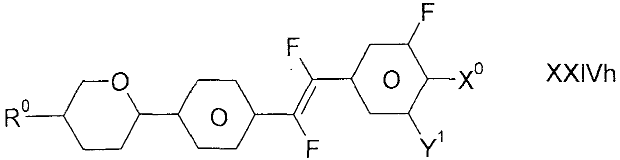 worin R⁰, X⁰ und Y¹ die oben angegebenen Bedeutungen haben. Vorzugsweise bedeutet R⁰ Alkyl mit 1 bis 6 C-Atomen. X⁰ bedeutet vorzugsweise F und Y¹ ist vorzugsweise F; 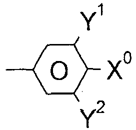 ist vorzugsweise 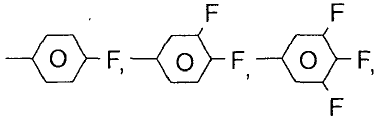 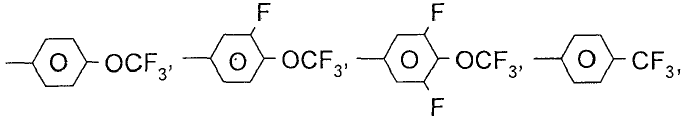 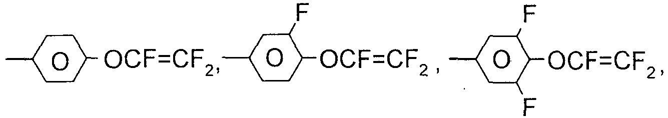 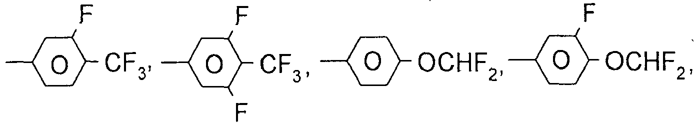 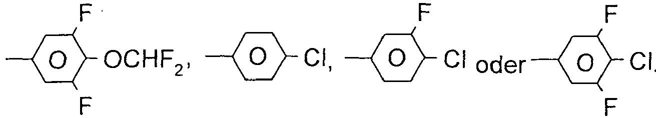
- R⁰ ist geradkettiges Alkyl oder Alkenyl mit 2 bis 6 C-Atomen;
- Das Medium enthält eine oder mehrere Verbindungen der folgenden Formeln 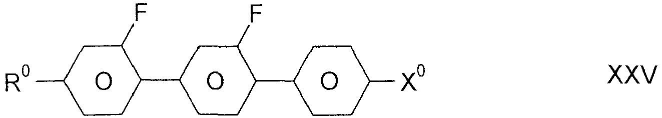 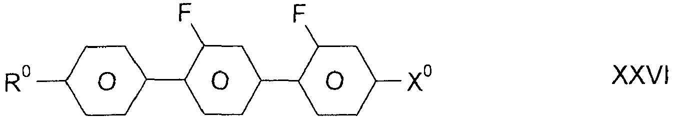 worin R⁰ und X⁰ die oben angegebenen Bedeutungen besitzen. R⁰ bedeutet vorzugsweise Alkyl mit 1 bis 6 C-Atomen. X⁰ bedeutet vorzugsweise F oder Cl. In der Formel XXV bedeutet X⁰ ganz besonders bevorzugt Cl.
- Das Medium enthält eine oder mehrere Verbindungen der folgenden Formeln, 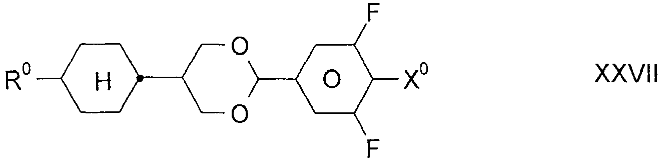 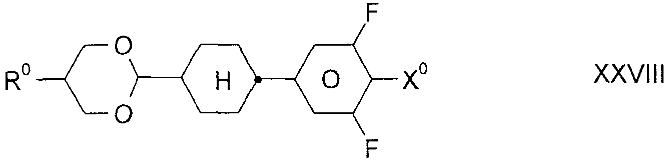 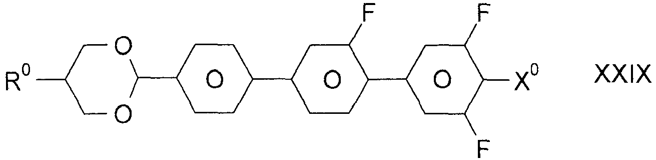 worin R⁰ und X⁰ die oben angegebene Bedeutung besitzen. R⁰ bedeutet vorzugsweise Alkyl mit 1 bis 6 C-Atomen. X⁰ bedeutet vorzugsweise F; Insbesondere bevorzugt enthält das erfindungsgemäße Medium eine oder mehrere Verbindungen der Formel XXIX, worin X⁰ vorzugsweise F bedeutet. Die Verbindung(en) der Formeln XXVII - XXIX werden vorzugsweise in Mengen von 1-20 Gew.%, besonders bevorzugt 1-15 Gew.% in den erfindungsgemäßen Mischungen eingesetzt. Besonders bevorzugte Mischungen enthalten mindestens eine Verbindung der Formel XXIX.
- Das Medium enthält eine oder mehrere Verbindungen der folgenden Pyrimidin- oder Pyridin-Verbindungen der Formeln, 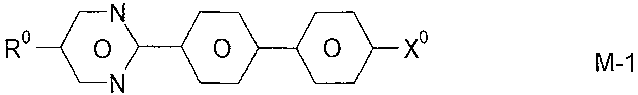 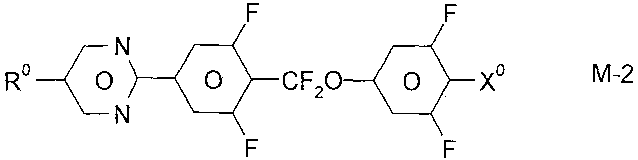 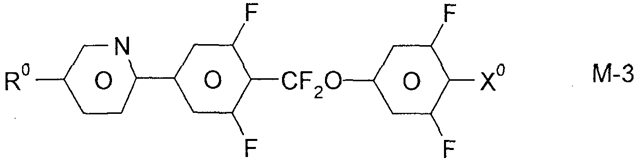
worin R⁰ und X⁰ die oben angegebene Bedeutung besitzen. R⁰ bedeutet vorzugsweise Alkyl mit 1 bis 6 C-Atomen. X⁰ bedeutet vorzugsweise F; Insbesondere bevorzugt enthält das erfindungsgemäße Medium eine oder mehrere Verbindungen der Formel M-1, worin X⁰ vorzugsweise F. bedeutet. Die Verbindung(en) der Formeln M-1 bis M-3 werden vorzugsweise in Mengen von 1-20 Gew.%, besonders bevorzugt 1-15 Gew.% in den erfindungsgemäßen Mischungen eingesetzt.

Nachfolgend werden weitere bevorzugte Ausführungsformen angegeben:
- Das Medium enthält zwei oder mehr Verbindungen der Formel I, insbesondere der Formel I-4;
- Das Medium enthält 2-40 Gew.%, bevorzugt 4-30 Gew.%, besonders bevorzugt 3-15 Gew.% an Verbindungen der Formel I, insbesondere eine oder mehrere Verbindungen der Formel I-4;
- Das Medium enthält neben einer oder mehreren Verbindungen der Formeln I ein oder mehrere Verbindungen ausgewählt aus der Gruppe der Verbindungen der Formeln II, III, IX-XIII, XVII und XVIII;
- Der Anteil an Verbindungen der Formeln II, III, IX-XIII, XVII und XVIII im Gesamtgemisch beträgt 40 bis 95 Gew.%;
- Das Medium enthält 10-50 Gew.%, besonders bevorzugt 12-40 Gew.% an Verbindungen der Formel II und/oder III;
- Das Medium enthält 20-70 Gew.%, besonders bevorzugt 25-65 Gew.% an Verbindungen der Formeln IX-XIII;
- Das Medium enthält 4-30 Gew.%, besonders bevorzugt 5-20 Gew.% an Verbindungen der Formel XVII;
- Das Medium enthält 1-20 Gew.%, besonders bevorzugt 2-15 Gew.% an Verbindungen der Formel XVIII.
- Das Medium enthält mindestens zwei Verbindungen der Formeln 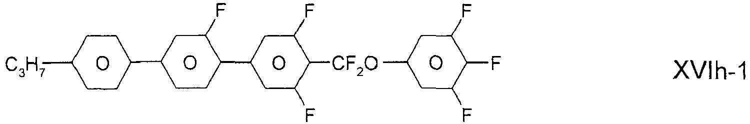 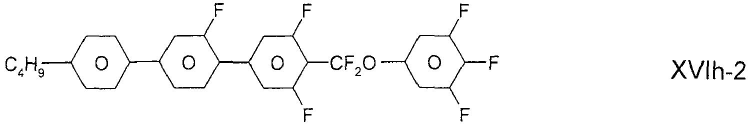 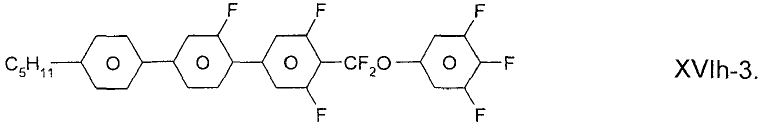
- Das Medium enthält mindestens zwei Verbindungen der Formeln 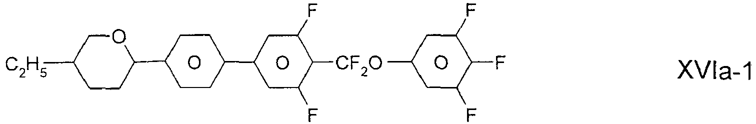 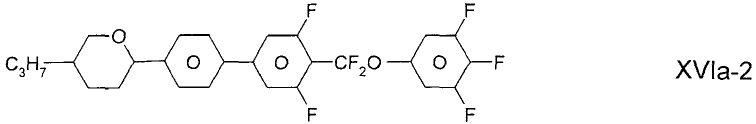 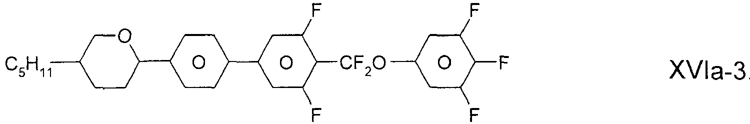
- Das Medium enthält mindestens eine Verbindungen der Formel I und mindestens eine Verbindung der Formel IIIa;
- Das Medium enthält ≥ 20 Gew.%, vorzugsweise ≥ 24 Gew.%, vorzugsweise 25-60 Gew.%, an Verbindungen der Formel IXb, insbesondere die Verbindung der Formel IXb-1, 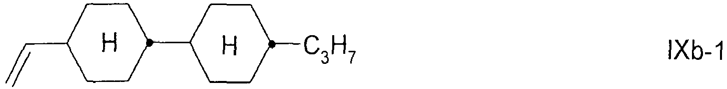
- Das Medium enthält mindestens eine Verbindung der Formel IXb-1 und mindestens eine Verbindung der Formel IXc-1: 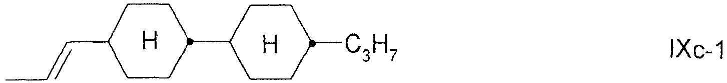
- Das Medium enthält mindestens eine Verbindung der Formel DPGU-n-F.
- Das Medium enthält mindestens eine Verbindung der Formel CDUQU-n-F.
- Das Medium enthält mindestens eine Verbindung der Formel CPU-n-OXF.
- Das Medium enthält mindestens eine Verbindung der Formel PPGU-n-F.
- Das Medium enthält mindestens eine Verbindung der Formel PGP-n-m, vorzugsweise zwei oder drei Verbindungen.
- Das Medium enthält mindestens eine Verbindung der Formel PGP-2-2V mit der Struktur

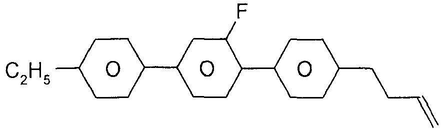

Es wurde gefunden, dass ≥ 2 Gew.% einer oder mehrerer Verbindungen der Formel I im Gemisch mit üblichen Flüssigkristallmaterialien, insbesondere jedoch mit einer oder mehreren Verbindungen der Formeln II bis XXVIII zu einer beträchtlichen Erhöhung der Lichtstabilität und zu hohen Werten für die Doppelbrechung führt, wobei gleichzeitig breite nematische Phasen mit tiefen Übergangstemperaturen smektisch-nematisch beobachtet werden, wodurch die Lagerstabilität verbessert wird. Gleichzeitig zeigen die Mischungen sehr niedrige Schwellenspannungen und sehr gute Werte für die VHR bei UV-Belastung und sehr hohe Klärpunkte.

Der Ausdruck "Alkyl" bzw. "Alkyl*" oder "alkyl" bzw. "alkyl*" umfasst in dieser Anmeldung geradkettige und verzweigte Alkylgruppen mit 1-6 Kohlenstoffatomen, insbesondere die geradkettigen Gruppen Methyl, Ethyl, Propyl, Butyl, Pentyl und Hexyl. Gruppen mit 2-5 Kohlenstoffatomen sind im allgemeinen bevorzugt.

Der Ausdruck "Alkenyl" bzw. "Alkenyl*" umfasst geradkettige und verzweigte Alkenylgruppen mit 2-6 Kohlenstoffatomen, insbesondere die geradkettigen Gruppen. Bevorzugte Alkenylgruppen sind C₂-C₇-1E-Alkenyl, C₄-C₆-3E-Alkenyl, insbesondere C₂-C₆-1E-Alkenyl. Beispiele besonders bevorzugter Alkenylgruppen sind Vinyl, 1 E-Propenyl, 1 E-Butenyl, 1 E-Pentenyl, 1 E-Hexenyl, 3-Butenyl, 3E-Pentenyl, 3E-Hexenyl, 4-Pentenyl, 4Z-Hexenyl, 4E-Hexenyl und 5-Hexenyl. Gruppen mit bis zu 5 Kohlenstoffatomen sind im allgemeinen bevorzugt, insbesondere CH₂=CH, CH₃CH=CH, CH₃CH₂CH₂CH₂=CH oder CH₃ CH₂CH₂=CH.

Der Ausdruck "Fluoralkyl" umfasst vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" bzw. "Alkoxy" umfasst vorzugsweise geradkettige Reste der Formel CₙH₂ₙ₊₁-O-(CH₂)ₘ, worin n und m jeweils unabhängig voneinander 1 bis 6 bedeuten. m kann auch 0 bedeuten. Vorzugsweise ist n = 1 und m 1-6 oder m = 0 und n = 1-3.

Durch geeignete Wahl der Bedeutungen von R⁰ und X⁰ können die Ansprechzeiten, die Schwellenspannung, die Steilheit der Transmissionskennlinien etc. in gewünschter Weise modifiziert werden. Beispielsweise führen 1 E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten. Die erfindungsgemäßen Mischungen zeichnen sich insbesondere durch hohe An-Werte aus und besitzen somit deutlich schnellere Schaltzeilen als die Mischungen aus dem Stand der Technik.

Das optimale Mengenverhältnis der Verbindungen der oben genannten Formeln hängt weitgehend von den gewünschten Eigenschaften, von der Wahl der Komponenten der oben genannten Formeln und der Wahl weiterer gegebenenfalls vorhandener Komponenten ab.

Geeignete Mengenverhältnisse innerhalb des oben angegebenen Bereichs können von Fall zu Fall leicht ermittelt werden.

Die Gesamtmenge an Verbindungen der oben genannten Formeln in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die gewünschte Verbesserung der Eigenschaften der Mischung ist jedoch in der Regel umso größer je höher die Gesamtkonzentration an Verbindungen der oben genannten Formeln ist.

In einer besonders bevorzugten Ausführungsform enthalten die erfindungsgemäßen Medien Verbindungen der Formel II bis VIII (vorzugsweise II, III, IV und V, insbesondere IIa und IIIa), worin X⁰ F, OCF₃, OCHF₂, OCH=CF₂, OCF=CF₂ oder OCF₂-CF₂H bedeutet. Eine günstige synergistische Wirkung mit einer oder mehreren Verbindungen der Formel I führt zu besonders vorteilhaften Eigenschaften. Insbesondere Mischungen enthaltend eine oder mehrere Verbindungen der Formel I, IIa und IIIa zeichnen sich durch ihre niedrige Schwellenspannung aus.

Die einzelnen Verbindungen der oben genannten Formeln und deren Unterformeln, die in den erfindungsgemäßen Medien verwendet werden können, sind entweder bekannt, oder sie können analog zu den bekannten Verbindungen hergestellt werden.

Gegenstand der Erfindung sind auch elektrooptische Anzeigen, wie z. B. STN- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes. Die erzielbaren Kombinationen aus Klärpunkt, Viskosität bei tiefer Temperatur, thermischer und UV-Stabilität und hoher optischer Anisotropie übertreffen bei weitem bisherige Materialien aus dem Stand der Technik.

Die erfindungsgemäßen Mischungen sind insbesondere für mobile Anwendungen und TFT-Anwendungen, wie z. B. Mobiltelefone und PDAs geeignet. Weiterhin können die erfindungsgemäßen Mischungen in FFS-, VA-IPS, OCB- und IPS-Anzeigen Anwendung finden.

Die erfindungsgemäßen Flüssigkristallmischungen ermöglichen es, bei Beibehaltung der nematischen Phase bis -20 °C und bevorzugt bis -30 °C, besonders bevorzugt bis -40 °C, und des Klärpunkts ≥ 75 °C, vorzugsweise ≥ 80 °C, gleichzeitig Rotationsviskositäten γ₁ von ≤ 110 mPa·s, besonders bevorzugt ≤ 100 mPa·s zu erreichen, wodurch hervorragende MFK-Anzeigen mit schnellen Schaltzeiten erzielt werden können. Die Rotationsviskositäten sind bei 20 °C bestimmt.

Die dielektrische Anisotropie der erfindungsgemäßen Flüssigkristallmischungen Δε ist bei 20 °C vorzugsweise ≥ +8, besonders bevorzugt ≥ +10, insbesondere bevorzugt ≥ 12. Die Mischungen sind außerdem durch kleine Operationsspannungen gekennzeichnet. Die Schwellenspannung der erfindungsgemäßen Flüssigkristallmischungen ist vorzugsweise ≤ 2.0 V. Die Doppelbrechung Δn der erfindungsgemäßen Flüssigkristallmischungen ist bei 20 °C vorzugsweise ≥ 0,09, besonders bevorzugt ≥ 0,10.

Der nematische Phasenbereich der erfindungsgemäßen Flüssigkristallmischungen ist vorzugsweise mindestens 90°, insbesondere mindestens 100° breit. Vorzugsweise erstreckt sich dieser Bereich mindestens von -25° bis +70°C.

Es versteht sich, dass durch geeignete Wahl der Komponenten der erfindungsgemäßen Mischungen auch höhere Klärpunkte (z.B. oberhalb 100 °C) bei höheren Schwellenspannungen oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringen Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften, wie z.B. hohe Steilheit der Kennlinie und geringe Winkelabhängigkeit des Kontrastes (DE-PS 30 22 818) bei gleicher Schwellenspannung, wie in einer analogen Anzeige im zweiten Minimum, eine kleinere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Messungen des "Voltage Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, dass erfindungsgemäße Mischungen enthaltend Verbindungen der Formel IA und IB eine deutlich geringere Abnahme des HR unter UV-Belastung aufweisen als analoge Mischungen enthaltend anstelle den Verbindungen der Formel IA und IB Cyanophenylcyclohexane der Formel 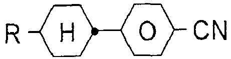 oder Ester der Formel

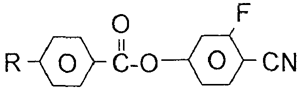

Die Lichtstabilität und UV-Stabilität der erfindungsgemäßen Mischungen ist erheblich besser, d.h. sie zeigen eine deutlich kleinere Abnahme des HR unter Licht- bzw. UV-Belastung.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht. Die Herstellung der erfindungsgemäß verwendbaren Flüssigkristallmischungen erfolgt in an sich üblicher Weise, beispielsweise indem man eine oder mehrere Verbindungen der Formeln I mit einer oder mehreren Verbindungen der Formeln II-XXVIII oder mit weiteren flüssigkristallinen Verbindungen und ggf. mit Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze, wie z. B. UV-Stabilisatoren wie Tinuvin^{®}, z.B. Tinuvin^{®} 770, der Fa. Ciba Chemicals, Antioxidantien, z.B. TEMPOL, Mikropartikel, Radikalfänger, Nanopartikel, etc. enthalten. Beispielsweise können 0-15 % pleochroitische Farbstoffe oder chirale Dotierstoffe zugesetzt werden. Geeignete Stabilisatoren und Dotierstoffe werden nachfolgend in den Tabellen C und D genannt.

Den erfindungsgemäßen Mischungen können weiterhin polymerisierbare Verbindungen, sogenannte reaktive Mesogene (RMs), beispielsweise wie in U.S. 6,861,107 offenbart, in Konzentrationen von bevorzugt 0,12 - 5 Gew.%, besonders bevorzugt 0,2 - 2 % bezogen auf die Mischung, zugesetzt werden. Optional können diese Mischungen auch einen Initiator enthalten, wie beispielsweise in der U.S 6,781,665 beschrieben. Der Initiator, z.B. Irganox-1076 der Fa. Ciba, wird vorzugsweise der Mischung enthaltend polymerisierbare Verbindungen in Mengen von 0-1 % zugesetzt. Derartige Mischungen können für sogenannte Polymer Stabilized VA-Modes (PS-VA) oder PSA (Polymer sustained VA), bei denen eine Polymerisierung der reaktiven Mesogene in der flüssigkristallinen Mischung erfolgen soll, verwendet werden. Voraussetzung hierfür ist, dass die Flüssigkristallmischung selbst keine polymerisierbaren Komponenten enthält.

In einer bevorzugten Ausführungsform der Erfindung sind die polymerisierbaren Verbindungen ausgewählt aus den Verbindungen der Formel M

R^{a}-A¹-(Z¹-A²)ₘ-R^{b} M

worin die einzelnen Reste folgende Bedeutung haben:
- R^{a} und R^{b}: jeweils unabhängig voneinander P, P-Sp-, H, Halogen, SF₅, NO₂, eine Kohlenstoffgruppe oder Kohlenwasserstoffgruppe, wobei mindestens einer der Reste R^{a} und R^{b} eine Gruppe P oder P-Sp- bedeutet oder enthält,
- P: bei jedem Auftreten gleich oder verschieden eine polymerisierbare Gruppe,
- Sp: bei jedem Auftreten gleich oder verschieden eine Abstandsgruppe oder eine Einfachbindung,
- A¹ und A²: jeweils unabhängig voneinander eine aromatische, heteroaromatische, alicyclische oder heterocyclische Gruppe, vorzugsweise mit 4 bis 25 Ringatomen, welche auch anellierte Ringe enthalten kann, und welche auch durch L ein- oder mehrfach substituiert sein kann,
- L: P-Sp-, H, OH, CH₂OH, Halogen, SF₅, NO₂, eine Kohlenstoffgruppe oder Kohlenwasserstoffgruppe,
- Z¹: bei jedem Auftreten gleich oder verschieden -O-, -S-, -CO-, -CO-O-, -OCO-, -O-CO-O-, -OCH₂-, -CH₂O-, -SCH₂-, -CH₂S-, -CF₂O-, -OCF₂-, -CF₂S-, -SCF₂-, -(CH₂)ₙ₁-, -CF₂CH₂-, -CH₂CF₂-, -(CF₂)ₙ₁-, -CH=CH-, -CF=CF-, -C≡C-, -CH=CH-COO-, -OCO-CH=CH-, CR⁰R⁰⁰ oder eine Einfachbindung,
- R⁰ und R⁰⁰: jeweils unabhängig voneinander H oder Alkyl mit 1 bis 12 C-Atomen,
- m: 0, 1, 2, 3 oder 4,
- n1: 1, 2, 3 oder 4.

Besonders bevorzugte Verbindungen der Formel M sind solche, worin
- R^{a} und R^{b}: jeweils unabhängig voneinander P, P-Sp-, H, F, Cl, Br, I, -CN,-NO₂, -NCO, -NCS, -OCN, -SCN, SF₅ oder geradkettiges oder verzweigtes Alkyl mit 1 bis 25 C-Atomen, worin auch eine oder mehrere nicht benachbarte CH₂-Gruppen jeweils unabhängig voneinander durch -C(R⁰)=C(R⁰⁰)-, -C≡C-, -N(R⁰⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, Br, I, CN, P oder P-Sp- ersetzt sein können, wobei mindestens einer der Reste R^{a} und R^{b} eine Gruppe P oder P-Sp- bedeutet oder enthält,
- A¹ und A²: jeweils unabhängig voneinander 1,4-Phenylen, Naphthalin-1,4-diyl, Naphthalin-2,6-diyl, Phenanthren-2,7-diyl, Anthracen-2,7-diyl, Fluoren-2,7-diyl, 2-Oxo-2H-chromen-3,6-diyl, 2-Oxo-2H-Chromen-3,7-diyl, 4-Oxo-4H-chromen-2,6-diyl, 4-Oxo-4H-chromen-3,6-diyl, 4-Oxo-4H-chromen-3,7-diyl (Trivialname Cumarin bzw. Flavon), wobei in diesen Gruppen auch eine oder mehrere CH-Gruppen durch N ersetzt sein können, Cyclohexan-1,4-diyl, worin auch eine oder mehrere nichtbenachbarte CH₂-Gruppen durch O und/oder S ersetzt sein können, 1,4-Cyclohexenylen, Bicyclo[1.1.1]pentan-1,3-diyl, Bicyclo[2.2.2]octan-1,4-diyl, Spiro[3.3]heptan-2,6-diyl, Piperidin-1,4-diyl, Decahydronaphthalin-2,6-diyl, 1,2,3,4-Tetrahydronaphthalin-2,6-diyl, Indan-2,5-diyl oder Octahydro-4,7-methano-indan-2,5-diyl, wobei alle diese Gruppen unsubstituiert oder durch L ein- oder mehrfach substituiert sein können,L P, P-Sp-, OH, CH₂OH F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, optional substituiertes Silyl, optional substituiertes Aryl mit 6 bis 20 C Atomen, oder geradkettiges oder verzweigtes Alkyl, Alkoxy, Alkylcarbonyl, Alkoxycarbonyl, Alkylcarbonlyoxy oder Alkoxycarbonyloxy mit 1 bis 25 C-Atomen, worin auch ein oder mehrere H-Atome durch F, Cl, P oder P-Sp- ersetzt sein können,
- P: eine polymerisierbare Gruppe,
- Y¹: Halogen,
- R^{x}: P, P-Sp-, H, Halogen, geradkettiges, verzweigtes oder cyclisches Alkyl mit 1 bis 25 C-Atomen, worin auch eine oder mehrere nicht benachbarte CH₂-Gruppen durch -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, P oder P-Sp- ersetzt sein können, eine optional substituierte Aryl- oder Aryloxygruppe mit 6 bis 40 C-Atomen, oder eine optional substituierte Heteroaryl- oder Heteroaryloxygruppe mit 2 bis 40 C-Atomen,
bedeuten.

Weitere bevorzugte Verbindungen der Formel M sind solche ausgewählt aus einer oder mehreren der folgenden Untergruppen:
- m ist 2 or 3,
- m ist 2,
- R^{a} und R^{b} bedeuten gleiche oder verschiedene Gruppen P-Sp-,
- R^{a} und R^{b} bedeuten gleiche oder verschiedene Gruppen P-Sp-, worin ein oder mehrere Gruppen Sp eine Einfachbindung bedeuten,
- m ist 2 oder 3 und R^{a} und R^{b} bedeuten gleiche Gruppen P-Sp-,
- einer der Reste R^{a} und R^{b} bedeutet P-Sp- und der andere bedeutet eine unpolymerisierbare Gruppe, vorzugsweise geradkettiges oder verzweigtes Alkyl mit 1 bis 25 C-Atomen, worin auch eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, -CO-, -CO-O-, -O-CO- oder -O-CO-O- so ersetzt sein können, dass O-und/oder S-Atome nicht direkt miteinander verknüpft sind, und worin auch ein oder mehrere H-Atome durch F, Cl, Br, I oder CN ersetzt sein können,
- eine oder mehrere Gruppen Sp bedeuten eine Einfachbindung,
- eine oder mehrere Gruppen Sp bedeuten -(CH₂)ₚ₁-, -(CH₂)ₚ₁-O-, -(CH₂)ₚ₁-OCO- oder -(CH₂)ₚ₁-OCOO-, worin p1 eine ganze Zahl von 1 bis 12 und r1 eine ganze Zahl von 1 bis 8 bedeuten,
- L bedeutet und/oder enthält keine polymerisierbare Gruppe,
- A¹ und A² bedeuten unabhängig voneinander 1,4-Phenylen oder Naphthalin-2,6-diyl, wobei in diesen Gruppen auch eine oder mehrere CH-Gruppen durch N ersetzt sein können, und welche auch ein-oder mehrfach fluoriert sein können,
- Z¹ ist ausgewählt aus der Gruppe bestehend aus -O-, -CO-O-, -OCO-, -OCHF, -CH₂O-, -CF₂O- -OCF₂-, -CH₂CH₂-, -CH=CH-, -CF=CF-, -CH=CF-, -CF=CH-, -C≡C- und einer Einfachbindung,
- L ist eine unpolymerisierbare Gruppe, vorzugsweise ausgewählt aus der Gruppe bestehend aus F, Cl, -CN, geradkettigem und verzweigtem Alkyl mit 1 bis 25 C-Atomen, worin auch eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, -CO-, -CO-O-, -O-CO- oder -O-CO-O- so ersetzt sein können, dass O- und/oder S-Atome nicht direkt miteinander verknüpft sind, und worin auch ein oder mehrere H-Atome durch F, Cl, Br, I oder CN ersetzt sein können.

Geeignete und bevorzugte Co-Monomere für die Herstellung von erfindungsgemäßen Mischungen für PS-VA-, PS-IPS und PS-FFS-Anwendungen sind beispielsweise ausgewählt aus den folgenden Formeln: 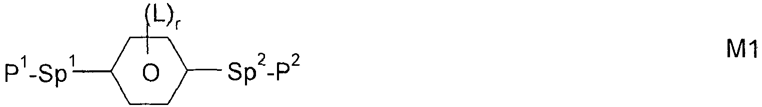 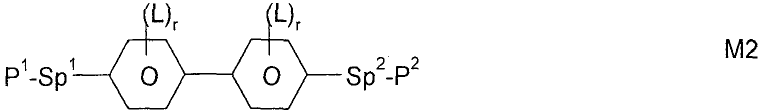 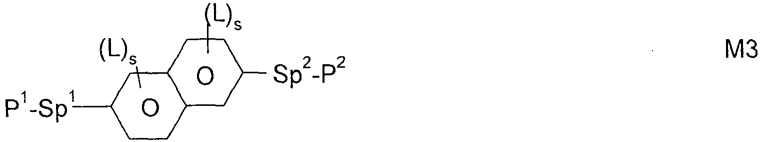 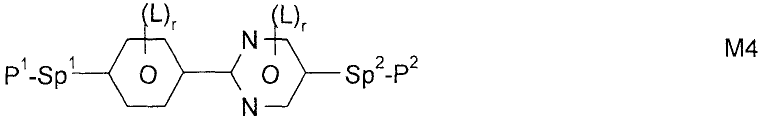 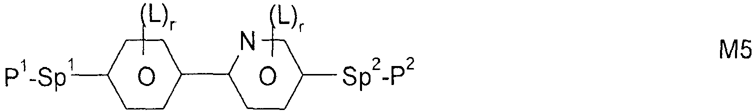 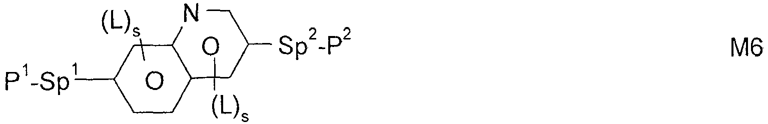 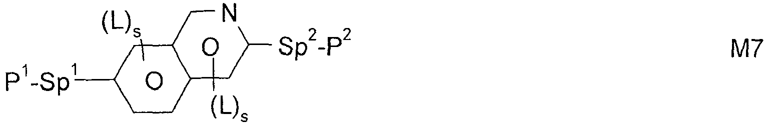 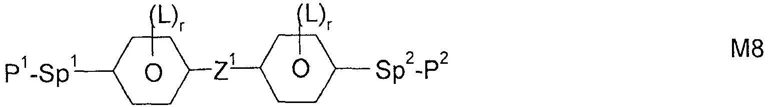 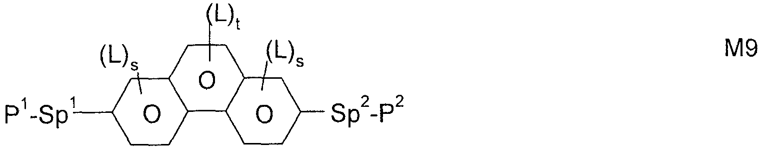 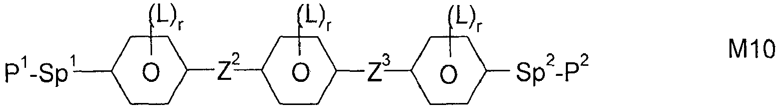 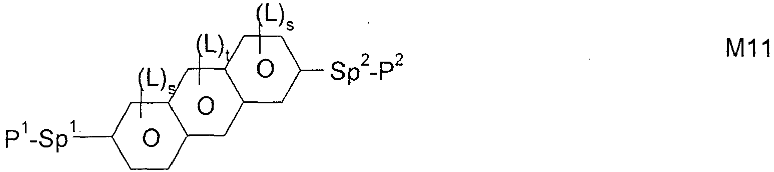 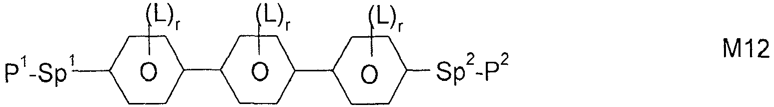 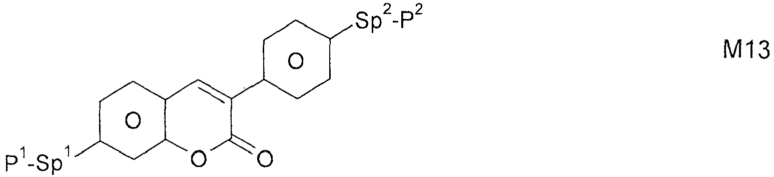 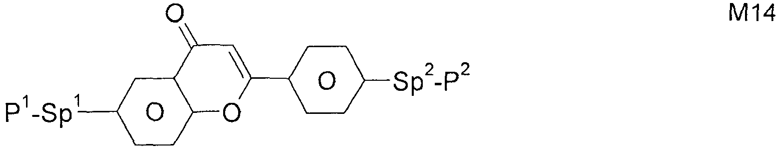 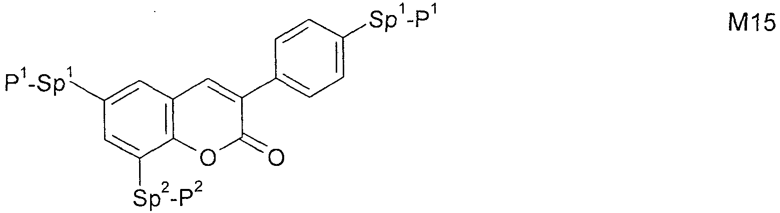 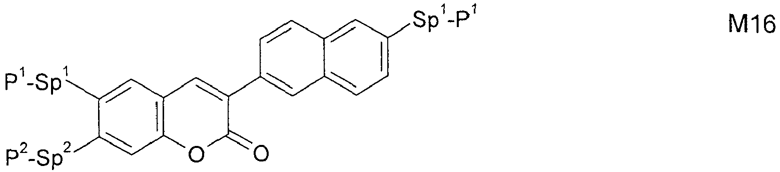 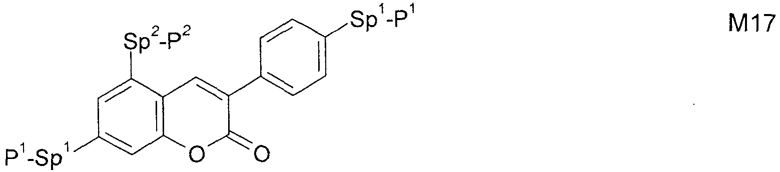 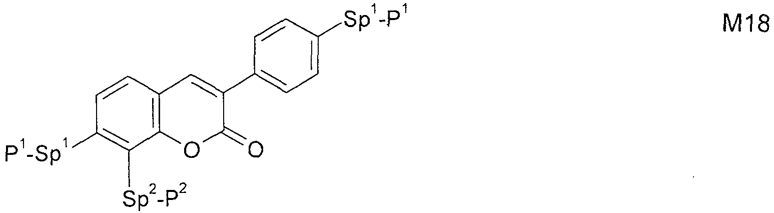 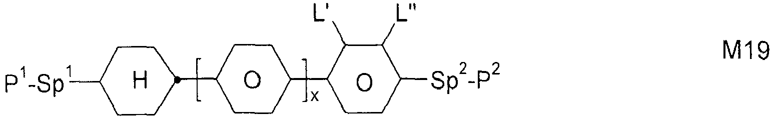 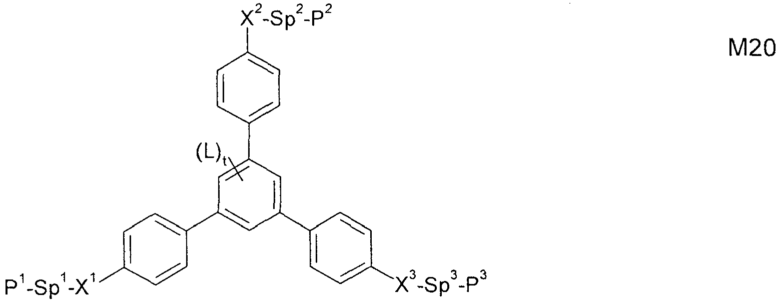 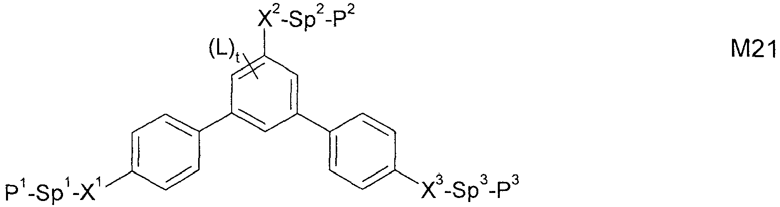 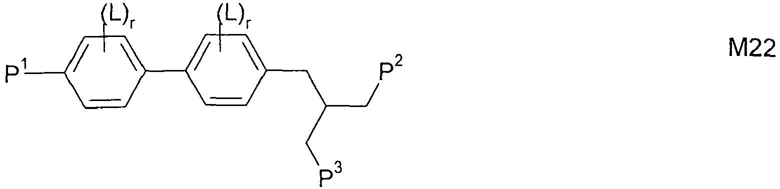 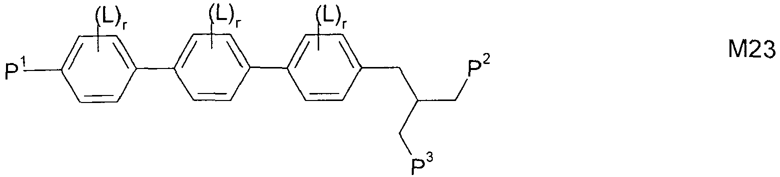 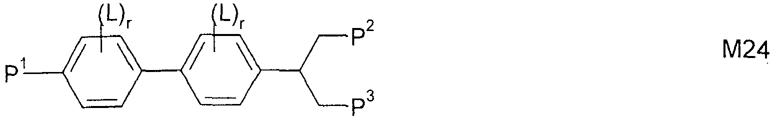 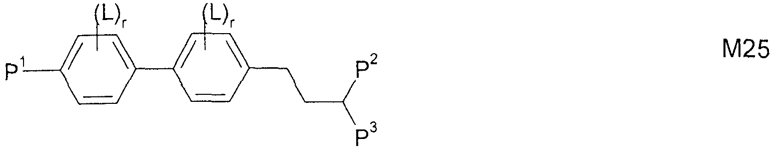 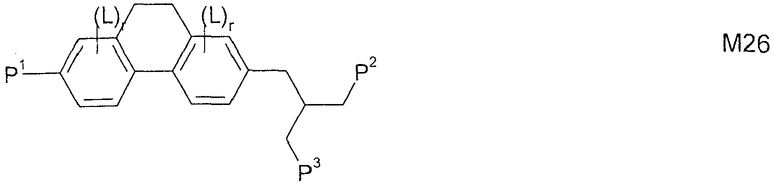 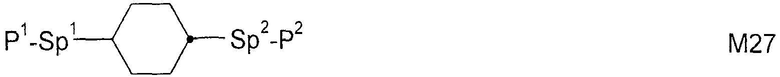 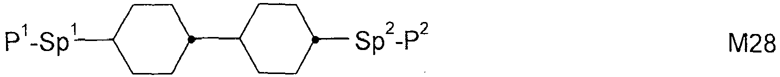 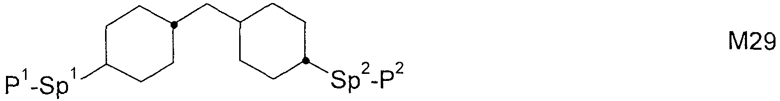 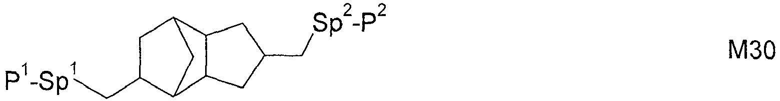 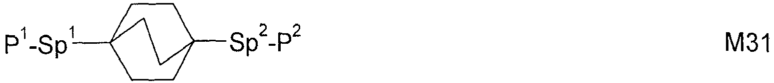 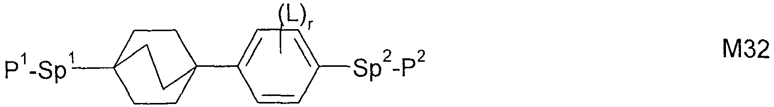 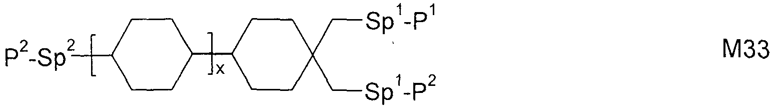 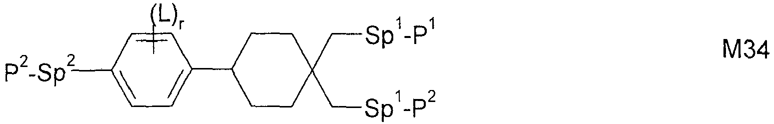 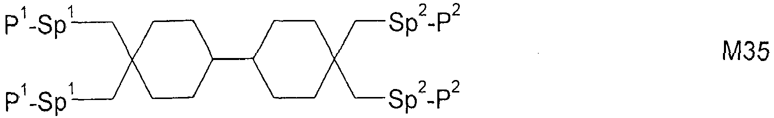 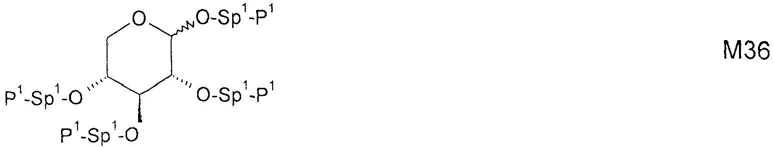 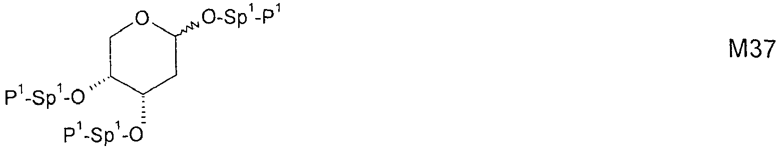 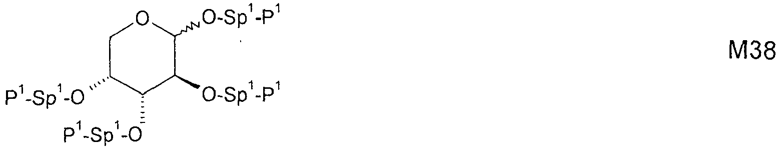 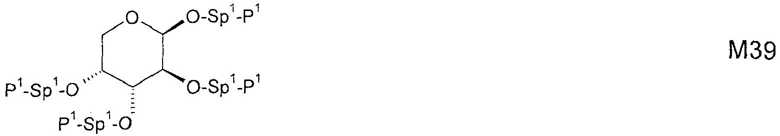 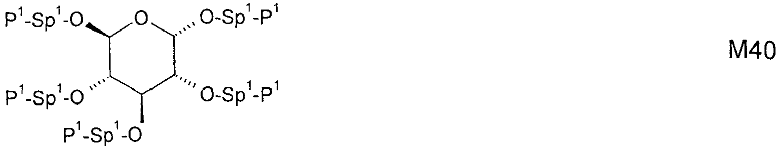 worin die einzelnen Reste folgende Bedeutung besitzen:
- P¹, P² und P³: jeweils unabhängig voneinander eine polymerisierbare Gruppe, vorzugsweise mit einer der vor- und nachstehend für P angegebenen Bedeutungen, besonders bevorzugt eine Acrylat-, Methacrylat-, Fluoracrylat-, Oxetan-, Vinyloxy- oder Epoxygruppe,
- Sp¹, Sp² und Sp³: jeweils unabhängig voneinander eine Einfachbindung oder eine Abstandsgruppe, vorzugsweise mit einer der vor- und nachstehend für Sp^{a} angegebenen Bedeutungen, und besonders bevorzugt-(CH₂)ₚ₁-, -(CH₂)ₚ₁-O-, -(CH₂)ₚ₁-CO-O- oder -(CH₂)ₚ₁-O-CO-O-, worin p1 eine ganze Zahl von 1 bis 12 ist, und wobei in den letztgenannten Gruppen die Verknüpfung zur benachbarten Ring über das O-Atom erfolgt, wobei auch einer oder mehrere der Reste P¹-Sp¹-, P²-Sp²- und P³-Sp³- einen Rest R^{aa} bedeuten können, mit der Maßgabe dass mindestens einer der vorhandenen Reste P¹-Sp¹-, P²-Sp²- und P³-Sp³- nicht R^{aa} bedeutet,
- R^{aa}: H, F, Cl, CN oder geradkettiges oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹-Sp¹-ersetzt sein können, besonders bevorzugt geradkettiges oder verzweigtes, optional ein- oder mehrfach fluoriertes, Alkyl, Alkoxy, Alkenyl, Alkinyl, Alkylcarbonyl, Alkoxycarbonyl, oder Alkylcarbonyloxy mit 1 bis 12 C-Atomen (wobei die Alkenyl- und Alkinylreste mindestens zwei und die verzweigten Reste mindestens drei C-Atome aufweisen),
- R⁰, R⁰⁰: jeweils unabhängig voneinander und bei jedem Auftreten gleich oder verschieden H oder Alkyl mit 1 bis 12 C-Atomen,
- R^{y} und R^{z}: jeweils unabhängig voneinander H, F, CH₃ oder CF₃,
- X¹, X² und X³: jeweils unabhängig voneinander -CO-O-, -O-CO- oder eine Einfachbindung,
- Z¹: -O-, -CO-, -C(R^{y}R^{z})-, oder -CF₂CF₂-,
- Z² und Z³: jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, Cl, CN, SCN, SF₅ oder geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen, vorzugsweise F,
- L' und L": jeweils unabhängig voneinander H, F oder Cl,
- r: 0, 1, 2, 3 oder 4,
- s: 0, 1, 2 oder 3,
- t: 0, 1 oder 2,
- x: 0 oder 1.

In den Verbindungen der Formeln M1 bis M34 bedeutet 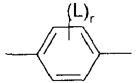 vorzugsweise 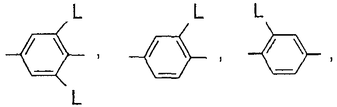 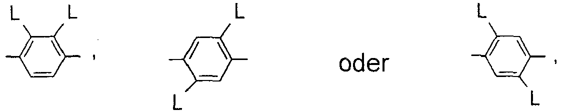 worin L, bei jedem Auftreten gleich oder verschieden, eine der vorstehenden Bedeutungen hat und vorzugsweise F, Cl, CN, NO₂, CH₃, C₂H₅, C(CH₃)₃, CH(CH₃)₂, CH₂CH(CH₃)C₂H₅, OCH₃, OC₂H₅, COCH₃, COC₂H₅, COOCH₃, COOC₂H₅, CF₃, OCF₃, OCHF₂, OC₂F₅ oder P-Sp-, besonders bevorzugt F, Cl, CN, CH₃, C₂H₅, OCH₃, COCH₃, OCF₃ oder P-Sp-, ganz besonders bevorzugt F, Cl, CH₃, OCH₃, COCH₃ oder OCF₃, insbesondere F oder CH₃ bedeutet.

Bevorzugt enthalten die flüssigkristallinen Medien gemäß der vorliegenden Anmeldung insgesamt 0,01 bis 10 %, bevorzugt 0,2 bis 4,0 %, besonders bevorzugt 0,2 bis 2,0 %, an polymerisierbaren Verbindungen.

Insbesondere bevorzugt sind die polymerisierbaren Verbindungen der Formel M.

Gegenstand der vorliegenden Erfindung ist somit auch die Verwendung der erfindungsgemäßen Mischungen in elektrooptischen Anzeigen sowie der Einsatz der erfindungsgemäßen Mischungen in Shutter-Brillen, insbesondere für 3D-Anwendungen, und in TN-, PS-TN-, STN-, TN-TFT-, OCB-, IPS-, PS-IPS-, FFS-, PS-FFS- und PS-VA-IPS-Anzeigen.

In der vorliegenden Anmeldung und in den folgenden Beispielen sind die Strukturen der Flüssigkristallverbindungen durch Acronyme angegeben, wobei die Transformation in chemische Formeln gemäß Tabelle A erfolgt. Alle Reste CₙH₂ₙ₊₁ und CₘH₂ₘ₊₁ sind geradkettige Alkylreste mit n bzw. m C-Atomen; n, m und k sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Acronym für den Grundkörper angegeben. Im Einzelfall folgt getrennt von Acronym für den Grundkörper mit einem Strick ein Code für die Substituenten R^{1*}, R^{2*}, L^{1*} und L^{2*}:

| Code für R^{1*}, R^{2*}, L^{1*}, L^{2*}, L^{3*} | R^{1*} | R^{2*} | L^{1*} | L^{2*} |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | F | H |
| nN.F.F | CₙH₂ₙ₊₁ | CN | F | F |
| nF | CₙH₂ₙ₊₁ | F | H | H |
| nCl | CₙH₂ₙ₊₁ | Cl | H | H |
| nOF | OCₙH₂ₙ₊₁ | F | H | H |
| nF.F | CₙH₂ₙ₊₁ | F | F | H |
| nF.F.F | CₙH₂ₙ₊₁ | F | F | F |
| nOCF₃ | CₙH₂ₙ₊₁ | OCF₃ | H | H |
| nOCF₃.F | CₙH₂ₙ₊₁ | OCF₃ | F | H |
| n-Vm | CₙH₂ₙ₊₁ | -CH=CH-CₘH₂ₘ₊₁ | H | H |
| nV-Vm | CₙH₂ₙ₊₁-CH=CH- | -CH=CH-CₘH₂ₘ₊₁ | H | H |

Bevorzugte Mischungskomponenten finden sich in den Tabellen A und B.

**Tabelle A**

| | |
|---|---|
| 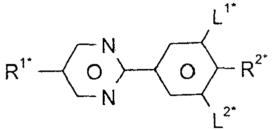 | 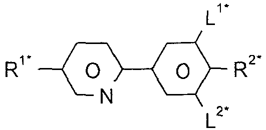 |
| **PYP** | **PYRP** |
| 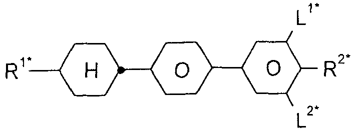 | 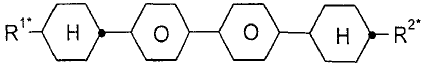 |
| **BCH** | **CBC** |
| 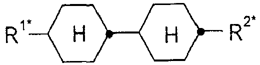 | 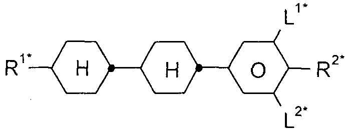 |
| **CCH** | **CCP** |
| 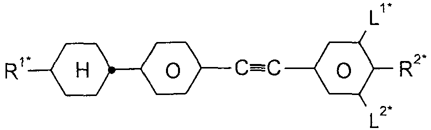 | 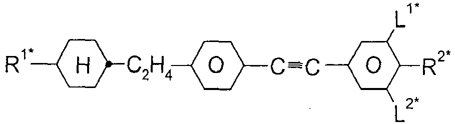 |
| **CPTP** | **CEPTP** |
| 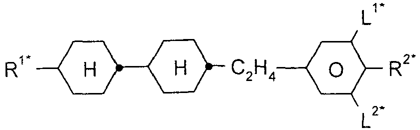 | 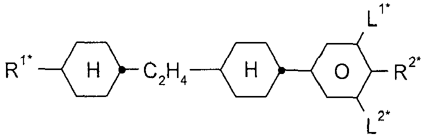 |
| **ECCP** | **CECP** |
| 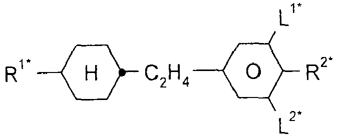 | 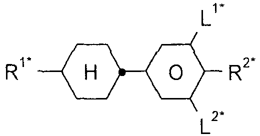 |
| **EPCH** | **PCH** |
| 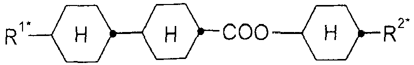 | |
| **CH** | |
| 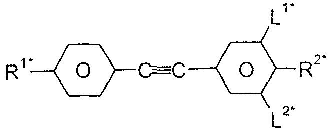 | 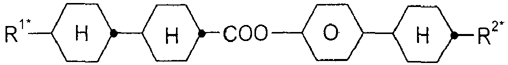 |
| **PTP** | **CCPC** |
| 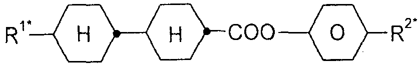 | 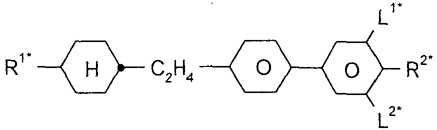 |
| **CP** | **BECH** |
| 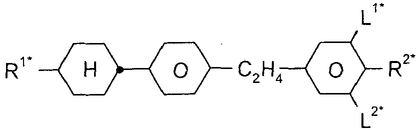 | 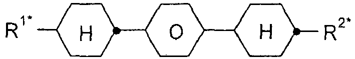 |
| **EBCH** | **CPC** |
| 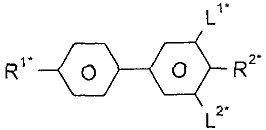 | 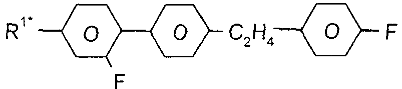 |
| **B** | **FET-nF** |
| 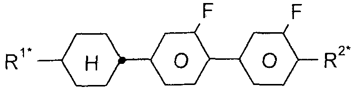 | 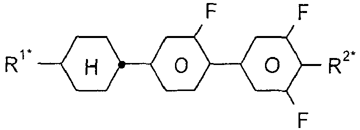 |
| **CGG** | **CGU** |
| 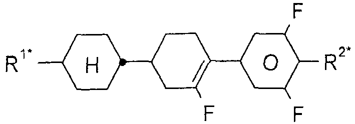 | |
| **CFU** | |

**Tabelle B**

| In den nachfolgenden Formeln bedeuten n und m jeweils unabhängig voneinander 0, 1, 2, 3, 4, 5, 6, 7, 8, 9, 10, 11 oder 12, insbesondere 2, 3, 5, ferner 0, 4, 6. k bedeutet 0, 1, 2, 3, 4, 5 oder 6. | | |
|---|---|---|
| 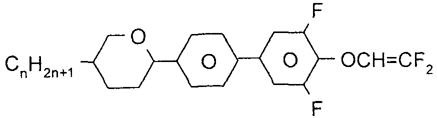 | 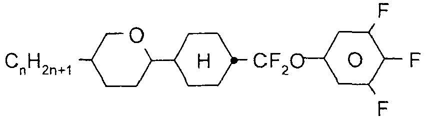 | |
| **APU-n-OXF** | **ACQU-n-F** | |
| 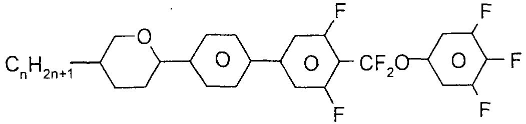 | | |
| **APUQU-n-F** | | |
| 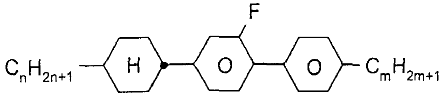 | 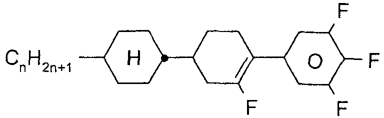 | |
| **BCH-n.Fm** | **CFU-n-F** | |
| 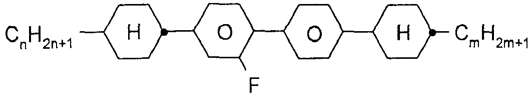 | | |
| **CBC-nmF** | | |
| 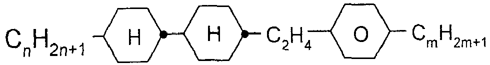 | 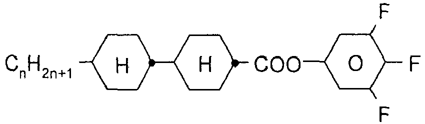 | |
| **ECCP-nm** | **CCZU-n-F** | |
| 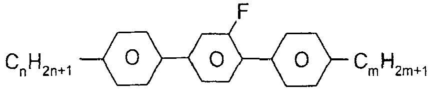 | 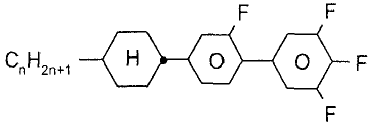 | |
| **PGP-n-m** | **CGU-n-F** | |
| 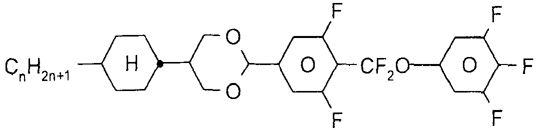 | | |
| **CDUQU-n-F** | | |
| 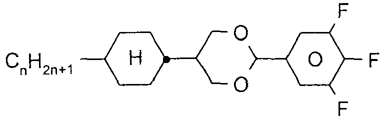 | 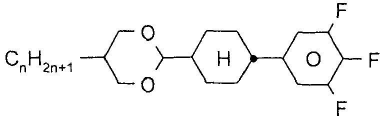 | |
| **CDU-n-F** | **DCU-n-F** | |
| 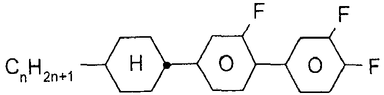 | 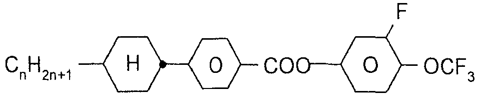 | |
| **CGG-n-F** | **CPZG-n-OT** | |
| 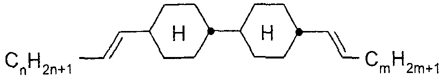 | 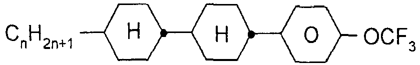 | |
| **CC-nV-Vm** | **CCP-nOCF₃** | |
| 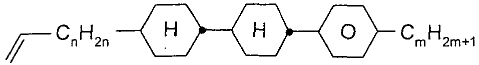 | 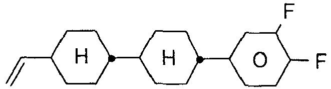 | |
| **CCP-Vn-m** | **CCG-V-F** | |
| 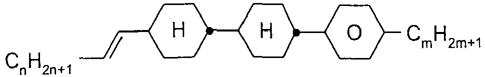 | 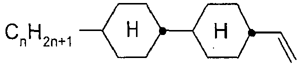 | |
| **CCP-nV-m** | **CC-n-V** | |
| 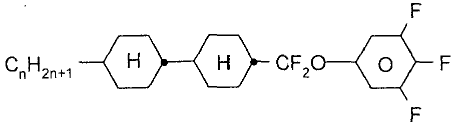 | 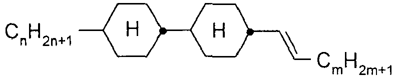 | |
| **CCQU-n-F** | **CC-n-Vm** | |
| 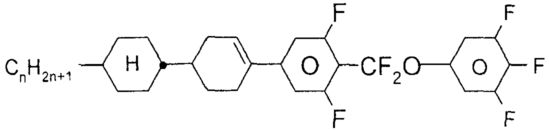 | | |
| **CLUQU-n-F** | | |
| 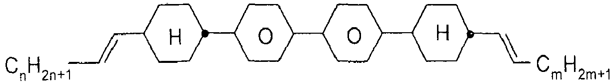 | | |
| **CPPC-nV-Vm** | | |
| 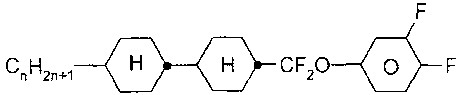 | 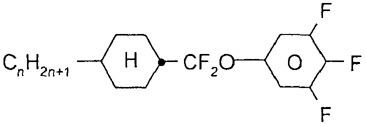 | |
| **CCQG-n-F** | **CQU-n-F** | |
| 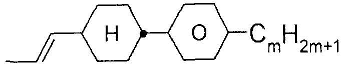 | | |
| **CP-1V-m** | | |
| 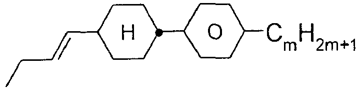 | 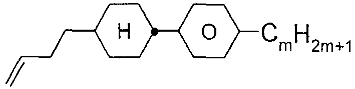 | |
| **CP-2V-m** | **CP-V2-m** | |
| 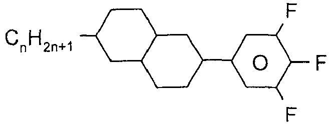 | 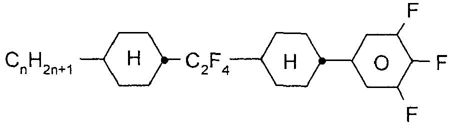 | |
| **Dec-U-n-F** | **CWCU-n-F** | |
| 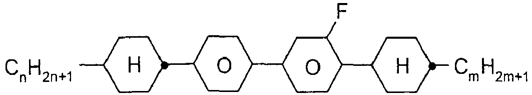 | | |
| **CPGP-n-m** | | |
| 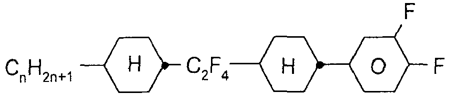 | | |
| **CWCG-n-F** | | |
| 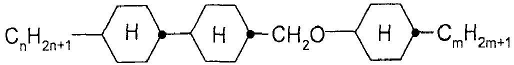 | | |
| **CCOC-n-m** | | |
| 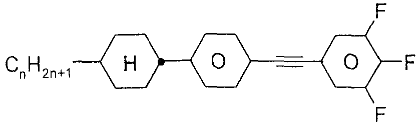 | 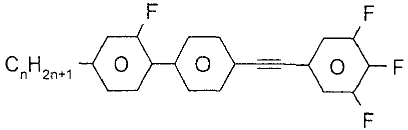 | |
| **CPTU-n-F** | **GPTU-n-F** | |
| 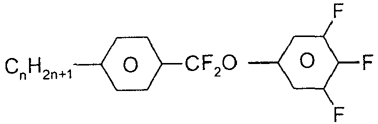 | | 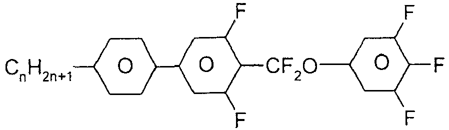 |
| **PQU-n-F** | | **PUQU-n-F** |
| 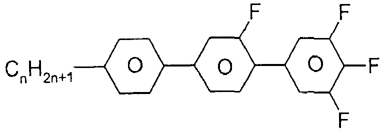 | | 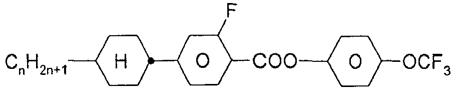 |
| **PGU-n-F** | | **CGZP-n-OT** |
| 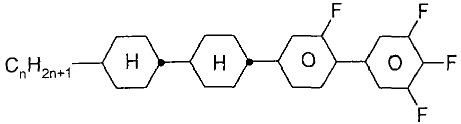 | | 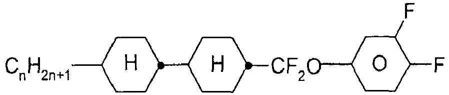 |
| **CCGU-n-F** | | **CCQG-n-F** |
| 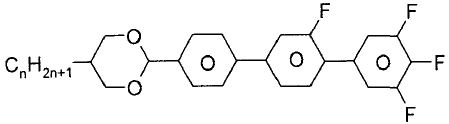 | | 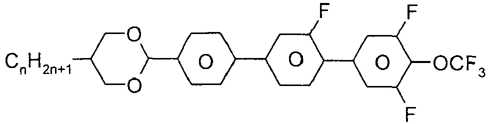 |
| **DPGU-n-F** | | **DPGU-n-OT** |
| 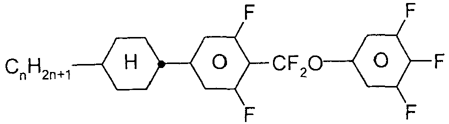 | | |
| **CUQU-n-F** | | |
| 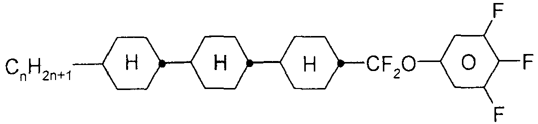 | | |
| **CCCQU-n-F** | | |
| 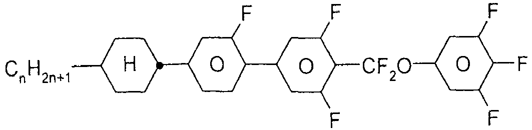 | | |
| **CGUQU-n-F** | | |
| 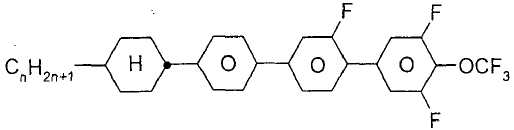 | | |
| **CPGU-n-OT** | | |
| 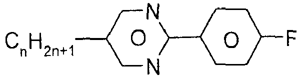 | | |
| **PYP-nF** | | |
| 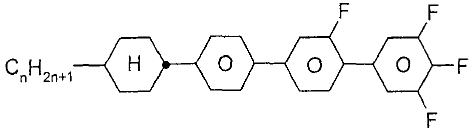 | | |
| **CPGU-n-F** | | |
| 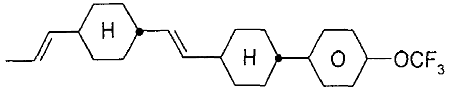 | | 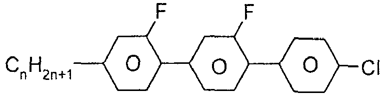 |
| **CVCP-1V-OT** | | **GGP-n-Cl** |
| 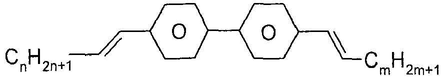 | | 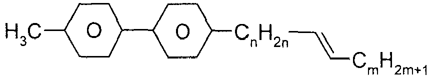 |
| **PP-nV-Vm** | | **PP-1-nVm** |
| 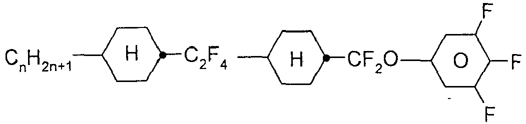 | | |
| **CWCQU-n-F** | | |
| 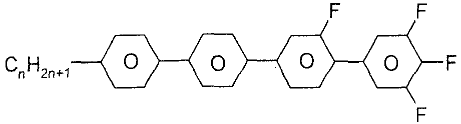 | | |
| **PPGU-n-F** | | |
| 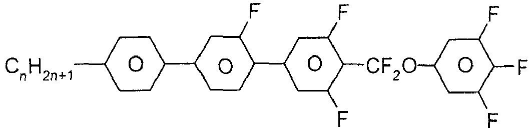 | | |
| **PGUQU-n-F** | | |
| 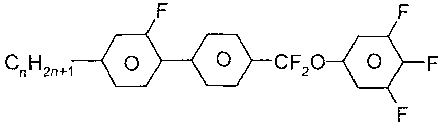 | | 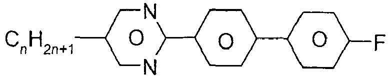 |
| **GPQU-n-F** | | **MPP-n-F** |
| 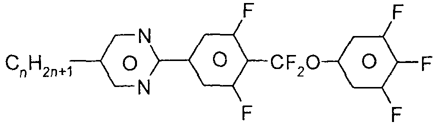 | | 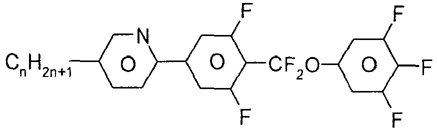 |
| **MUQU-n-F** | | **NUQU-n-F** |
| 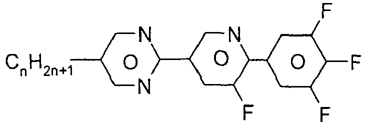 | | 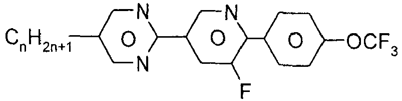 |
| **MN(F)U-n-F** | | **MN(F)P-n-OT** |
| 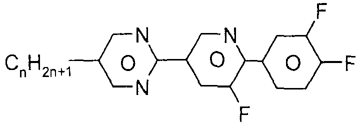 | | 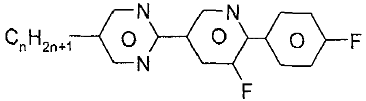 |
| **MN(F)G-n-F** | | **MN(F)P-n-F** |
| 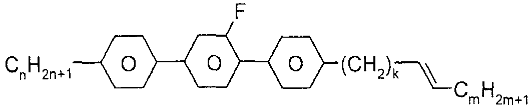 | | |
| **PGP-n-kVm** | | |
| 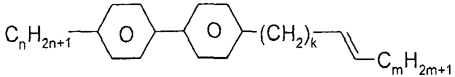 | | |
| **PP-n-kVm** | | |
| 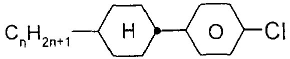 | | 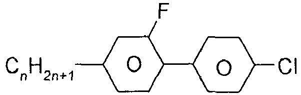 |
| **PCH-nCl** | | **GP-n-Cl** |
| 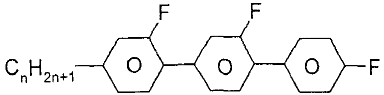 | | 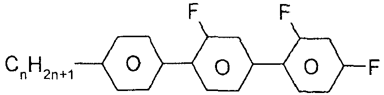 |
| **GGP-n-F** | | **PGIGI-n-F** |
| 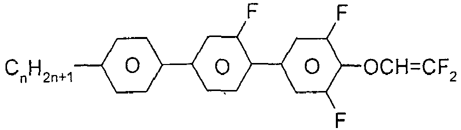 | | 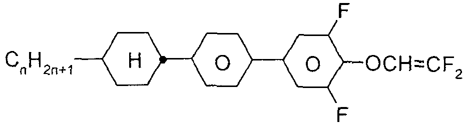 |
| **PGU-n-OXF** | | **CPU-n-OXF** |

Besonders bevorzugt sind flüssigkristalline Mischungen, die neben der Verbindung der Formel I mindestens ein, zwei, drei, vier oder mehr Verbindungen aus der Tabelle B enthalten.

**Tabelle C**

| In der Tabelle C werden mögliche Dotierstoffe angegeben, die in der Regel den erfindungsgemäßen Mischungen zugesetzt werden. Vorzugsweise enthalten die Mischungen 0-10 Gew.%, insbesondere 0,01-5 Gew.% und besonders bevorzugt 0,01-3 Gew.% an Dotierstoffen. | | |
|---|---|---|
| 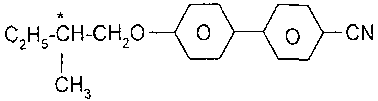 | | 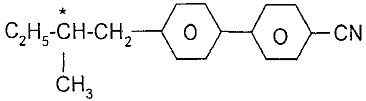 |
| **C 15** | | **CB 15** |
| 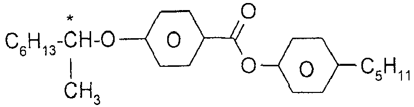 | | 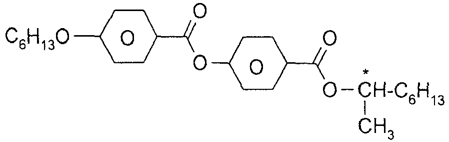 |
| **CM 21** | | **R/S-811** |
| 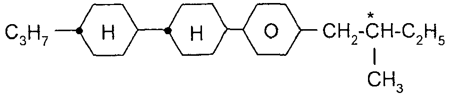 | | 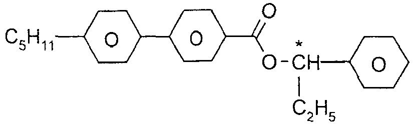 |
| **CM 44** | | **CM 45** |
| 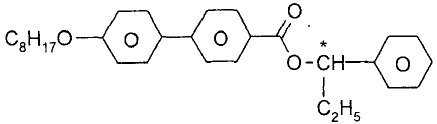 | | 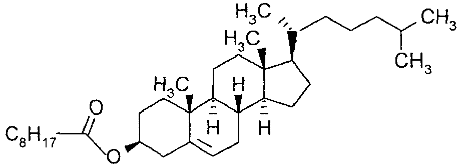 |
| **CM 47** | | **CN** |
| 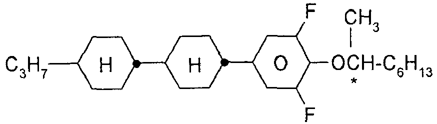 | | 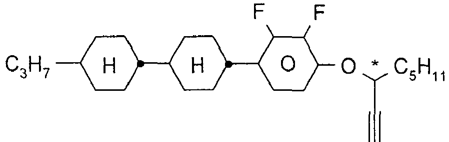 |
| **R/S-2011** | | **R/S-3011** |
| 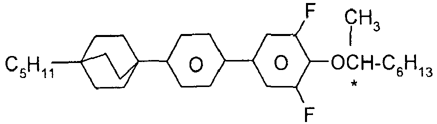 | | 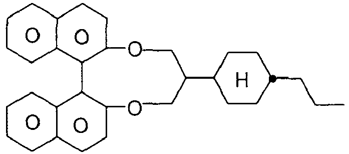 |
| **R/S-4011** | | **R/S-5011** |
| 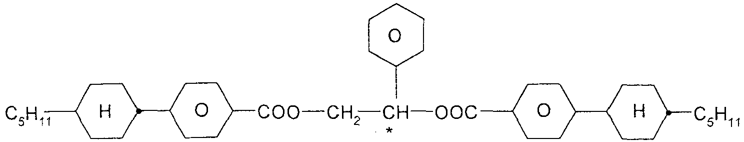 | | |
| **R/S-1011** | | |

**Tabelle D**

| Stabilisatoren, die beispielsweise den erfindungsgemäßen Mischungen in Mengen von 0-10 Gew.% zugesetzt werden können, werden nachfolgend genannt. (n = 1-12) | | |
|---|---|---|
| 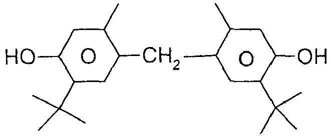 | | 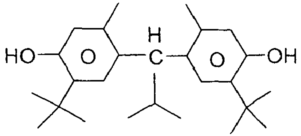 |
| 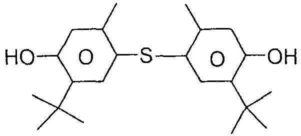 | | 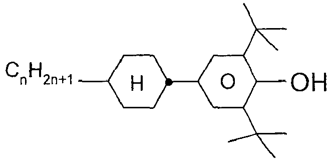 |
| 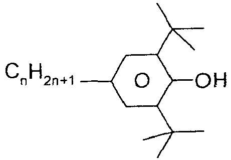 | | 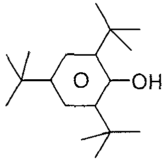 |
| n = 1, 2, 3, 4, 5, 6 oder 7 | | |
| 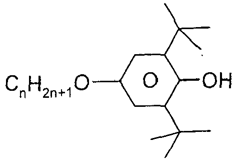 | | 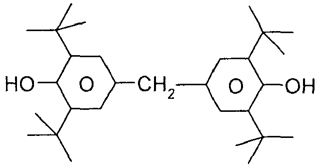 |
| 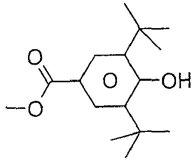 | | 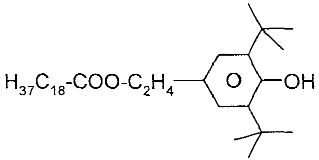 |
| 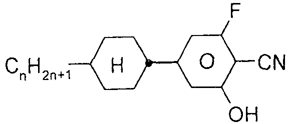 | | 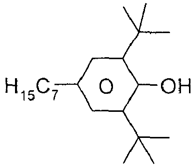 |
| 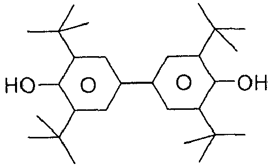 | 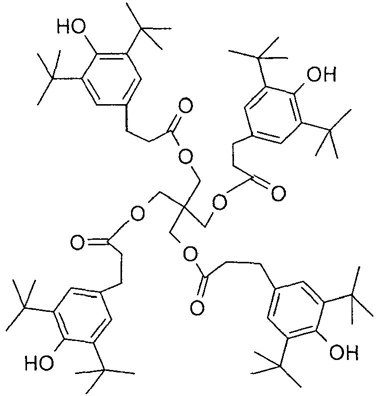 | |
| 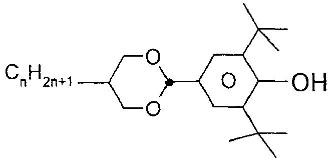 | | |
| n = 1, 2, 3, 4, 5, 6 oder 7 | | |
| 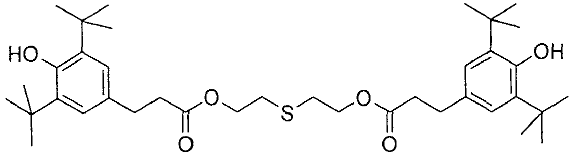 | | |
| 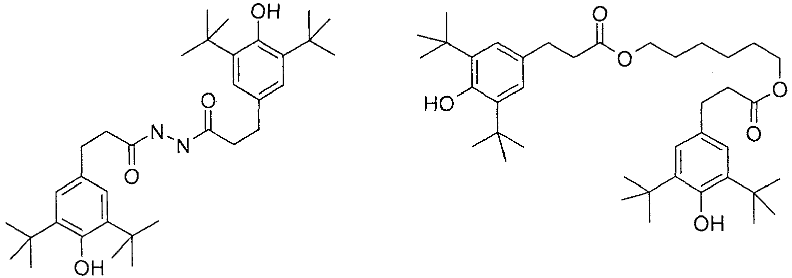 | | |
| 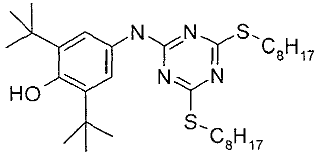 | | 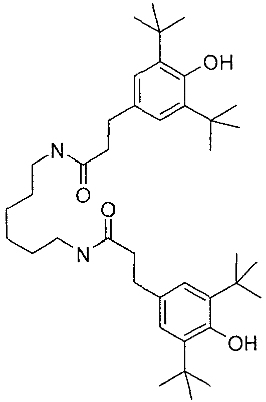 |
| 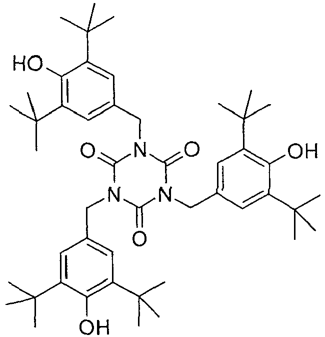 | | 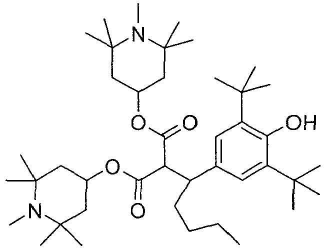 |
| 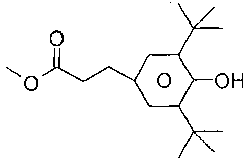 | | 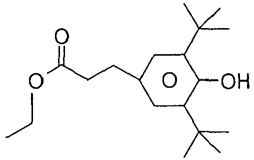 |
| 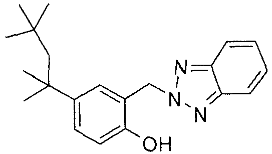 | | 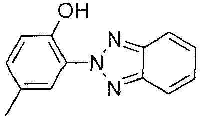 |
| 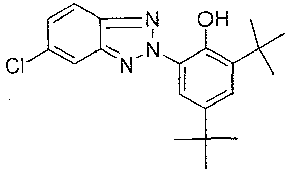 | 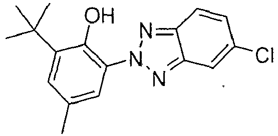 | |
| 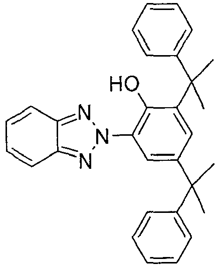 | 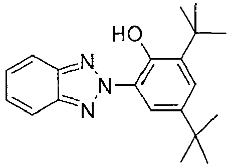 | |
| 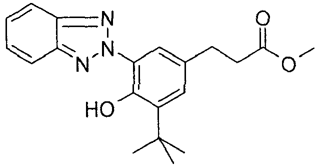 | 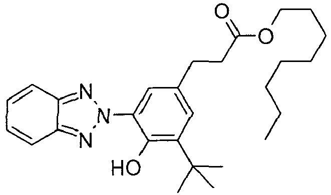 | |
| 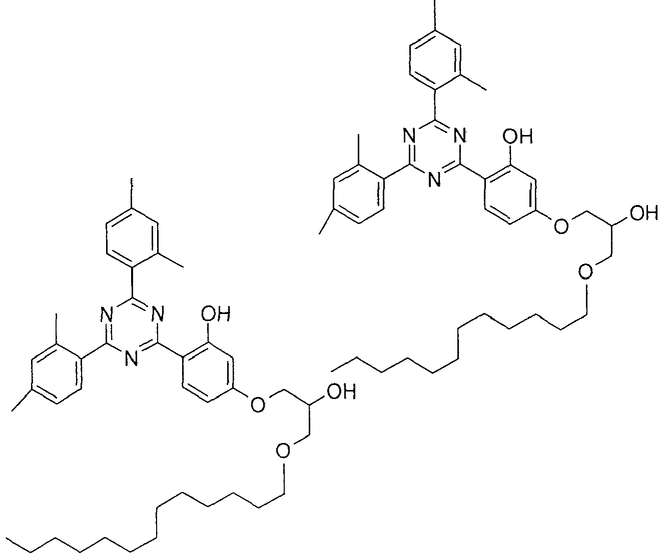 | | 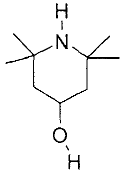 |
| 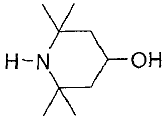 | 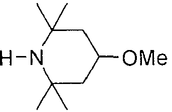 | 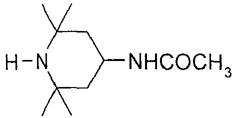 |
| 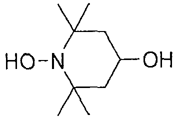 | 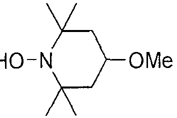 | 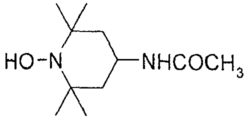 |
| 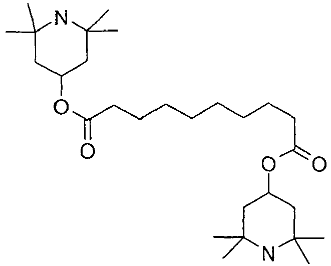 | 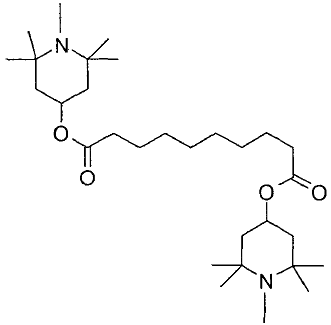 | |
| 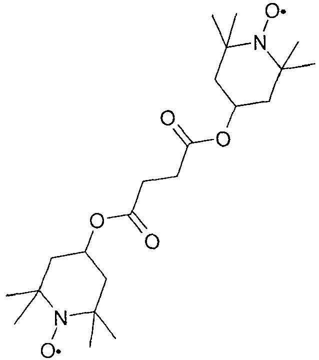 | 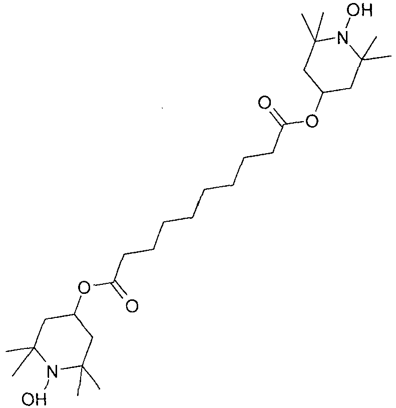 | |

Geeignete polymerisierbare Verbindungen (reaktive Mesogene) für den Einsatz in den erfindungsgemäßen Mischungen, vorzugsweise in PSA- und PS-VA-Anwendungen oder PS-IPS/FFS-Anwendungen, werden nachfolgend in Tabelle E genannt:

**Tabelle E**

| In der Tabelle E sind Beispielverbindungen zusammengestellt, die in den erfindungsgemäßen Mischungen vorzugsweise als polymerisierbare Verbindungen (reaktive mesogene Verbindungen) zur Herstellung beispielsweise von PSV, PS-VA-, PS-IPS- oder PS-FFS-Mischungen verwendet werden können. | |
|---|---|
| 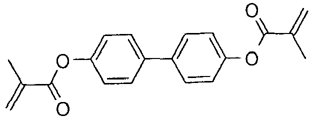 | RM-1 |
| 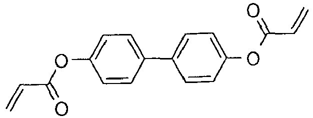 | RM-2 |
| 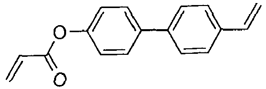 | RM-3 |
| 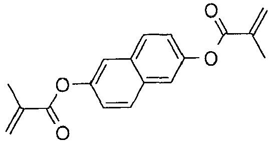 | RM-4 |
| 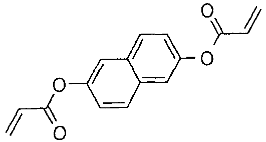 | RM-5 |
| 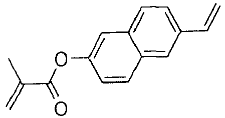 | RM-6 |
| 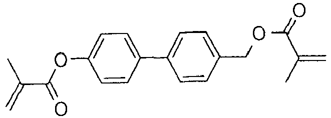 | RM-7 |
| 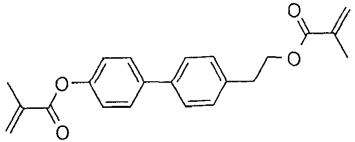 | RM-8 |
| 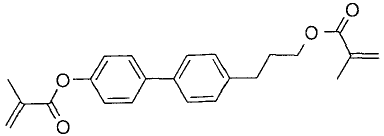 | RM-9 |
| 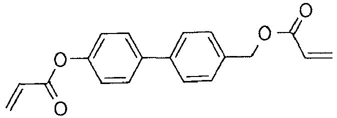 | RM-10 |
| 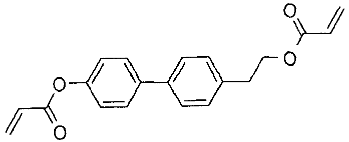 | RM-11 |
| 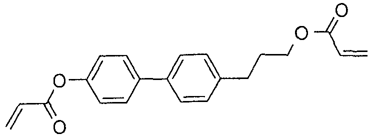 | RM-12 |
| 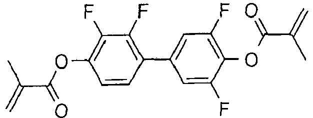 | RM-13 |
| 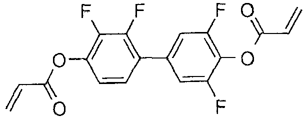 | RM-14 |
| 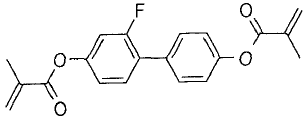 | RM-15 |
| 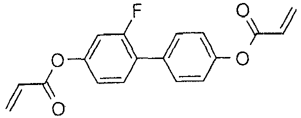 | RM-16 |
| 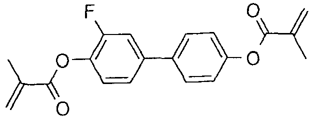 | RM-17 |
| 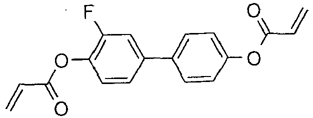 | RM-18 |
| 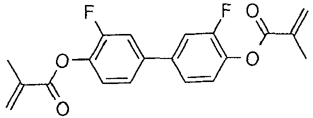 | RM-19 |
| 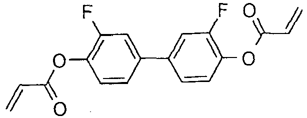 | RM-20 |
| 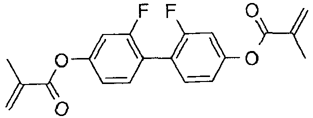 | RM-21 |
| 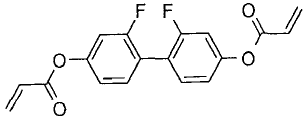 | RM-22 |
| 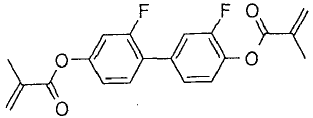 | RM-23 |
| 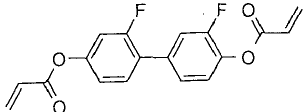 | RM-24 |
| 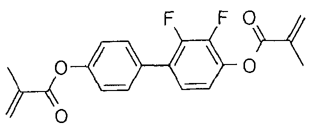 | RM-25 |
| 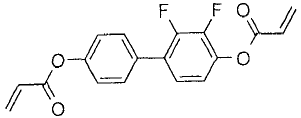 | RM-26 |
| 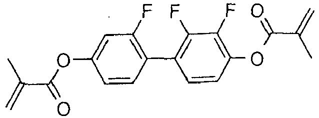 | RM-27 |
| 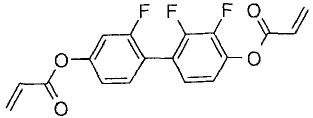 | RM-28 |
| 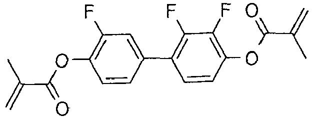 | RM-29 |
| 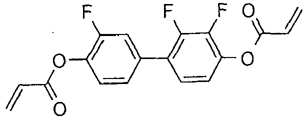 | RM-30 |
| 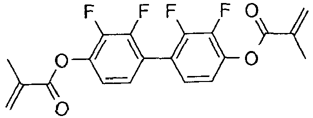 | RM-31 |
| 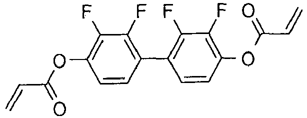 | RM-32 |
| 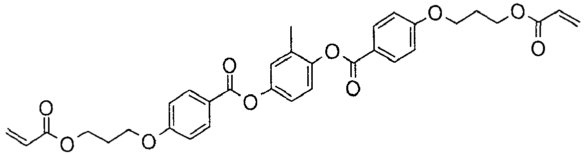 | RM-33 |
| 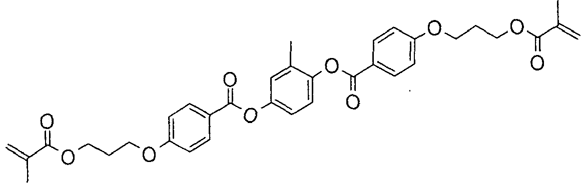 | RM-34 |
| 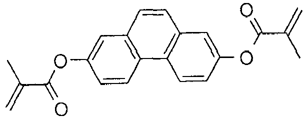 | RM-35 |
| 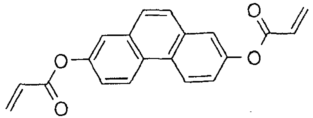 | RM-36 |
| 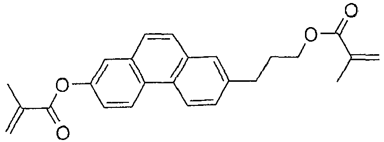 | RM-37 |
| 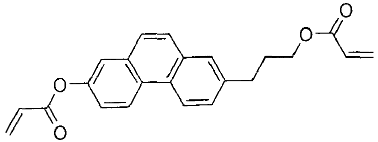 | RM-38 |
| 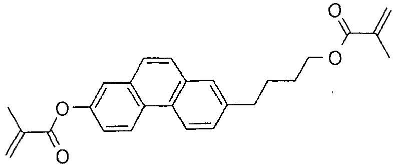 | RM-39 |
| 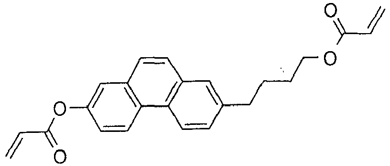 | RM-40 |
| 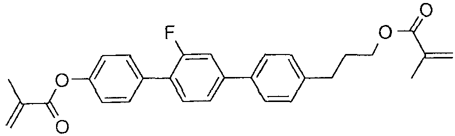 | RM-41 |
| 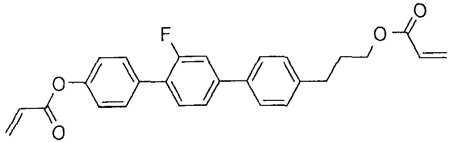 | RM-42 |
| 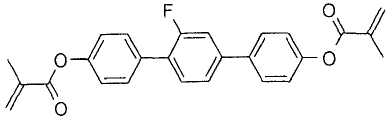 | RM-43 |
| 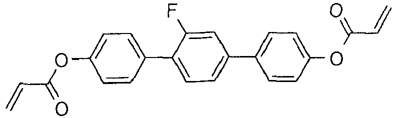 | RM-44 |
| 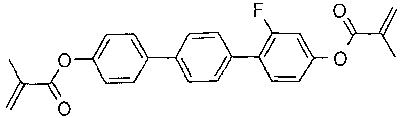 | RM-45 |
| 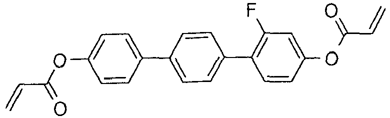 | RM-46 |
| 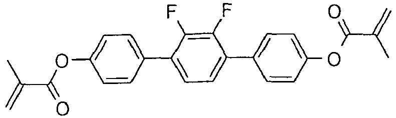 | RM-47 |
| 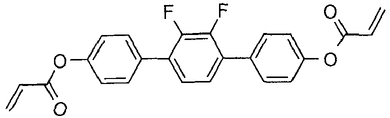 | RM-48 |
| 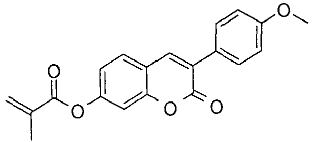 | RM-49 |
| 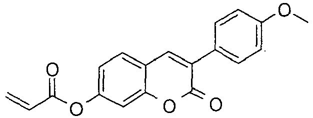 | RM-50 |
| 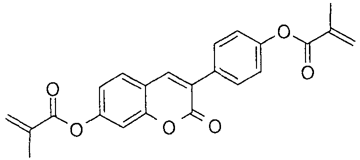 | RM-51 |
| 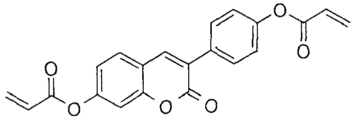 | RM-52 |
| 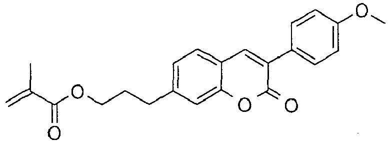 | RM-53 |
| 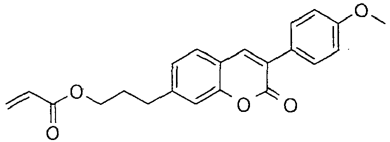 | RM-54 |
| 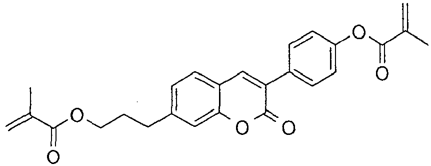 | RM-55 |
| 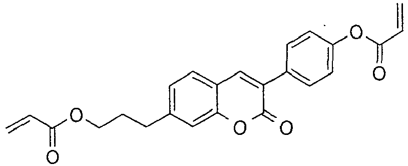 | RM-56 |
| 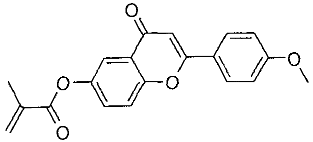 | RM-57 |
| 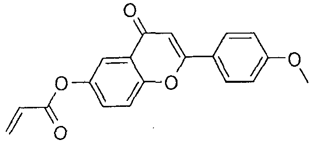 | RM-58 |
| 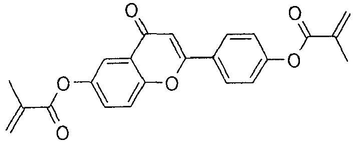 | RM-59 |
| 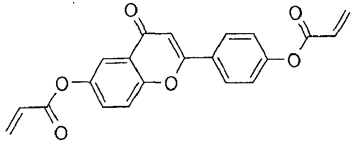 | RM-60 |
| 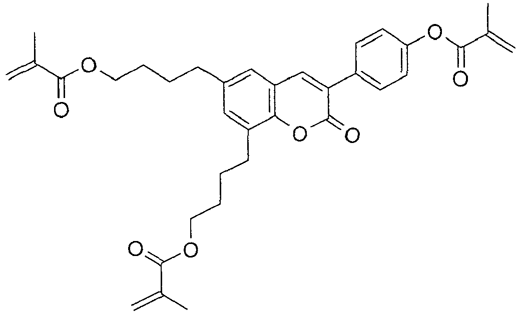 | RM-61 |
| 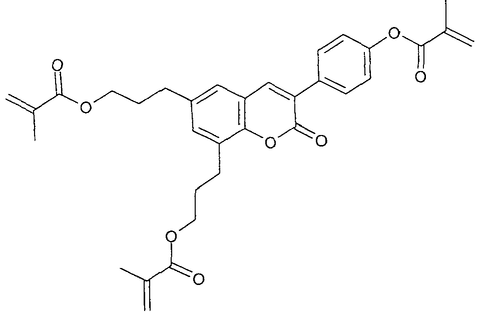 | RM-62 |
| 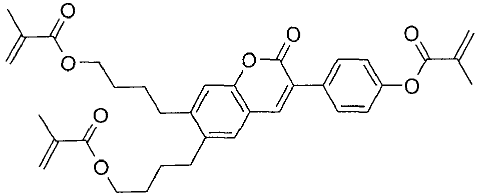 | RM-63 |
| 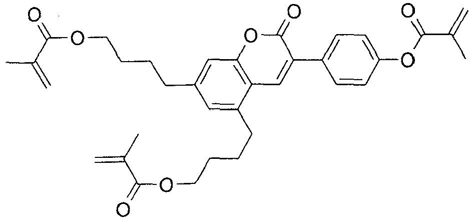 | RM-64 |
| 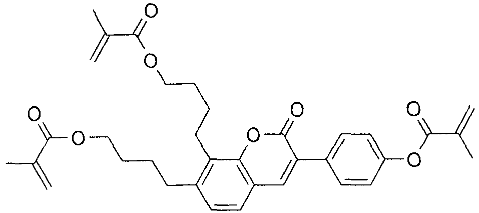 | RM-65 |
| 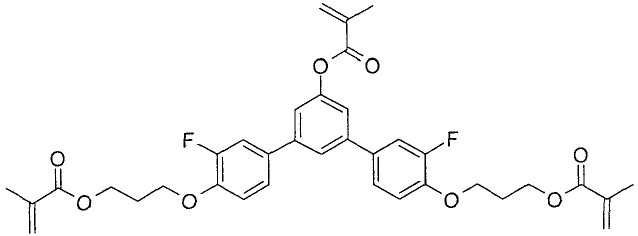 | RM-66 |
| 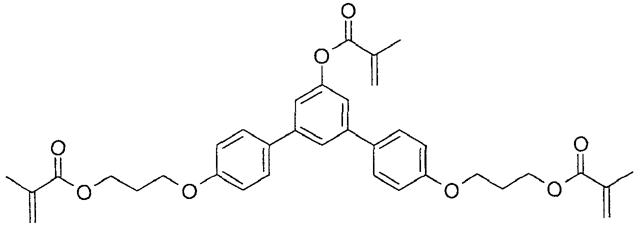 | RM-67 |
| 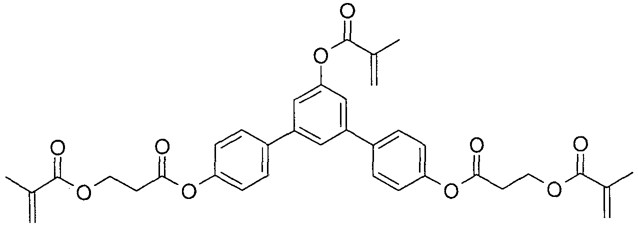 | RM-68 |
| 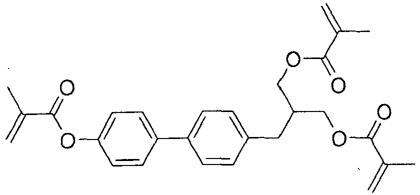 | RM-69 |
| 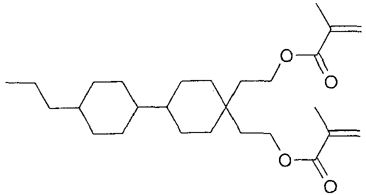 | RM-70 |
| 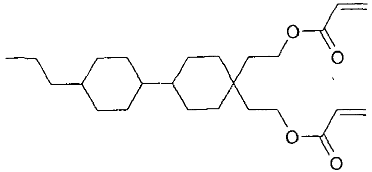 | RM-71 |
| 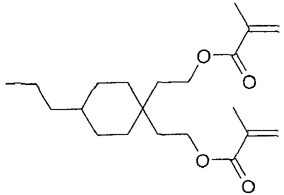 | RM-72 |
| 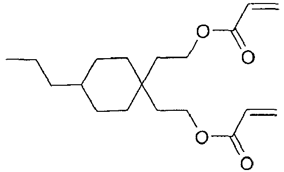 | RM-73 |
| 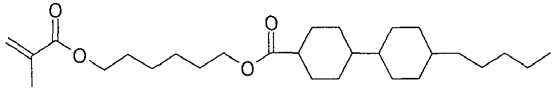 | RM-74 |
| 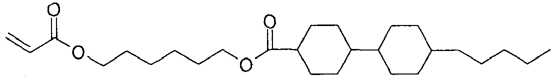 | RM-75 |
| 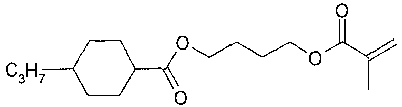 | RM-76 |
| 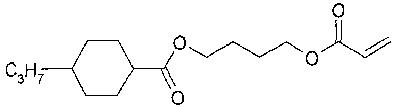 | RM-77 |
| 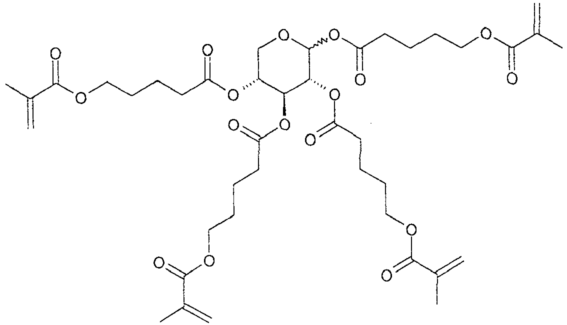 | RM-78 |
| 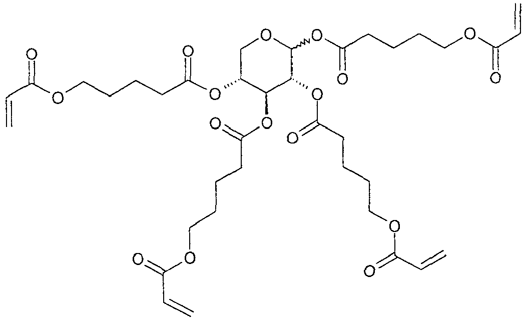 | RM-79 |
| 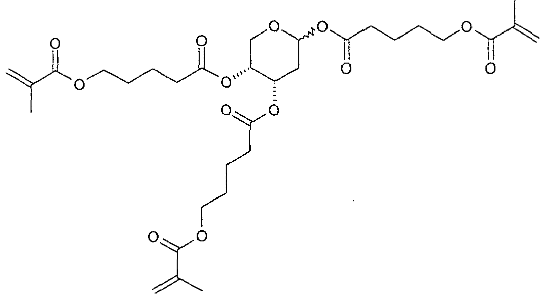 | RM-80 |
| 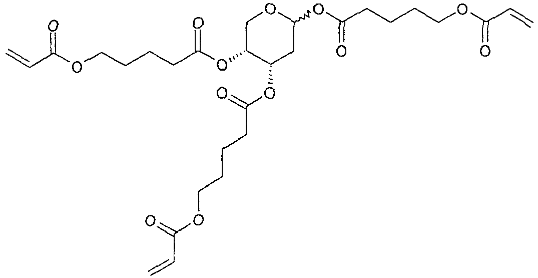 | RM-81 |
| 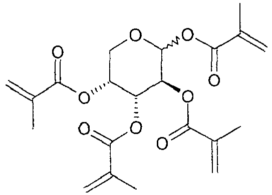 | RM-82 |
| 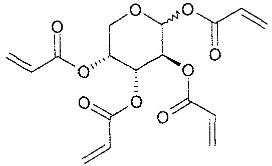 | RM-83 |

Sofern die erfindungsgemäßen Mischungen eine oder mehrere mesogene Verbindungen enthalten, ist die mesogene Verbindung in einer bevorzugten Ausführungsform eine Verbindung ausgewählt aus der Tabelle E.

Die folgenden Beispiele sollen die Erfindung erläutern, ohne sie zu begrenzen.

### Beispiele

Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Weiterhin bedeuten
- An die optische Anisotropie bei 589 nm und 20 °C,
- γ₁ die Rotationsviskosität (mPa·s) bei 20 °C,
- Δε die dielektrische Anisotropie bei 20 °C und 1 kHz (Δε = ε_{∥} - ε_{⊥}, wobei ε_{∥} die Dielektrizitätskonstante parallel zu den Moleküllängsachsen und ε_{⊥} die Dielektrizitätskonstante senkrecht dazu bedeutet),
- V₁₀ die Spannung (V) für 10 % Transmission (Blickrichtung senkrecht zur Plattenoberfläche), (Schwellenspannung), bestimmt in einer TN Zelle (90 Grad Verdrillung) im 1. Minimum (d.h. bei einem dΔn-Wert von 0,5 µm) bei 20 °C,
- V₀ die kapazitiv bestimmte Freedericksschwellenspannung in einer antiparallel geriebenen Zelle bei 20 °C.

Alle physikalischen Eigenschaften werden nach "Merck Liquid Crystals, Physical Properties of Liquid Crystals" Status Nov. 1997, Merck KGaA, Deutschland bestimmt und gelten für eine Temperatur von 20 °C, sofern nicht explizit anders angegeben.

### Beispiel 1

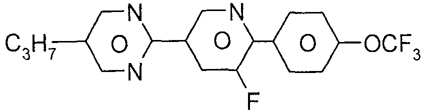

### Stufe 1

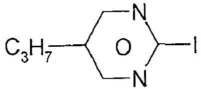

160 mmol 2-Chlor-5-propyl-pyrimidin werden bei 0 °C mit 150 ml 57 %iger Jodwasserstoffsäure versetzt und 1 Stunde bei 0 °C gerührt. Danach wird bei 0 °C mit Sodalösung neutralisiert und zur Entfärbung Natriumsulfitlösung zugesetzt. Der Ansatz wird mit Methyl-tert. Butylether extrahiert und die organische Phase mit Wasser gewaschen, mit Natriumsulfat getrocknet und eingedampft. Der Rückstand wird mit Dichlormethan über Kieselgel chromatographiert.

### Stufe 2

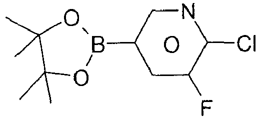

190 mmol 5-Brom-2-chlor-3-fluorpyridin und 190mmol Bispinacolatodiboron werden in 500ml Dioxan gelöst und mit 580 mmol Kaliumacetat und 6 mmol Pd(DPPF)Cl₂ versetzt. Man erhitzt 17 Stunden unter Rühren bei 100 °C. Die Reaktionslösung wird mit Wasser und Methyl-tert. Butylether versetzt und die organische Phase mit gesättigter NaCl-Lösung gewaschen, mit Natriumsulfat getrocknet und eingeengt. Der Rückstand wird mit Dichlormethan/MTB-Ether 95/5 über Kieselgel chromatographisch gereinigt, und man erhält das 2-Chloro-3-fluoro-5-(4,4,5,5-tetraethyl-[1,3,2]dioxaborolan-2-yl)-pyridin.

### Stufe 3

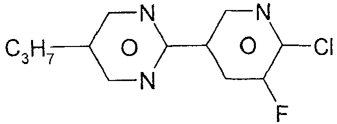

65 mmol 2-Jod-5-propyl-pyrimidin und 65 mmol 2-Chloro-3-fluoro-5-(4,4,5,5-tetraethyl-[1,3,2]dioxaborolan-2-yl)-pyridin werden in 155 ml Dioxan gelöst und mit 130 mmol tri-Kaliumphosphat und 2 mmol Tetrakis(triphenylphospin)palladium-Katalysator versetzt. Man erhitzt unter Rühren 17 Stunden auf 100 °C. Das Reaktionslösung wird mit Wasser und Methyl-tert. Butylether versetzt und die organische Phase mit Wasser gewaschen, mit Natriumsulfat getrocknet und eingeengt. Der Rückstand wird mit Dichlormethan/MTB-Ether 90/10 über Kieselgel chromatographisch gereinigt, und man erhält das 2-(6-Chloro-5-fluoro-pyridin-3-yl)-5-propyl-pyrimidin.

### Stufe 4

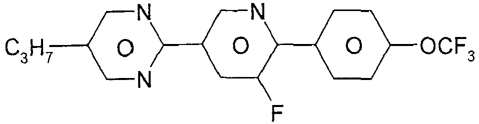

10,2 mmol 2-(6-Chloro-5-fluoro-pyridin-3-yl)-5-propyl-pyrimidin und 10,2mmol 4,4,5,5-Tetramethyl-2-(4-trifluoromethoxy-phenyl)-[1,3,2]dioxaborolane werden werden in 30ml Dioxan gelöst und mit 20,4mmol Cäsiumfluorid und 0,5mmol Bis(tricyclohexylphospin)-palladiumdichlorid Katalysator versetzt. Man erhitzt unter Rühren 18 Stunden auf 100°C. Der Ansatz wird mit Wasser und MTB-Ether versetzt und die organische Phase mit Wasser gewaschen, mit Natriumsulfat getrocknet und eingeengt. Der Rückstand wird mit Heptan/MTB-Ether 2/1 über Kieselgel chromatographisch gereinigt und aus Isopropanol umkristallisiert, und man erhält 2-[5-Fluoro-6-(4-trifluoromethoxy-phenyl)-pyridin-3-yl]-5-propyl-pyrimidin.
K 108 S_{A} 173 I; Δn = 0,238; Δε = 30,5; γ₁ = 83

Analog werden die folgenden Verbindungen der Formel 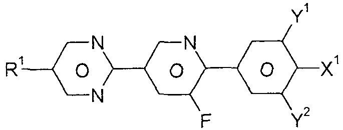 hergestellt:

| R¹ | X¹ | Y¹ | Y² |
|---|---|---|---|
| CH₃ | OCF₃ | H | H |
| C₂H₅ | OCF₃ | H | H |
| n-C₄H₉ | OCF₃ | H | H |
| n-C₅H₁₁ | OCF₃ | H | H |
| CH₃ | OCF₃ | F | H |
| C₂H₅ | OCF₃ | F | H |
| n-C₃H₇ | OCF₃ | F | H |
| n-C₄H₉ | OCF₃ | F | H |
| n-C₅H₁₁ | OCF₃ | F | H |
| n-C₅H₁₁ | OCF₃ | F | H |
| CH₃ | OCF | F | F |
| C₂H₅ | OCF₃ | F | F |
| n-C₃H₇ | OCF₃ | F | F |
| n-C₄H₉ | OCF₃ | F | F |
| n-C₅H₁₁ | OCF₃ | F | F |
| CH₃ | F | H | H |
| C₂H₅ | F | H | H |
| n-C₃H₇ | F | H | H |
| n-C₄H₉ | F | H | H |
| n-C₃H₇ | F | H | H |
| n-C₃H₇ | F | F | H |
| n-C₅H₁₁ | F | F | F |
| n-C₃H₇ | CN | F | H |
| n-C₅H₁₁ | Cl | H | H |
| n-C₅H₁₁ | OCHF₂ | H | H |

### Mischungsbeispiele

**Beispiel M1**

| | | | |
|---|---|---|---|
| PUQU-3-F | 7,0% | Klärpunkt [°C]: | 69,0 |
| CC-3-V1 | 7,0 % | □n [589 nm, 20 °C]: | 0,1001 |
| CC-3-V | 48,0% | Δε [1 kHz, 20 °C]: | 3,0 |
| CCP-V-1 | 13,5% | ε_{∥} [1 kHz, 20°C]: | 5,6 |
| PP-1-2V1 | 6,5 % | ε_{⊥} [1 kHz, 20°C]: | 2,6 |
| PGP-2-3 | 4,0 % | K₃ [pN, 20°C]: | 13,0 |
| PGP-2-4 | 5,0 % | K₃/K₁ [20°C]: | 1,02 |
| CCP-30CF₃ | 4,0% | γ₁ [mPa·s, 20°C]: | 42 |
| 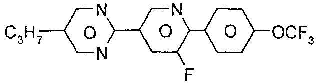 | 5,0 % | V₀ [V]: | 2,23 |

**Beispiel M2**

| | | | |
|---|---|---|---|
| PGP-2-3 | 2,0% | Klärpunkt [°C]: | 74,5 |
| PGP-2-4 | 5,0 % | □n [589 nm, 20 °C] | 0,1030 |
| CCP-V-1 | 9,0 % | Δε [1 kHz, 20 °C]: | 7,0 |
| CC-3-V | 48,0 % | ε_{∥} [1 kHz, 20°C]: | 10,1 |
| CC-3-V1 | 8,0% | ε_{⊥} [1 kHz, 20°C]: | 3,1 |
| PUQU-3-F | 6,0 % | K₃ [pN, 20°C]: | 13,3 |
| APUQU-2-F | 7,0 % | K₃/K₁ [20°C]: | 0,99 |
| APUQU-3-F | 7,0 % | γ₁ [mPa·s, 20°C]: | 50 |
| 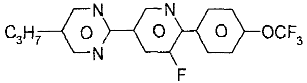 | 8,0 % | V₀ [V]: | 1,46 |

**Beispiel M3**

| | | | |
|---|---|---|---|
| CC-3-V | 41,0 % | Klärpunkt [°C]: | 75,0 |
| CC-3-V1 | 8,0 % | □n [589 nm, 20 °C]: | 0,1321 |
| CCP-V2-1 | 3,0 % | Δε [1 kHz, 20 °C]: | 4,4 |
| CPGU-3-OT | 6,0% | ε_{∥} [1 kHz, 20°C]: | 7,2 |
| PGP-2-2V | 18,0 % | ε_{⊥} [1 kHz, 20°C]: | 2,8 |
| PP-1-2V1 | 10,5 % | γ₁ [mPa·s, 20°C]: | 49 |
| PUQU-3-F | 6,0 % | | |
| 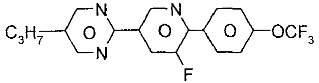 | 7,5 % | | |

## Patentansprüche

1. Flüssigkristallines Medium, **dadurch gekennzeichnet, dass** es eine oder mehrere Verbindungen der Formel I, 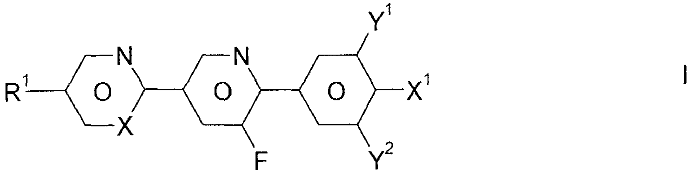 worin
R¹ einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 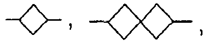 -O-, -CO-O- oder -O-CO-so ersetzt sein können, dass O-Atome nicht direkt miteinander verknüpft sind,
X¹ F, Cl, CN, SF₅, SCN, NCS, halogenierter Alkylrest, halogenierter Alkenylrest, halogenierter Alkoxyrest oder halogenierter Alkenyloxyrest jeweils mit bis zu 6 C-Atomen,
X C-H oder N, und
Y¹ und Y² jeweils unabhängig voneinander H oder F
bedeuten,
enthält.

2. Flüssigkristallines Medium nach Anspruch 1, **dadurch gekennzeichnet, dass** es eine oder mehrere Verbindungen ausgewählt aus den Verbindungen der Formeln I-1 bis I-15, 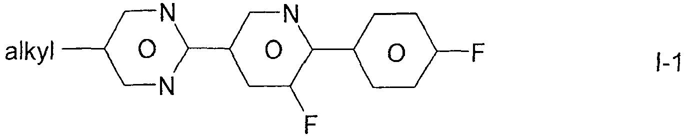 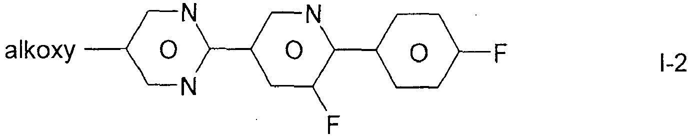 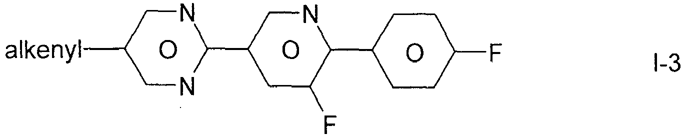 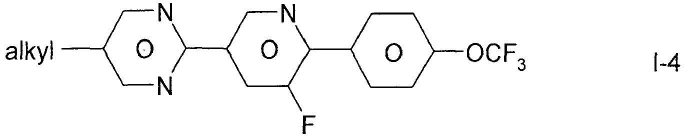 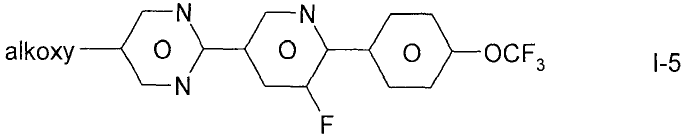 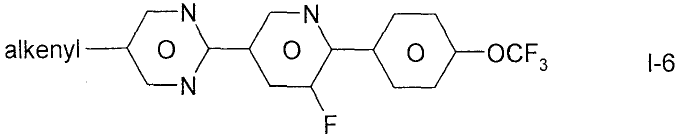 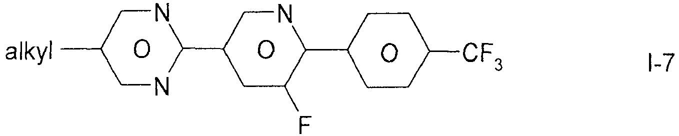 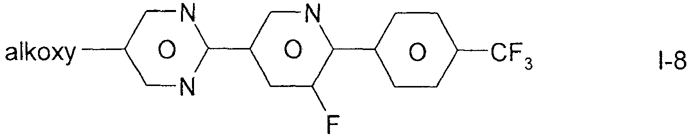 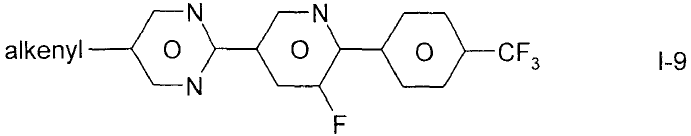 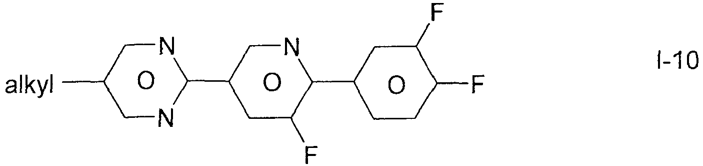 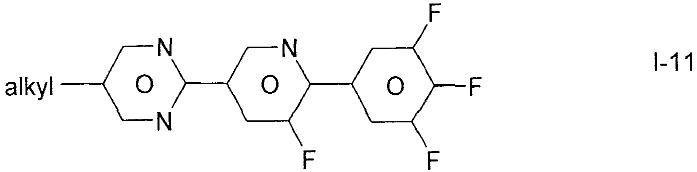 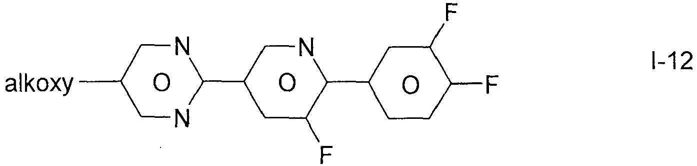 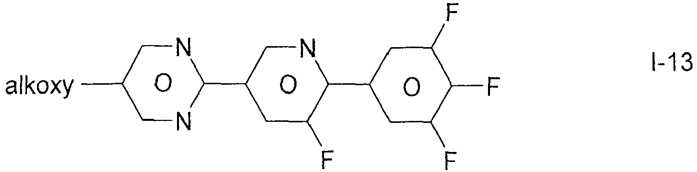 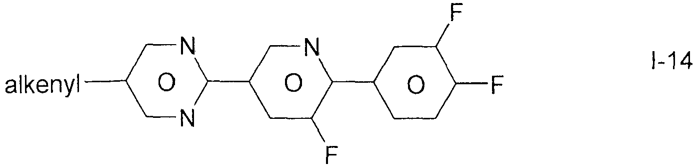 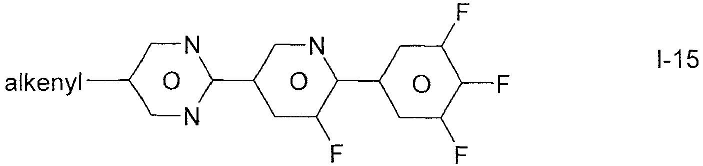 worin
alkyl ein geradkettiger Alkylrest mit 1-6 C-Atomen,
alkenyl ein geradkettiger Alkenylrest mit 2-6 C-Atomen,
alkoxy ein geradkettiger Alkoxylrest mit 1-6 C-Atomen,
bedeuten.
enthält.

3. Flüssigkristallines Medium nach Anspruch 1 oder 2, **dadurch gekennzeichnet, dass** es eine oder mehrere Verbindungen der Formel I-4 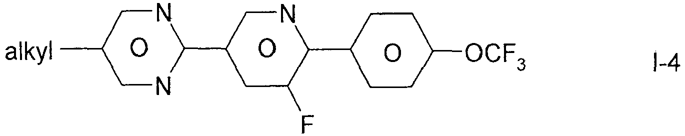 worin
alkyl ein geradkettigen Alkylrest mit 1 bis 6 C-Atomen
bedeutet,
enthält.

4. Flüssigkristallines Medium nach einem oder mehreren der Ansprüche 1 bis 3, **dadurch gekennzeichnet, dass** es zusätzlich eine oder mehrere Verbindungen ausgewählt aus den Formeln II und/oder III, 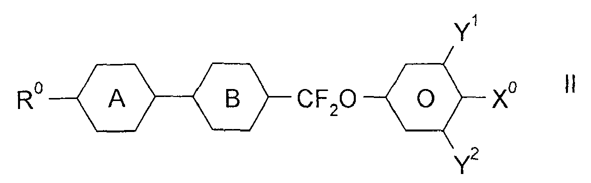 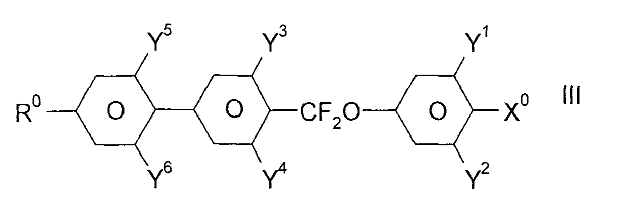 worin
R⁰ einen halogenierten oder unsubstituierten Alkyl- oder Alkoxyrest mit jeweils 1 bis 15 C-Atomen, wobei in diesen Resten auch eine oder mehrere CH₂-Gruppen jeweils unabhängig voneinander durch -C=C-, -CF₂O-, -CH=CH-, 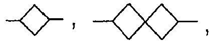 -O-, -CO-O- oder -O-CO- so ersetzt sein können, dass O-Atome nicht direkt miteinander verknüpft sind,
X⁰ F, Cl, CN, SF₅, SCN, NCS, halogenierter Alkylrest, halogenierter Alkenylrest, halogenierter Alkoxyrest oder halogenierter Alkenyloxyrest mit jeweils bis zu 6 C-Atomen, und
Y¹⁻⁶ jeweils unabhängig voneinander H oder F 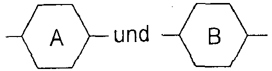 jeweils unabhängig voneinander 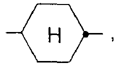 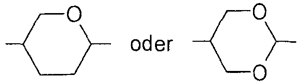
bedeuten,
enthält.

5. Flüssigkristallines Medium nach einem oder mehreren der Ansprüche 1 bis 4, **dadurch gekennzeichnet, dass** es zusätzlich eine oder mehrere Verbindungen ausgewählt aus den Formeln IV bis VIII, 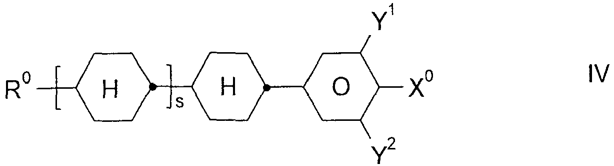 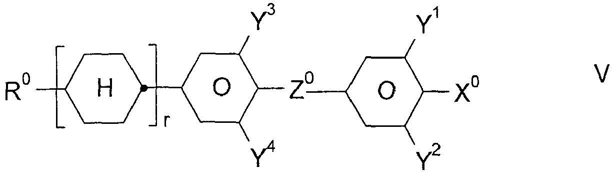 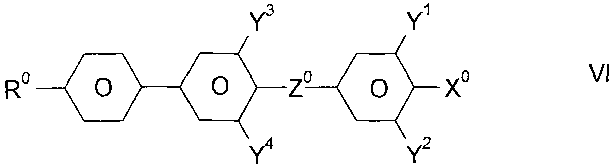 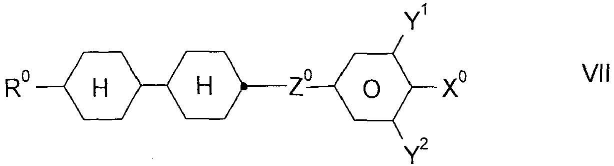 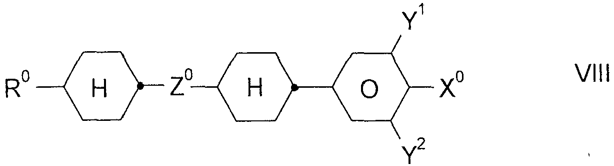 worin R⁰, X⁰ und Y¹⁻⁴ die in Anspruch 4 angegebenen Bedeutungen besitzen,
Z⁰ -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- oder -OCF₂-, in Formel V und VI auch eine Einfachbindung,
r 0 oder 1, und
s 0 oder 1
bedeuten,
enthält.

6. Flüssigkristallines Medium nach einem oder mehreren der Ansprüche 1 bis 5, **dadurch gekennzeichnet, dass** es zusätzlich eine oder mehrere Verbindungen ausgewählt aus den Formeln IX bis XII, 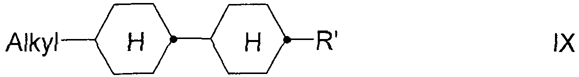 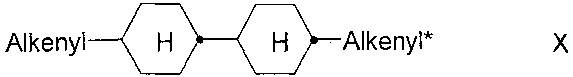 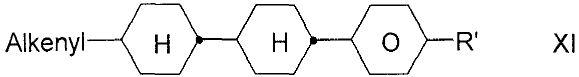 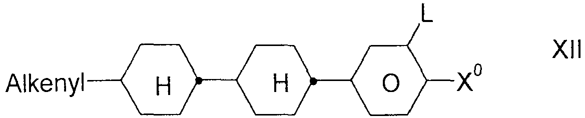 worin X⁰ die in Anspruch 4 angegebenen Bedeutungen besitzt,
L H oder F,
"Alkyl" C₁₋₆-Alkyl,
R' C₁₋₆-Alkyl, C₁₋₆-Alkoxy oder C₂₋₆-Alkenyl, und
"Alkenyl" und "Alkenyl*" jeweils unabhängig voneinander C₂₋₆-Alkenyl bedeuten,
enthält.

7. Flüssigkristallines Medium nach einem oder mehreren der Ansprüche 1 bis 6, **dadurch gekennzeichnet, dass** es zusätzlich eine oder mehrere Verbindungen der Formel XIII, 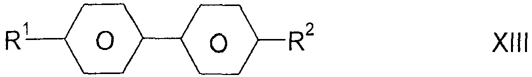 worin R¹ und R² jeweils unabhängig voneinander n-Alkyl, Alkoxy, Oxaalkyl, Fluoralkyl oder Alkenyl mit jeweils bis zu 6 C-Atomen bedeuten,
enthält.

8. Flüssigkristallines Medium nach einem oder mehreren der Ansprüche 1 bis 7, **dadurch gekennzeichnet, dass** es zusätzlich eine oder mehrere Verbindungen der Formel XVII, 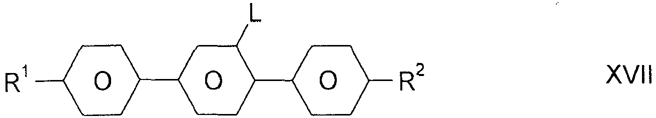 worin R¹ und R² jeweils unabhängig voneinander n-Alkyl, Alkoxy, Oxaalkyl, Fluoralkyl oder Alkenyl mit jeweils bis zu 8 C-Atomen, und L H oder F bedeuten,
enthält.

9. Flüssigkristallines Medium nach einem oder mehreren der Ansprüche 1 bis 8, **dadurch gekennzeichnet, dass** es zusätzlich eine oder mehrere Verbindungen ausgewählt aus der Gruppe der Verbindungen der Formeln XXVII, XXVIII und XXIX, 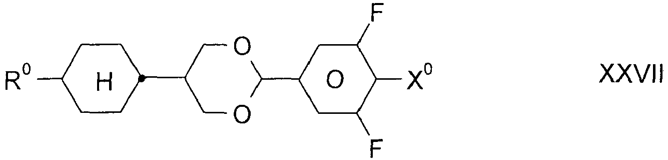 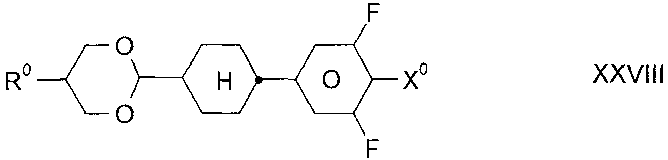 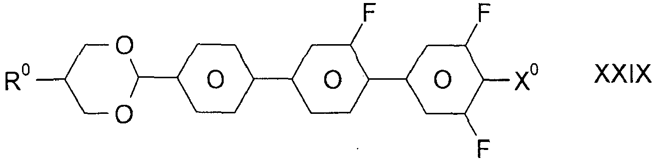 worin R⁰ und X⁰ die in Anspruch 4 angegebenen Bedeutungen haben,
enthält.

10. Flüssigkristallines Medium nach einem oder mehreren der Ansprüche 1 bis 9, **dadurch gekennzeichnet, dass** es zusätzlich eine oder mehrere Verbindungen ausgewählt aus der Gruppe der Verbindungen der Formeln XIX, XX, XXI, XXII, XXIII und XXIV, 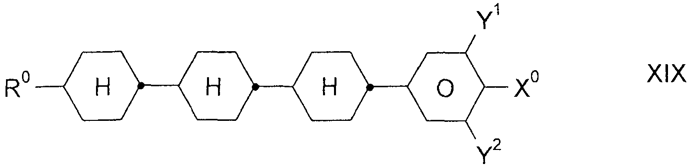 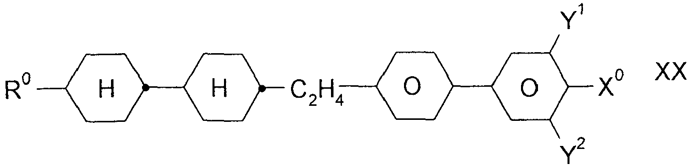 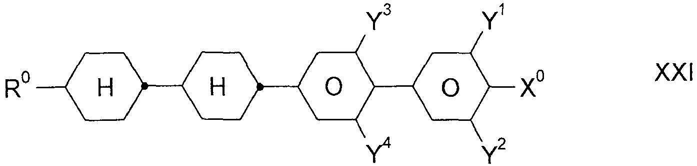 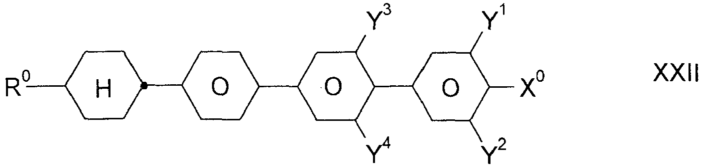 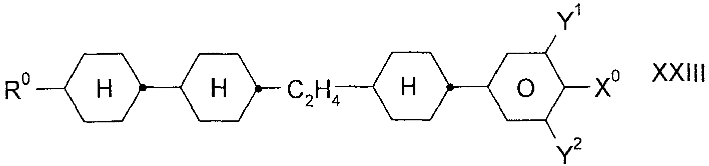 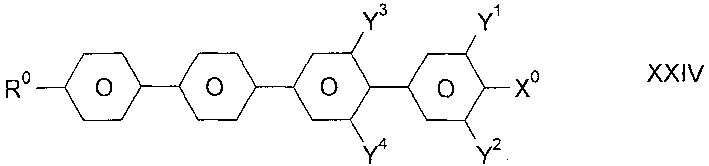 worin R⁰ und X⁰ die in Anspruch 4 angegebenen Bedeutungen haben und Y¹⁻⁴ jeweils unabhängig voneinander H oder F bedeuten,
enthält.

11. Flüssigkristallines Medium nach einem oder mehreren der Ansprüche 1 bis 10, **dadurch gekennzeichnet, dass** es ≥ 20 Gew.% der Verbindung der Formel IXb, 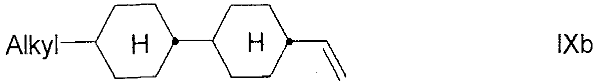 worin Alkyl die in Anspruch 6 angegebene Bedeutung hat,
enthält.

12. Flüssigkristallines Medium nach einem oder mehreren der Ansprüche 1 bis 11, **dadurch gekennzeichnet, dass** es zusätzlich ein oder mehrere Additiv(e) ausgewählt aus der Gruppe der UV-Stabilisatoren, Dotierstoffe und Antioxidantien enthält.

13. Flüssigkristallines Medium nach einem oder mehreren der Ansprüche 1 bis 12, **dadurch gekennzeichnet, dass** es zusätzlich ein oder mehrere polymerisierbare Verbindungen enthält.

14. Verfahren zur Herstellung eines flüssigkristallinen Mediums nach einem oder mehreren der Ansprüche 1 bis 13, **dadurch gekennzeichnet, dass** man eine oder mehrere Verbindungen der Formel I, wie in Anspruch 1 definiert, mit einer oder mehreren Verbindungen gemäß einem oder mehreren der Ansprüche 4 bis 11 oder mit weiteren flüssigkristallinen Verbindungen und gegebenenfalls noch mit ein oder mehreren Additiven und/oder mindestens einer polymerisierbaren Verbindung mischt.

15. Verwendung eines flüssigkristallinen Mediums nach einem oder mehreren der Ansprüche 1 bis 13 für elektrooptische Zwecke.

16. Verwendung des flüssigkristallinen Mediums nach Anspruch 15 in Shutter-Brillen, für 3D-Anwendungen, in TN-, PS-TN-, STN-, TN-TFT-, OCB-, IPS-, PS-IPS-, FFS-, PS-FFS- und PS-VA-IPS-Anzeigen.

17. Elektrooptische Flüssigkristallanzeige enthaltend ein flüssigkristallines Medium nach einem oder mehreren der Ansprüche 1 bis 13.

18. Verbindungen der Formel I 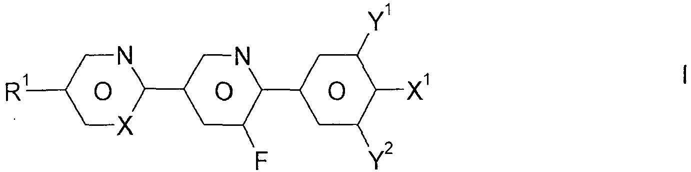 worin
R¹ einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 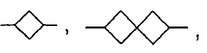 -CO-O- oder -O-CO- so ersetzt sein können, dass O-Atome nicht direkt miteinander verknüpft sind,
X¹ F, Cl, CN, SF₅, SCN, NCS, halogenierter Alkylrest, halogenierter Alkenylrest, halogenierter Alkoxyrest oder halogenierter Alkenyloxyrest jeweils mit bis zu 6 C-Atomen,
X C-H oder N, und
Y¹ und Y² jeweils unabhängig voneinander H oder F
bedeuten.

## Claims

1. Liquid-crystalline medium, **characterised in that** it comprises one or more compounds of the formula I, 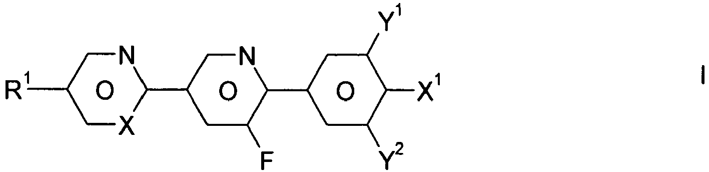 in which
R¹ denotes a halogenated or unsubstituted alkyl or alkoxy radical having 1 to 15 C atoms, where, in addition, one or more CH₂ groups in these radicals may each be replaced, independently of one another, by -C≡C-, -CF₂O-, -CH=CH-, 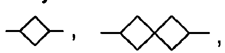 -O-, -CO-O- or -O-CO- in such a way that O atoms are not linked directly to one another,
X¹ denotes F, Cl, CN, SF₅, SCN, NCS, a halogenated alkyl radical, a halogenated alkenyl radical, a halogenated alkoxy radical or a halogenated alkenyloxy radical, in each case having up to 6 C atoms,
X C-H or N, and
Y¹ and Y² each, independently of one another, denote H or F.

2. Liquid-crystalline medium according to Claim 1, **characterised in that** it comprises one or more compounds selected from the compounds of the formulae I-1 to I-15, 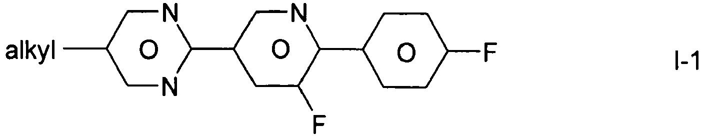 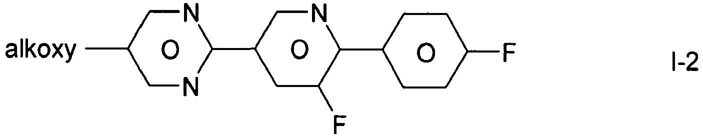 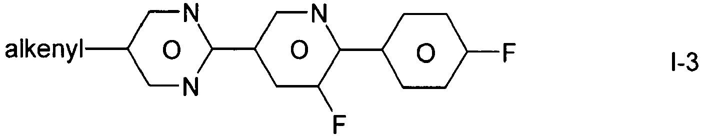 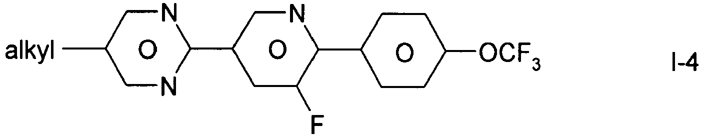 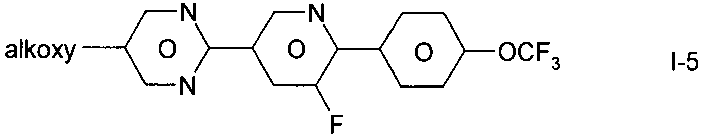 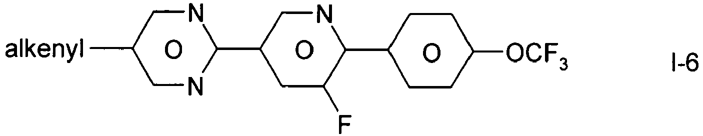 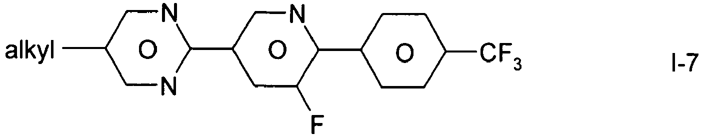 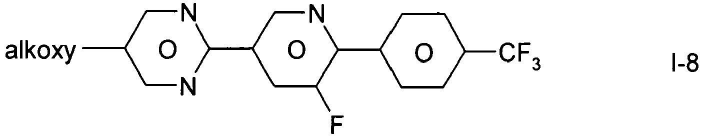 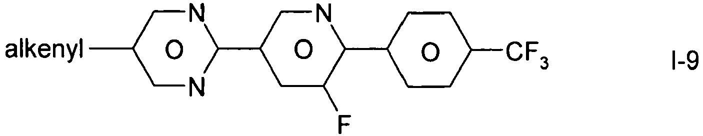 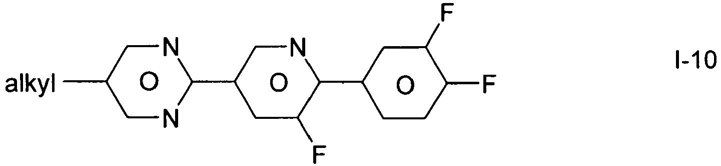 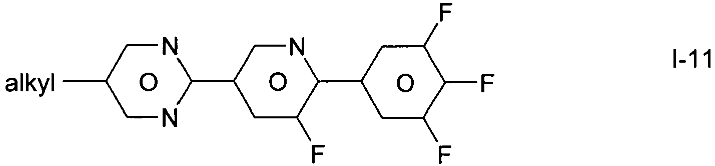 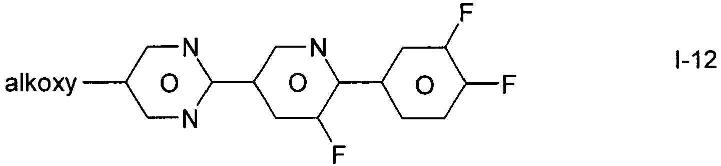 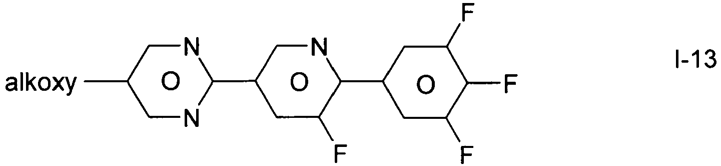 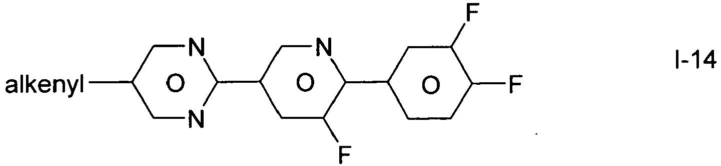 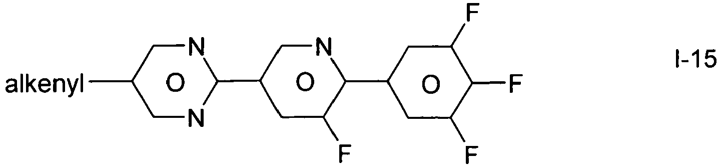 in which
alkyl denotes a straight-chain alkyl radical having 1-6 C atoms,
alkenyl denotes a straight-chain alkenyl radical having 2-6 C atoms,
alkoxy denotes a straight-chain alkoxy radical having 1-6 C atoms.

3. Liquid-crystalline medium according to Claim 1 or 2, **characterised in that** it comprises one or more compounds of the formula I-4 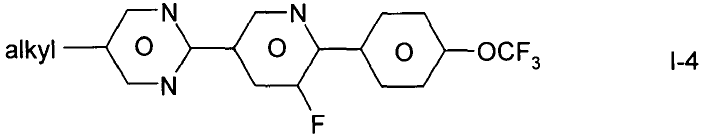 in which
alkyl denotes a straight-chain alkyl radical having 1-6 C atoms,

4. Liquid-crystalline medium according to one or more of Claims 1 to 3, **characterised in that** it additionally comprises one or more compounds selected from the formulae II and/or III, 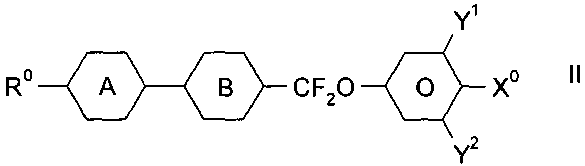 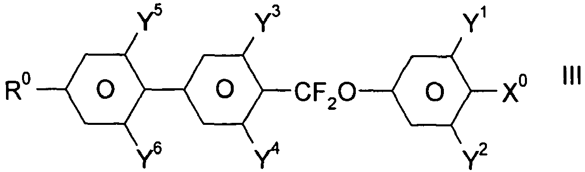 in which
R⁰ denotes a halogenated or unsubstituted alkyl or alkoxy radical, in each case having 1 to 15 C atoms, where, in addition, one or more CH₂ groups in these radicals may each be replaced, independently of one another, by -C≡C-, -CF₂O-, -CH=CH-, 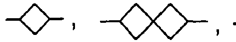 -O-, -CO-O- or -O-CO- in such a way that O atoms are not linked directly to one another,
X⁰ denotes F, Cl, CN, SF₅, SCN, NCS, a halogenated alkyl radical, a halogenated alkenyl radical, a halogenated alkoxy radical or a halogenated alkenyloxy radical, in each case having up to 6 C atoms, and
Y¹⁻⁶ each, independently of one another, denote H or F, 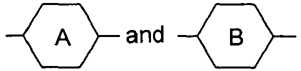 each, independently of one another, denote

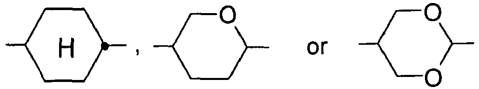

5. Liquid-crystalline medium according to one or more of Claims 1 to 4, **characterised in that** it additionally comprises one or more compounds selected from the formulae IV to VIII, 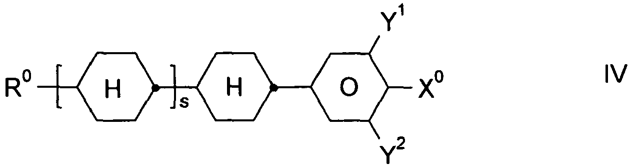 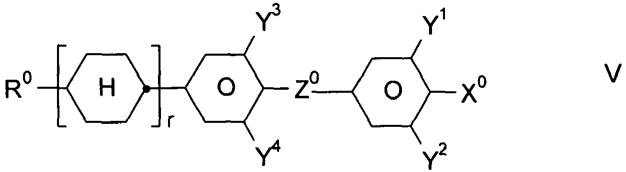 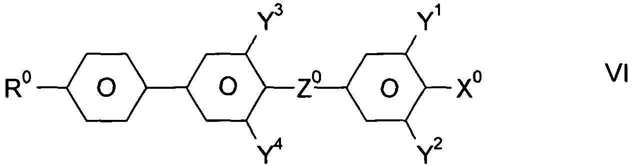 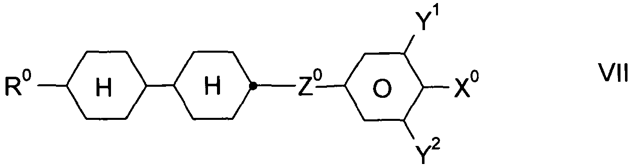 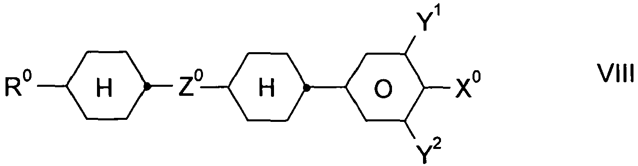 in which R⁰, X⁰ and Y¹⁻⁴ have the meanings indicated in Claim 4,
Z⁰ denotes -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- or -OCF₂-, in formulae V and VI also a single bond,
r denotes 0 or 1, and
s denotes 0 or 1.

6. Liquid-crystalline medium according to one or more of Claims 1 to 5, **characterised in that** it additionally comprises one or more compounds selected from the formulae IX to XII, 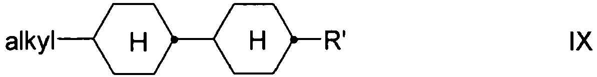 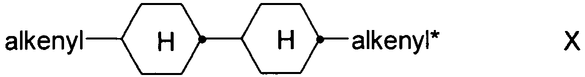 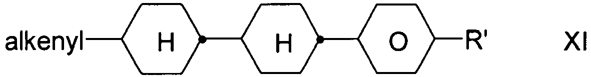 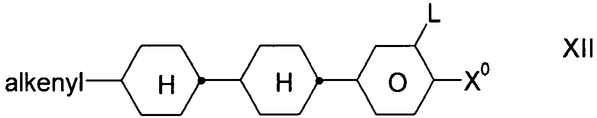 in which X⁰ has the meanings indicated in Claim 4,
L denotes H or F,
"alkyl" denotes C₁₋₆-alkyl,
R' denotes C₁₋₆-alkyl, C₁₋₆-alkoxy or C₂₋₆-alkenyl, and
"alkenyl" and "alkenyl*" each, independently of one another, denote C₂₋₆-alkenyl.

7. Liquid-crystalline medium according to one or more of Claims 1 to 6, **characterised in that** it additionally comprises one or more compounds of the formula XIII, 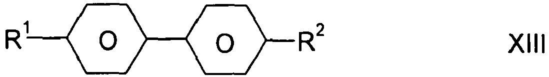 in which R¹ and R² each, independently of one another, denote n-alkyl, alkoxy, oxaalkyl, fluoroalkyl or alkenyl, in each case having up to 6 C atoms.

8. Liquid-crystalline medium according to one or more of Claims 1 to 7, **characterised in that** it additionally comprises one or more compounds of the formula XVII, 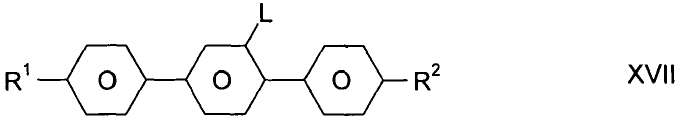 in which R¹ and R² each, independently of one another, denote n-alkyl, alkoxy, oxaalkyl, fluoroalkyl or alkenyl, in each case having up to 8 C atoms, and L denotes H or F.

9. Liquid-crystalline medium according to one or more of Claims 1 to 8, **characterised in that** it additionally comprises one or more compounds selected from the group of the compounds of the formulae XXVII, XXVIII and XXIX, 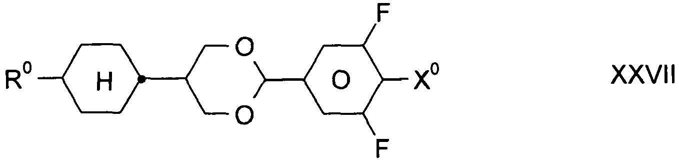 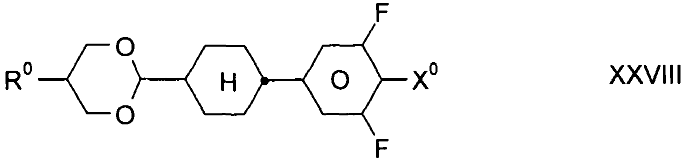 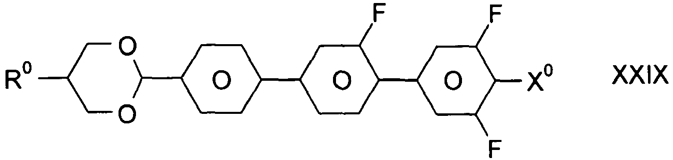 in which R⁰ and X⁰ have the meanings indicated in Claim 4.

10. Liquid-crystalline medium according to one or more of Claims 1 to 9, **characterised in that** it additionally comprises one or more compounds selected from the group of the compounds of the formulae XIX, XX, XXI, XXII, XXIII and XXIV, 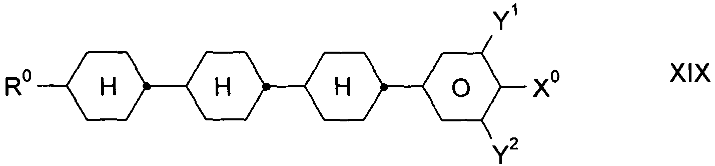 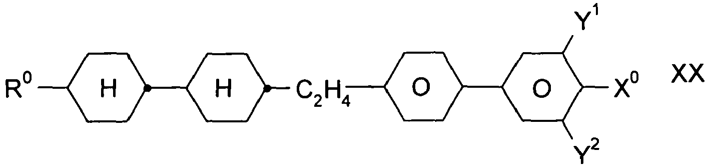 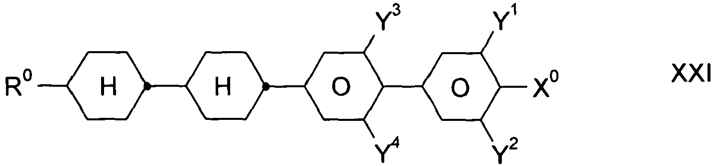 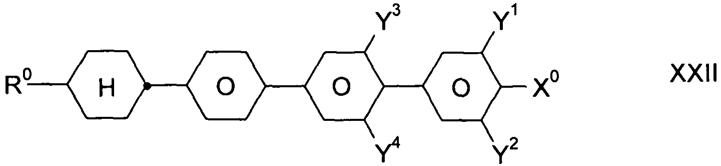 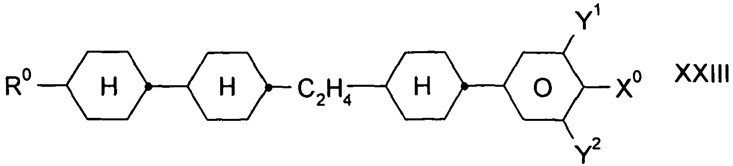 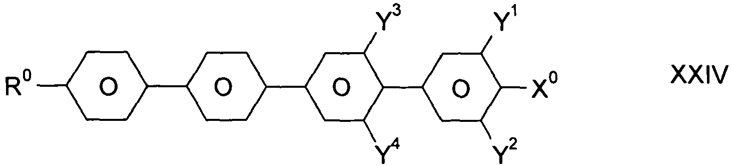 in which R⁰ and X⁰ have the meanings indicated in Claim 4, and Y¹⁻⁴ each, independently of one another, denote H or F.

11. Liquid-crystalline medium according to one or more of Claims 1 to 10, **characterised in that** it comprises ≥ 20% by weight of the compound of the formula IXb, 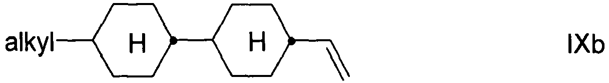 in which alkyl has the meaning indicated in Claim 6.

12. Liquid-crystalline medium according to one or more of Claims 1 to 11, **characterised in that** it additionally comprises one or more additive(s) selected from the group of the UV stabilisers, dopants and antioxidants.

13. Liquid-crystalline medium according to one or more of Claims 1 to 12, **characterised in that** it additionally comprises one or more polymerisable compounds.

14. Process for the preparation of a liquid-crystalline medium according to one or more of Claims 1 to 13, **characterised in that** one or more compounds of the formula I, as defined in Claim 1, are mixed with one or more compounds according to one or more of Claims 4 to 11 or with further liquid-crystalline compounds and optionally also with one or more additives and/or at least one polymerisable compound.

15. Use of a liquid-crystalline medium according to one or more of Claims 1 to 13 for electro-optical purposes.

16. Use of the liquid-crystalline medium according to Claim 15 in shutter spectacles, for 3D applications, in TN, PS-TN, STN, TN-TFT, OCB, IPS, PS-IPS, FFS, PS-FFS and PS-VA-IPS displays.

17. Electro-optical liquid-crystal display containing a liquid-crystalline medium according to one or more of Claims 1 to 13.

18. Compounds of the formula I 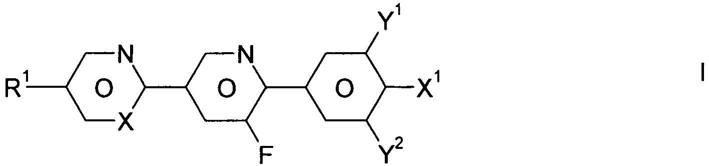 in which
R¹ denotes a halogenated or unsubstituted alkyl or alkoxy radical having 1 to 15 C atoms, where, in addition, one or more CH₂ groups in these radicals may each be replaced, independently of one another, by -C≡C-, -CF₂O-, -CH=CH-, 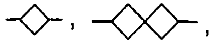 -O-, -CO-O- or -O-CO- in such a way that O atoms are not linked directly to one another,
X¹ denotes F, Cl, CN, SF₅, SCN, NCS, a halogenated alkyl radical, a halogenated alkenyl radical, a halogenated alkoxy radical or a halogenated alkenyloxy radical, in each case having up to 6 C atoms,
X denotes C-H or N, and
Y¹ and Y² each, independently of one another, denote H or F.

## Revendications

1. Milieu cristallin liquide, **caractérisé en ce qu'**il comprend un ou plusieurs composé(s) de la formule I : 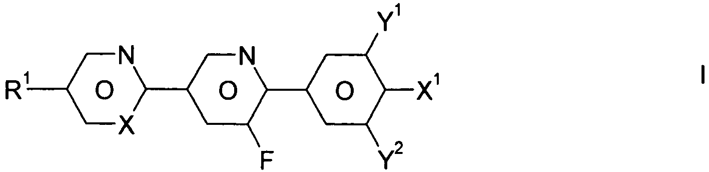 dans laquelle :
R¹ représente un radical alkyle ou alcoxy halogéné ou non substitué comportant 1 à 15 atome(s) de C, où, en outre, un ou plusieurs groupe(s) CH₂ dans ces radicaux peut/ peuvent chacun être remplacé(s), indépendamment les uns des autres, par -C≡C-, -CF₂O-, -CH=CH-, 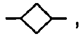 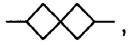 -O-, -CO-O- ou -O-CO- de telle sorte que des atomes de O ne soient pas liés directement les uns aux autres,
X¹ représente F, CI, CN, SF₅, SCN, NCS, un radical alkyle halogéné, un radical alkényle halogéné, un radical alcoxy halogéné ou un radical alkényloxy halogéné, dans chaque cas comportant jusqu'à 6 atomes de C,
X est C-H ou N, et
Y¹ et Y² représentent, chacun indépendamment de l'autre, H ou F.

2. Milieu cristallin liquide selon la revendication 1, **caractérisé en ce qu'**il comprend un ou plusieurs composé(s) choisi(s) parmi les composés des formules I-1 à I-15 : 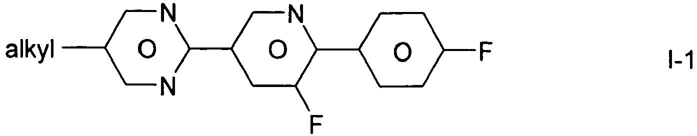 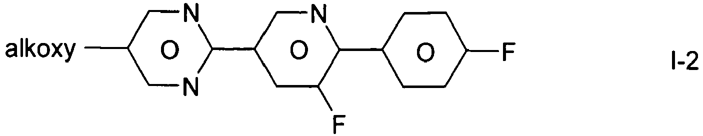 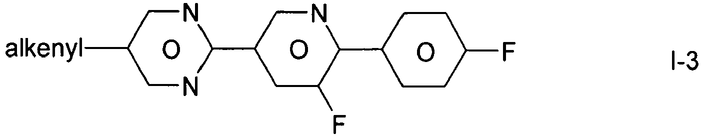 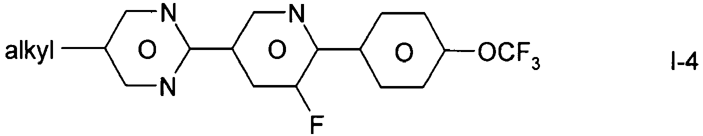 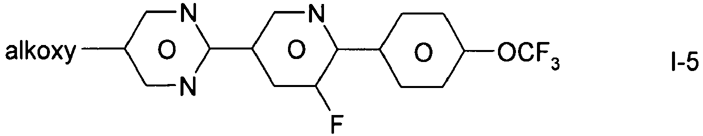 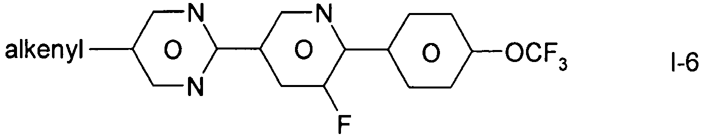 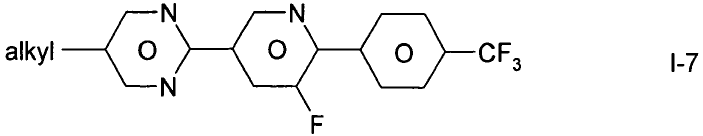 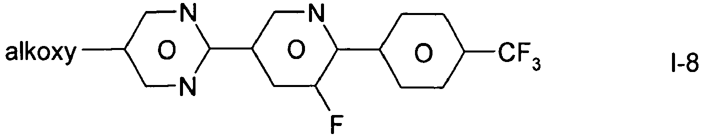 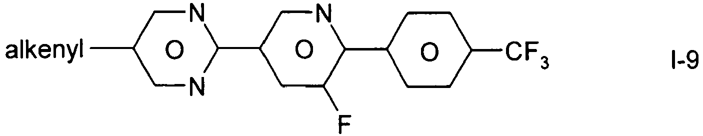 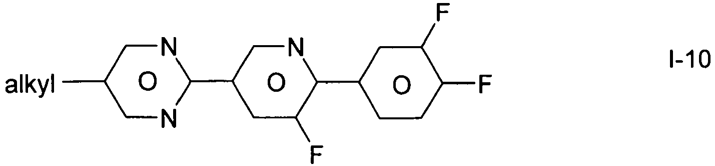 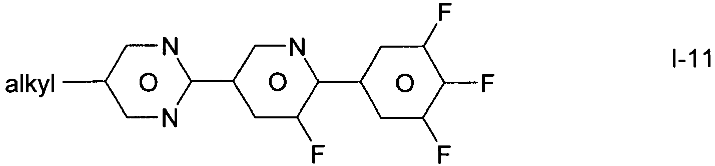 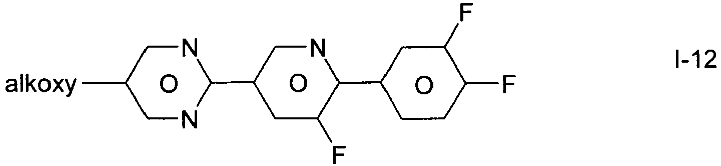 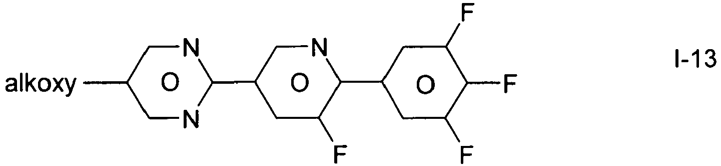 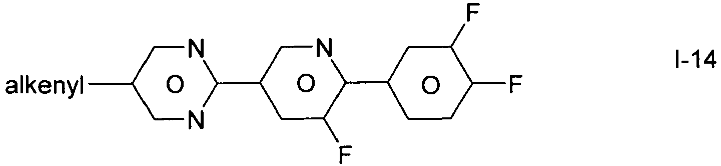 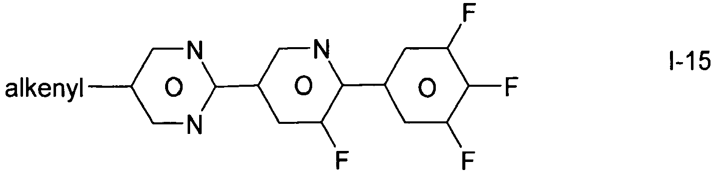 dans lesquelles:
alkyl représente un radical alkyle en chaîne droite comportant 1-6 atome(s) de C,
alkenyl représente un radical alkényle en chaîne droite comportant 2-6 atomes de C, et
alkoxy représente un radical alcoxy en chaîne droite comportant 1-6 atome(s) de C.

3. Milieu cristallin liquide selon la revendication 1 ou 2, **caractérisé en ce qu'**il comprend un ou plusieurs composé(s) de la formule I-4 : 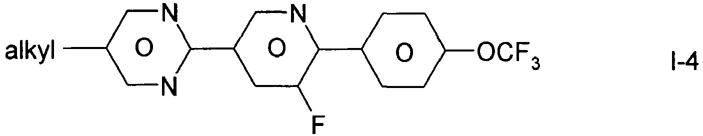 dans laquelle :
alkyl représente un radical alkyle en chaîne droite comportant 1-6 atome(s) de C.

4. Milieu cristallin liquide selon une ou plusieurs des revendications 1 à 3, **caractérisé en ce qu'**il comprend de façon additionnelle un ou plusieurs composé(s) choisi(s) parmi les formules II et/ou III : 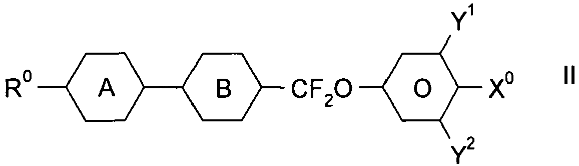 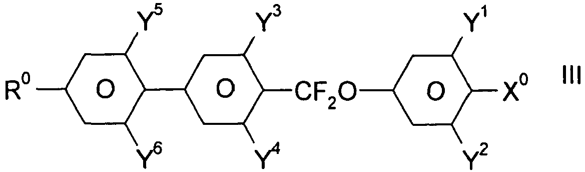 dans lesquelles :
R⁰ représente un radical alkyle ou alcoxy halogéné ou non substitué, dans chaque cas comportant 1 à 15 atome(s) de C, où, en outre, un ou plusieurs groupe(s) CH₂ dans ces radicaux peut/ peuvent chacun être remplacé(s), indépendamment les uns des autres, par -C≡C-, -CF₂O-, -CH=CH-, 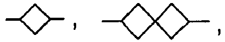 -O-, -CO-O- ou -O-CO- de telle sorte que des atomes de O ne soient pas liés directement les uns aux autres,
X⁰ représente F, CI, CN, SF₅, SCN, NCS, un radical alkyle halogéné, un radical alkényle halogéné, un radical alcoxy halogéné ou un radical alkényloxy halogéné, dans chaque cas comportant jusqu'à 6 atomes de C, et
Y¹⁻⁶ représentent chacun, indépendamment les uns des autres, H ou F, 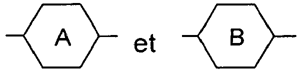 représentent chacun, indépendamment l'un de l'autre,

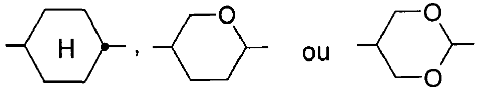

5. Milieu cristallin liquide selon une ou plusieurs des revendications 1 à 4, **caractérisé en ce qu'**il comprend de façon additionnelle un ou plusieurs composé(s) choisi(s) parmi les formules IV à VIII : 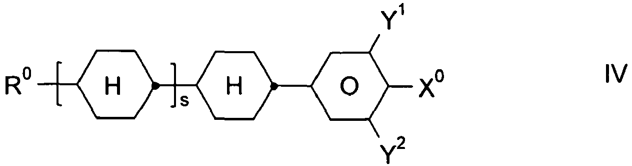 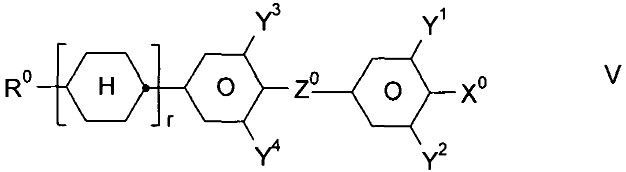 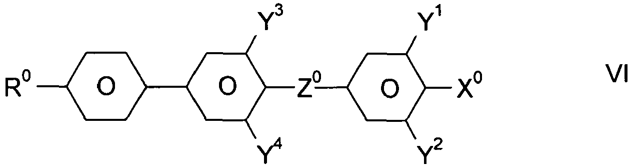 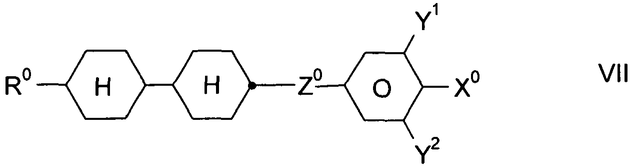 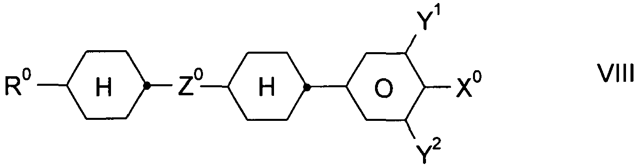 dans lesquelles R⁰, X⁰ et Y¹⁻⁴ présentent les significations indiquées selon la revendication 4,
Z⁰ représente -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- ou -OCF₂-, dans les formules V et VI également une liaison simple,
r représente 0 ou 1, et
s représente 0 ou 1.

6. Milieu cristallin liquide selon une ou plusieurs des revendications 1 à 5, **caractérisé en ce qu'**il comprend de façon additionnelle un ou plusieurs composé(s) choisi(s) parmi les formules IX à XII : 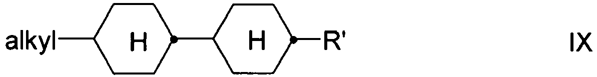 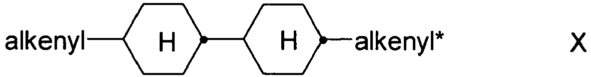 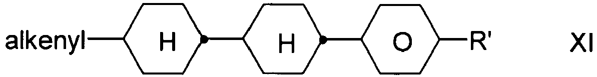 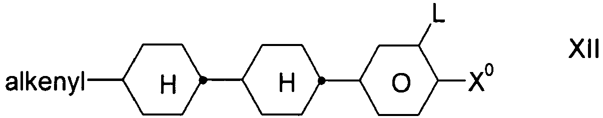 dans lesquelles X⁰ présente les significations indiquées selon la revendication 4,
L représente H ou F,
"alkyl" représente C₁₋₆-alkyle,
R' représente C₁₋₆-alkyle, C₁₋₆-alcoxy ou C₂₋₆-alkényle, et
"alkenyl" et "alkenyl*" représentent chacun, indépendamment l'un de l'autre, C₂₋₆-alkényle.

7. Milieu cristallin liquide selon une ou plusieurs des revendications 1 à 6, **caractérisé en ce qu'**il comprend de façon additionnelle un ou plusieurs composé(s) de la formule XIII : 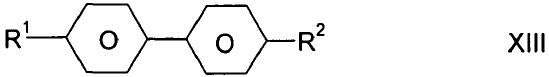 dans laquelle R¹ et R² représentent chacun, indépendamment l'un de l'autre, n-alkyle, alcoxy, oxaalkyle, fluoroalkyle ou alkényle, dans chaque cas comportant jusqu'à 6 atomes de C.

8. Milieu cristallin liquide selon une ou plusieurs des revendications 1 à 7, **caractérisé en ce qu'**il comprend de façon additionnelle un ou plusieurs composé(s) de la formule XVII : 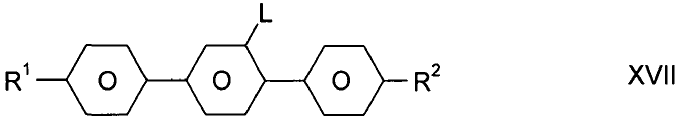 dans laquelle R¹ et R² représentent chacun, indépendamment l'un de l'autre, n-alkyle, alcoxy, oxaalkyle, fluoroalkyle ou alkényle, dans chaque cas comportant jusqu'à 8 atomes de C, et L représente H ou F.

9. Milieu cristallin liquide selon une ou plusieurs des revendications 1 à 8, **caractérisé en ce qu'**il comprend de façon additionnelle un ou plusieurs composé(s) choisi(s) parmi le groupe des composés des formules XXVII, XXVIII et XXIX : 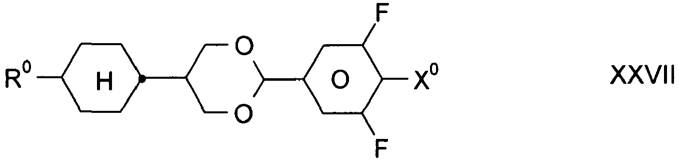 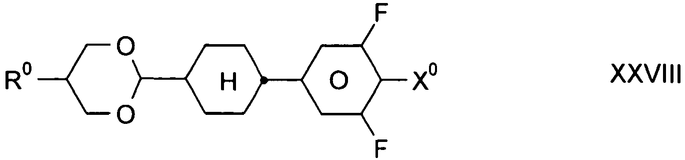 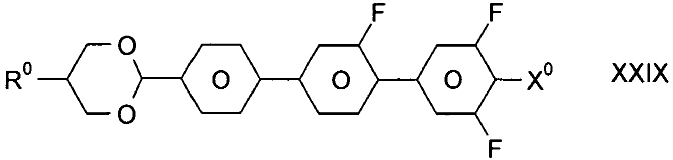 dans lesquelles R⁰ et X⁰ présentent les significations indiquées selon la revendication 4.

10. Milieu cristallin liquide selon une ou plusieurs des revendications 1 à 9, **caractérisé en ce qu'**il comprend de façon additionnelle un ou plusieurs composé(s) choisi(s) parmi le groupe des composés des formules XIX, XX, XXI, XXII, XXII et XXIV : 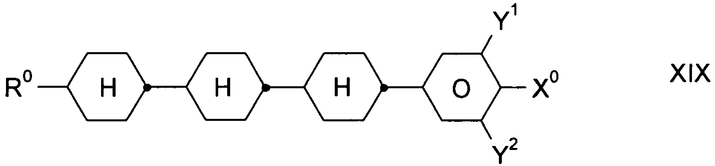 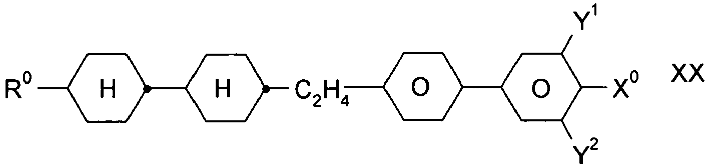 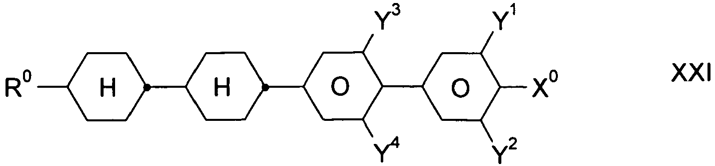 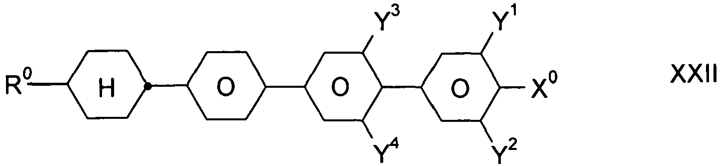 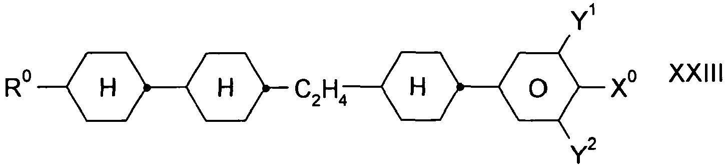 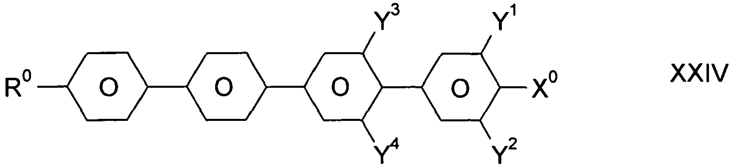 dans lesquelles R⁰ et X⁰ présentent les significations indiquées selon la revendication 4, et Y¹⁻⁴ représentent chacun, indépendamment les uns des autres, H ou F.

11. Milieu cristallin liquide selon une ou plusieurs des revendications 1 à 10, **caractérisé en ce qu'**il comprend ≥ 20% en poids du composé de la formule IXb : 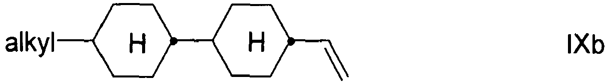 dans laquelle alkyl présente la signification indiquée selon la revendication 6.

12. Milieu cristallin liquide selon une ou plusieurs des revendications 1 à 11, **caractérisé en ce qu'**il comprend de façon additionnelle un ou plusieurs additif(s) choisi(s) parmi le groupe des stabiliseurs UV, des dopants et des antioxydants.

13. Milieu cristallin liquide selon une ou plusieurs des revendications 1 à 12, **caractérisé en ce qu'**il comprend de façon additionnelle un ou plusieurs composé(s) polymérisable(s).

14. Procédé pour la préparation d'un milieu cristallin liquide selon une ou plusieurs des revendications 1 à 13, **caractérisé en ce qu'**un ou plusieurs composé(s) de la formule I, comme défini selon la revendication 1, est/sont mélangé(s) avec un ou plusieurs composé(s) selon une ou plusieurs des revendications 4 à 11 ou avec d'autres composés cristallins liquides et en option également, avec un ou plusieurs additif(s) et/ou au moins un composé polymérisable.

15. Utilisation d'un milieu cristallin liquide selon une ou plusieurs des revendications 1 à 13 à des fins électro-optiques.

16. Utilisation du milieu cristallin liquide selon la revendication 15 dans des lunettes à obturateurs, pour des applications 3D, dans des affichages TN, PS-TN, STN, TN-TFT, OCB, IPS, PS-IPS, FFS, PS-FFS et PS-VA-IPS.

17. Affichage à cristaux liquides électro-optique contenant un milieu cristallin liquide selon une ou plusieurs des revendications 1 à 13.

18. Composés de la formule I : 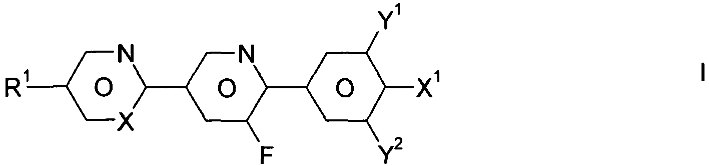 dans laquelle :
R¹ représente un radical alkyle ou alcoxy halogéné ou non substitué comportant 1 à 15 atome(s) de C, où, en outre, un ou plusieurs groupe(s) CH₂ dans ces radicaux peut/peuvent chacun être remplacé(s), indépendamment les uns des autres, par -C≡C-, -CF₂O-, -CH=CH-, 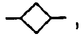 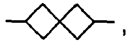 -O-, -CO-O- ou -O-CO- de telle sorte que des atomes de O ne soient pas liés directement les uns aux autres,
X¹ représente F, CI, CN, SF₅, SCN, NCS, un radical alkyle halogéné, un radical alkényle halogéné, un radical alcoxy halogéné ou un radical alkényloxy halogéné, dans chaque cas comportant jusqu'à 6 atomes de C,
X représente C-H ou N, et
Y¹ et Y² représentent chacun, indépendamment l'un de l'autre, H ou F.
